# EUROPEAN PATENT APPLICATION

(11) **EP 4 458 952 A1**
(43) Date of publication of application: **06.11.2024**
(21) Application number: 22915071.9
(22) Date of filing: 29.12.2022
(51) Int. Cl.: C12N 5/0783, C07K 14/73, C07K 14/725, C07K 16/10, C12N 15/113, A61K 35/17, A61P 31/18

(54) **CHIMERIC ANTIGEN RECEPTOR T CELLS TARGETING HIV-INFECTED CELLS**

(30) Priority: 31.12.2021 CN 202111666522; 02.04.2022 CN 202210350268
(71) Applicant: Beijing SoloBio Genetechnology Co., Ltd., Beijing 100176 (CN)
(72) Inventor: ZHU, Weijun, Beijing 100176 (CN); WANG, Hongwei, Beijing 100176 (CN); YANG, Ying, Beijing 100176 (CN); FAN, Jundie, Beijing 100176 (CN)
(74) Representative: Barker Brettell LLP
(86) International application number: PCT/CN2022/143440
(87) International publication number: WO 2023/125822

(57) **Abstract**

One aspect of the present application relates to a recombinant cell comprising a chimeric antigen receptor and shRNA targeted to inhibit the life cycle of HIV. Another aspect of the present application relates to a chimeric antigen molecule, the extracellular region of which is from or comprising the extracellular region of human CD4 molecule, the transmembrane region of which is from or comprising the transmembrane domain of CD8α, and a recombinant cell comprising the above chimeric antigen molecule. The above recombinant cell can be used to treat HIV infection, and have stronger comprehensive killing efficacy, longer effective time, and less risk of causing cytokine storm in the environment of HIV infection.

## Description

### TECHNICAL FIELD

The present application relates to the technical field of infectious disease immunotherapy, particularly to an engineered immune effector cell for HIV immunotherapy.

### BACKGROUND

AIDS, also known as acquired immunodeficiency syndrome, is a major infectious disease threatening human life, and is caused by human immunodeficiency virus (HIV). HIV is a virus that can attack the human immune system. HIV mainly attacks and destroys CD4 positive T lymphocytes in the human immune system, depriving the human body of immune function and making it easy to develop various disease or even malignant tumors, with relatively high fatality rate. By the end of 2019, about 38 million people in the world were infected with HIV, about 1.7 million people were newly infected and680 thousand people died due to HIV (UNAIDS/WHO) in the current year. It has been 40 years since the first AIDS person was diagnosed, while AIDS is still an important public health issue that seriously threatens public health.

In the present, there are no effective AIDS vaccines in the world. Highly Active Antiretroviral Therapy (HAART), commonly known as "cocktail therapy" and also known as Antiretroviral Therapy (ART), is mainly used in clinical treatment. By the end of 2017, nearly 21.7 million people have received ART (UNAIDS/WHO). The present ART treatment does not clear HIV-1 viral reservoir, and all of the patients require lifetime treatment. Once the treatment is stopped, the virus in the viral reservoir can be reactivated and replicated, making the disease bounce back quickly, so patients need to take medicine permanently.

To date, only two patients in the world have been confirmed to be cured, they are "Berlin patient" and "London patient". Both of them were cured by transplanting bone marrow from donors that lack normal receptor genes (CCR5) required for the cell infection of most HIV strains. However, the mortality rate of bone marrow transplantation itself is high, and for most people, the matched bone marrow with such genetic features barely exists, which prevents this method being widely applied in clinical.

ART has transformed HIV infection into a chronic infection. At present, the unsatisfied clinical needs mainly include long-lasting, safer, and tolerable therapy solutions. About 10% of patients who are resistant to the ART need treatments with new mechanism. Treatments against viral reservoir are needed to archive functional cure and even clearance of the reservoir for eradication.

In order to achieve functional cure, broad-spectrum neutralizing antibodies can be used, multi-specific molecules can also be developed on the basis of antibodies, and T cells can be modified by utilizing chimeric antigen receptors (CAR). In addition to neutralizing viruses directly, the broad-spectrum neutralizing antibody can also clear the cells displaying viral proteins by binding macrophages, NK cells, and CD8 + T cells with Fc. For example, for gp120, many broad-spectrum neutralizing antibodies including 3BNC117 have been generated (Scheid et al., Science 2011). However, many virus strains have developed resistance to these antibodies, and the resistance must be overcome for optimal clinical efficacy (Qian Wang et al., Front Med. 2020 Feb;14(1):30-42) . On the other hand, based on broad-spectrum neutralizing antibodies or virus receptors, CAR-T cells can be directly modified to specifically recognize viruses and are able to lyse HIV-infected cells both *in vivo* and *in vitro.* Two clinical trials based on third-generation CAR-T using broad-spectrum neutralizing antibodies are now in progress and carried out in China. One of the trials is the cooperation between Guangzhou Eighth People's Hospital and Sun Yat-sen University, which is related to modifying CD8+ T cells based on VRC01 for effective treatment of patients (NCT03240328; Liu et al., J Clin. Invest. 2021); the other one is the cooperation between Wuhan Yungu and Wuhan Jinyintan Hospital, which is based on N6 and combined with HAART treatment for newly treated patients (ChiCTR-OPN-17013068). The results disclosed by NCT0340328 show that the use of CAR-T is safe, however, during the observation period of 6 months, the HIV load of patients rebounded in about 5.3 weeks by average after HAART treatment was stopped, and after rebounded the viruses all escape the neutralizing antibody VRC01. There is no formal result disclosed in the N6-based CAR-T clinical trial.

Another idea for curing AIDS is to reactivate latent HIV, which, by combined with the method of enhancing HIV-specific immunity in the body, can repair the immune damage caused by HIV, and enhance the clearance of the HIV reservoir. Early in 1994, Roberts *et al.* try to treat HIV infection with CAR-T cells, in which complete CD4 sequence was selected as an antigen-specific recognition domain for binding to the envelope protein gp120 on the surface of HIV-infected cells. Although the CAR-T cells had partial function of killing infected cells, however, after years of efforts, it finally ended in failure. The reason was, on one hand, that the specific killing activity of the CAR-T cells needed to be further improved, on the other hand, that the CAR-T cells were also susceptible to HIV infection, and were easily exhausted in an HIV-infected environment, which have shortened the duration of efficacy.

When HIV infects cells, the virus envelope glycoprotein gp120 first binds to CD4 molecule on the cell surface and then binds to the auxiliary receptor CCR5 or CXCR4 or the like, which thereby mediates the entry of viral core into the cell. CD4 on the cell surface is the primary binding target of gp120 and they have a very high binding affinity with each other. The dissociation constant Kd of CD4 and several gp120 is 2.2×10⁻⁸-8×10⁻¹⁰M (Lasky et al Cell 1987; Zhang Biochemistry et al 2000; Myszka et al PNAS 2000) . In comparison, the dissociation constant Kd of CD4 or CD8 with a particular MHC-polypeptide complex is only 10⁻⁴-10⁻⁶M (ZeNan L. Chang and Yvonne Y Chen Trends in Molecular Medicine 2017) . Moreover, the affinity of CD4 to several typical HIV-1 gp120 (e.g., AC10.29 using CCR5 as an auxiliary receptor, NL4-3 using CCR5 as an auxiliary receptor, and SF162 capable of using two auxiliary receptors at the same time) is no weaker than the affinity of scFv from typical broad-spectrum neutralizing antibodies (3BNC117) to these typical HIV-1 gp120 (Scheid et al Science 2011; van Dorsten et al J Viral 2020) .

American Gene Technologies (AGT) and other companies applied shRNA for CCR5 and combined it with two or more shRNA for HIV, which showed a good effect in vitro. AGT expects to induce HIV-specific CD4 cells by prior polypeptide immunization in vivo, introduce shRNA into the cells in vitro and then reinfused them back in vivo, expecting in vivo proliferation of the protected CD4 cell which accounted for about 0.3% of the total CD4 cells of the human body. The clinical trial of this project is in progress, and only one participant has been reinfused. However, there is no result yet.

At present, active measures are still needed to achieve a functional cure for AIDS and even the latent reservoir clearance, and finally achieve total eradication and cure.

### SUMMARY OF THE INVENTION

In order to further improve the cell therapy method (for example, CAR-T cells) for HIV infection, in one aspect, the present application provides a recombinant cell (for example, CAR-T cells) that has stronger killing activity to HIV-infected cells, maintains for a longer period of time in HIV-infected environment, and has a longer duration of efficacy. In another aspect, the present application also provides a chimeric antigen receptor (CAR) for HIV, a cell comprising the chimeric antigen receptor has efficient and specific killing activity against HIV-infected cells, and a combination of the CAR and at least one shRNA/the nucleic acid coding for shRNA /vector containing the nucleic acid coding for shRNA. In addition, the recombinant cell or the cell containing the chimeric antigen receptor provided by the present application is not easy to cause cytokine storm.

In one aspect, the present application relates to a recombinant cell, in particular:
1. A recombinant cell comprising the following functional structure:
   1) a chimeric antigen receptor (CAR) or a nucleic acid coding therefor, wherein the CAR comprises an extracellular region, a transmembrane region and an intracellular region, wherein the extracellular region can specifically bind to the gp120 protein of HIV.
   2) at least one short hairpin RNA (shRNA), or a nucleic acid coding therefor, wherein the shRNA targets one or more host genes or HIV genes involved in the life cycle of HIV selected from the group consisting of NF-κB, CCR5, TSG 101, CXCR4, P-TEFb, tat, rev, nef, env, LTR and gag.

In some embodiments, the recombinant cells are derived from human or primate animals. In other embodiments, the recombinant cells are derived from patients infected with HIV, or from healthy population. In some embodiments, the recombinant cells are derived from HIV receptor cells or peripheral blood mononuclear cells. In a further preferred embodiment of the present invention, the recombinant cells are derived from lymphocytes. In some embodiments, the recombinant cells are derived from T cells. In other embodiments, the recombinant cells are derived from naive T cells, memory T cells, or effector T cells. In other embodiments, the recombinant cells are derived from cytotoxic T cells, helper T cells, or regulatory T cells. In other embodiments, the T cells are derived from CD4+ T cells or CD8+ T cells. In other embodiments, the recombinant cells are derived from NKT cells. In other embodiments, the recombinant cells are derived from γδ T cells. In other embodiments, the recombinant cells are derived from NK cells. In other embodiments, the recombinant cells are derived from antigen-presenting cells, such as macrophages and dendritic cells.

In a further preferred embodiment of the present invention, the recombinant cells are derived from progenitor cells or stem cells. In some embodiments of the present invention, the progenitor cells or stem cells include hematopoietic stem cells (e.g., CD34+ cells) or hematopoietic progenitor cells. In other embodiments of the present invention, the stem cells include memory T stem cells, such as central memory T cells, effector memory T cells, or stem cell memory T cells. In another embodiment of the invention, the stem cells are induced to differentiate into any of the T cells as previously described.

In some embodiments, the recombinant cells are derived from patients infected with HIV.

In some embodiments, the recombinant cells are derived from healthy population.
2. The recombinant cell of item 1, wherein the shRNA targets HIV gag gene and LTR gene. In some embodiments, the shRNA targets HIV gag gene and nef gene.
3. The recombinant cell of item 1 or 2, wherein the nucleic acid sequence of the shRNA comprises the sequence of SEQ ID NO: 1, 2, 5, and/or 6 or the sequence having at least 85%, 90%, 93%, 95%, 97%, 98%, 99% identity to the sequence of SEQ ID NO: 1, 2, 5, and/or 6.

In some embodiments, the promoter used in the expression of SEQ ID NO: 1 and 2 is a strong promoter, such as H1 promoter and U6 promoter. In some embodiments, the expression of SEQ ID NO: 1 and 2 uses the same promoter. In some embodiments, the expression of SEQ ID NO: 1 and 2 uses different promoters. In some embodiments, the expression of SEQ ID NO: 1 and 2 uses H1 promoter and U6 promoter respectively.

In some embodiments, the promoter used in the expression of SEQ ID NO: 5 and 6 is a strong promoter, such as H1 promoter and U6 promoter. In some embodiments, the expression of SEQ ID NO: 5 and 6 uses the same promoter. In some embodiments, the expression of SEQ ID NO: 5 and 6 uses different promoters. In some embodiments, the expression of SEQ ID NO: 5 and 6 uses H1 promoter and U6 promoter respectively.

In some embodiments, the nucleic acid sequence of H1 promoter and the U6 promoter are as shown in SEQ ID NO: 9 and 10 respectively, or having at least 85%, 90%, 93%, 95%, 97%, 98%, 99% identity to SEQ ID NO: 9 and 10, respectively.

4. The recombinant cell of any one of items 1-3, wherein the extracellular region comprises the structure selected from the group consisting of D1 domain of human CD4 molecule, D1 and D2 domain of human CD4 molecule, D1-D3 domain of human CD4 molecule, D1-D4 domain of human CD4 molecule, an antibody specifically binding to gp120, or an antigen-binding fragment thereof (such as the sequence shown in SEQ ID NO: 15).

In some embodiments, the extracellular region comprises D1 and D2 domain of human CD4 molecule without D3 and D4 domain. In some particular embodiments, the extracellular region comprises the amino acid sequence of SEQ ID NO: 13 or the sequence having at least 85%, 90%, 93%, 95%, 97%, 98%, 99% or more identity to the amino acid sequence of SEQ ID NO: 13.
5. The recombinant cell of any one of items 1-4, wherein the transmembrane region is derived from or comprises the transmembrane domain of one or more of the protein molecules selected from the group consisting of: T cell receptor α, β or ζ chain, CD28, CD3ε, CD45, CD4, CD5, CD8, CD9, CD16, CD22, CD33, CD37, CD64, CD80, CD86, CD134, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2Rβ, IL2Rγ, IL7Rα, ITGA1, VLA1, CD49a, ITGA4, IA4, CD49D4, ITGA6, VLA-6, CD49f, ITGAD, CD11d, ITGAE, CD103, ITGAL, CD11a, ITGAM, CD11b, ITGAX, CD11c, ITGB1, CD29, ITGB2, CD18, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKp44, NKp30, NKp46, NKG2D, and NKG2C.
6. The recombinant cell of item 5, wherein the transmembrane region is from or comprises the transmembrane domain of CD8α protein molecule.
7. The recombinant cell of any one of items 1-6, the transmembrane region comprising the amino acid sequence of SEQ ID NO: 18 or the amino acid sequence having at least 85%, 90%, 93%, 95%, 97%, 98%, 99% identity to SEQ ID NO: 18.
8. The recombinant cell of any one of items 1-7, wherein the intracellular region comprises a primary signal transduction domain derived from or comprising the signal transduction domain selected from the group consisting of CD3ζ, CD3γ, CD3δ, CD3ε, FCER1G, FcεR1b, CD79a, CD79b, FcγRlla, DAP10 and DAP12.
9. The recombinant cell of item 8, wherein the intracellular region further comprises a co-stimulatory domain derived from or comprising the signal transduction domain of one or more of the protein molecules selected from the group consisting of: CD27, CD28, 4-1 BB, OX40, CD30, CD40, PD-1, ICOS, LFA-1, CD2, CD7, LIGHT, NKG2C, B7-H3, ligands specifically bind to CD83, CDS, ICAM-1, GITR, BAFFR, HVEM (LIGHTR), SLAMF7, NKp80, CD160, CD19, CD4, CD8α, CD8β, IL2Rβ, IL2Rγ, IL7Rα, VLA1, CD49a, ITGA4, IA4, CD49D, ITGA6, VLA-6, CD49f, ITGAD, CD11d, ITGAE, CD103, ITGAL, CD11a, ITGAM, CD11b, ITGAX, CD11c, ITGB1, CD29, ITGB2, CD18, ITGB7, TNFR2, TRANCE/RANKL, DNAM1, SLAMF4, CD84, CD96, CEACAM1, CRTAM, Ly9, CD160, PSGL1, CD100, CD69, SLAMF6, SLAM, BLAME, SELPLG, LTBR, LAT, GADS, SLP-76, PAG/Cbp, NKp44, NKp30, NKp46, NKG2D, and DAP10.
10. The recombinant cell of item 8 or 9, wherein the primary signal transduction domain is derived from or comprises the signal transduction domain of CD3ζ.
11. The recombinant cell of item 9 or 10, wherein the co-stimulatory domain is derived from or comprises one of the signal transduction domains of CD137, CD28, 2B4 or DAP10, or a combination thereof.
12. The recombinant cell of item 9 or 11, wherein a linker peptide is also comprised in the intracellular region between the primary signal transduction domain and each co-stimulatory domain, wherein the linker peptide is any natural or synthetic peptides that can connect the primary signal transduction domain and each co-stimulatory domain and maintain a certain relative spatial distance to ensure their functional integrity and signal conduction between them. In some specific embodiments, the linker peptide is a short peptide formed by G and S, such as (GS)n or (G)n(S)m, wherein n or m is any positive integer between 1-20, for example (GGGGS) 1,2,3.
13. The recombinant cell of any one of items 1-12, wherein the intracellular region comprises any one of the amino acid sequences of SEQ ID NO: 28-34 or the amino acid sequence having at least 85%, 90%, 93%, 95%, 97%, 98%, 99% identity to any one of SEQ ID NO: 28-34.
14. The recombinant cell of any one of items 1-13, wherein the extracellular region is connected to the transmembrane region by the hinge region.
15. The recombinant cell of item 14, wherein the hinge region is from or comprises human Ig hinge region, GS linker, KIR2DS2 hinge, or the hinge region of CD8α. In some embodiments, the hinge region is from or comprises the hinge region of human CD8α. In some embodiments, the hinge region comprises the amino acid sequence of SEQ ID NO: 20 or the amino acid sequence having at least 85%, 90%, 93%, 95%, 97%, 98%, 99% identity to SEQ ID NO: 20.
16. The recombinant cell of any one of items 1-15, wherein the CAR comprises an amino acid sequence selected from any one of SEQ ID NO: 35-46 or the amino acid sequence having at least 85%, 90%, 93%, 95%, 97%, 98%, 99% identity to any one of SEQ ID NO: 35-46.
17. The recombinant cell of any one of items 1-16, wherein the CAR is further linked to a signal peptide that can locate the CAR on cell membrane of the recombinant cell. In some embodiments, the signal peptide is from or comprises the signal peptide of any secretory proteins or membrane proteins.
18. The recombinant cell of item 17, wherein the signal peptide is from or comprises the signal peptide of CD4 or CD8.
19. The recombinant cell of any one of items 1-18, the signal peptide comprising the amino acid sequence of SEQ ID NO: 16 or 17 or the amino acid sequence having at least 85%, 90%, 93%, 95%, 97%, 98%, 99% identity to SEQ ID NO: 16 or 17.

In some specific embodiments, the recombinant cell comprises the nucleic acid sequence of SEQ ID NO: 1 and 2 (or SEQ ID NO: 5 and 6) and the amino acid sequence of SEQ ID NO: 35 or the sequence having at least 85%, 90%, 93%, 95%, 97%, 98%, 99% identity to SEQ ID NO: 1 and 2 (or SEQ ID NO: 5 and 6) or SEQ ID NO: 35. In some specific embodiments, the recombinant cell comprises the nucleic acid sequence encoding the nucleic acid sequence of SEQ ID NO: 1 and 2 (or SEQ ID NO: 5 and 6) and the amino acid sequence of SEQ ID NO: 35. In some specific embodiments, the recombinant cell comprises the nucleic acid sequence of SEQ ID NO: 3 and 4 (or SEQ ID NO: 7 and 8) and the nucleic acid sequence encoding the amino acid sequence of SEQ ID NO: 35.

In some specific embodiments, the recombinant cell comprises the nucleic acid sequence of SEQ ID NO: 1 and 2 (or SEQ ID NO: 5 and 6) and the amino acid sequence of SEQ ID NO: 36 or the sequence having at least 85%, 90%, 93%, 95%, 97%, 98%, and 99% identity to SEQ ID NO: 1 and 2 (or SEQ ID NO: 5 and 6) or SEQ ID NO: 36. In some specific embodiments, the recombinant cell comprises a nucleic acid sequence encoding the nucleic acid sequence of SEQ ID NO: 1 and 2 (or SEQ ID NO: 5 and 6) and the amino acid sequence of SEQ ID NO: 36. In some specific embodiments, the recombinant cell comprises the nucleic acid sequence of SEQ ID NO: 3 and 4 (or SEQ ID NO: 7 and 8) and the nucleic acid sequence encoding the amino acid sequence of SEQ ID NO: 36.

In some specific embodiments, the recombinant cell comprises the nucleic acid sequence of SEQ ID NO: 1 and 2 (or SEQ ID NO: 5 and 6) and the amino acid sequence of SEQ ID NO: 43 or a sequence having at least 85%, 90%, 93%, 95%, 97%, 98%, and 99% identity to SEQ ID NO: 1 and 2 (or SEQ ID NO: 5 and 6) or SEQ ID NO: 43. In some specific embodiments, the recombinant cell comprises the nucleic acid sequence encoding the nucleic acid sequence of SEQ ID NO: 1 and 2 (or SEQ ID NO: 5 and 6) and the amino acid sequence of SEQ ID NO: 43. In some specific embodiments, the recombinant cell comprises the nucleic acid sequence of SEQ ID NO: 3 and 4 (or SEQ ID NO: 7 and 8) and a nucleic acid sequence encoding the amino acid sequence of SEQ ID NO: 43.

In some specific embodiments, the recombinant cell comprises the nucleic acid sequence of SEQ ID NO: 1 and 2 (or SEQ ID NO: 5 and 6) and the amino acid sequence of SEQ ID NO: 46 or the sequence having at least 85%, 90%, 93%, 95%, 97%, 98%, and 99% identity to SEQ ID NO: 1 and 2 (or SEQ ID NO: 5 and 6) or SEQ ID NO: 46. In some specific embodiments, the recombinant cell comprises the nucleic acid sequence encoding the nucleic acid sequence of SEQ ID NO: 1 and 2 (or SEQ ID NO: 5 and 6) and the amino acid sequence of SEQ ID NO: 46. In some specific embodiments, the recombinant cell comprises the nucleic acid sequence of SEQ ID NO: 3 and 4 (or SEQ ID NO: 7 and 8) and the nucleic acid sequence encoding the amino acid sequence of SEQ ID NO: 46.

In other embodiments, the recombinant cell further comprises a reporter molecule and/or a safety switch. In some specific embodiments, the safety switch is selected from one or more of the following: iCaspase-9, iCaspase-1, iCaspase-8, thymidine kinase (e.g., HSV-TK, VZV-TK), cytosine deaminase (CD), CD20, tEGFR, FR806, and RQP8. In another preferred embodiment, the safety switch is tEGFR, preferably, the amino acid sequence of the tEGFR is shown in SEQ ID NO: 49 or 50.
20. A vector comprising the polynucleotide encoding CAR and/or shRNA of the recombinant cell in any one of items 1-19.
21. The vector of item 20 is selected from plasmids, viral vectors or linear nucleic acid molecules. In some particular embodiments, the vector is retroviral shuttle plasmid vector. In some embodiments, the vector is SIV viral vector.
22. The vector of item 20 or 21, wherein the expression of the chimeric antigen receptor and shRNA are driven by a single promoter or by different promoters.
23. A pharmaceutical composition, comprising the recombinant cell of any one of items 1-19 or the vector of any one of items 20-22. The pharmaceutical composition can be used for treating patients infected with HIV. In some embodiments, the HIV-infected patients have received long-term anti-retroviral therapy.
24. A method for treating and/or preventing HIV infection or AIDS, comprising administering to a subject infected with HIV or suffering from AIDS the recombinant cell of any one of items 1-19 or the vector of any one of items 20-22.
25. The application of the recombinant cell of any one of items 1-19 or the vector of any one of items 20-22 in the preparation of a medicament for treating and/or preventing HIV infection or AIDS.

In addition, the present application also provides a method for preparing the recombinant cell of any one of items 1-19, comprising introducing the vector of any one of items 20-22 into the cell for expression. In some embodiments, the method comprises packaging the SIV virus shuttle plasmid vector into SIV virus particles and infecting the cells using the virus particles.

In another aspect, the present application also relates to a chimeric antigen receptor (CAR), in particular:
1. A chimeric antigen receptor (CAR) comprising an extracellular region, a transmembrane region and an intracellular region, wherein the extracellular region is from or comprises the extracellular region of human CD4 molecule, and the transmembrane region is from or comprising the transmembrane domain of CD8α.
2. The chimeric antigen receptor of item 1, wherein the chimeric antigen receptor further comprises a hinge region. Preferably, the extracellular region is connected to the transmembrane region by the hinge region.
3. The chimeric antigen receptor of item 2, wherein the hinge region is from or comprises human Ig hinge region, GS linker, KIR2DS2 hinge, or the hinge region of CD8α, preferably the hinge region of CD8α.
4. The chimeric antigen receptor of item 3, wherein the hinge region comprises the amino acid sequence of SEQ ID NO: 20 or the amino acid sequence having at least 85%, 90%, 93%, 95%, 97%, 98%, 99% identity to the sequence of SEQ ID NO: 20.
5. The chimeric antigen receptor of any one of items 1-4, wherein the extracellular region of the human CD4 molecule consists of D1 and D2 domain of CD4 molecule.
6. The chimeric antigen receptor of item 5, wherein the extracellular region comprises the amino acid sequence of SEQ ID NO: 13.
7. The chimeric antigen receptor of any one of items 1-6, wherein the transmembrane region comprises the amino acid sequence of SEQ ID NO: 18.
8. The chimeric antigen receptor of any one of items 1-7, wherein the intracellular region comprises a primary signal transduction domain and a co-stimulatory domain, and the primary signal transduction domain is from or comprises the signal transduction domain of one or more protein molecule selected from the group consisting of: CD3ζ, CD3γ, CD3δ, CD3ε, FCER1G, FcεR1b, CD79a, CD79b, FcγRlla, DAP10, and DAP12, and the co-stimulatory domain is from or comprises the signal transduction domain of one or more protein molecule selected from the group consisting of: CD27, CD28, 4-1 BB, OX40, CD30, CD40, PD-1, ICOS, LFA-1, CD2, CD7, LIGHT, NKG2C, B7-H3, ligands specifically bind to CD83, CDS, ICAM-1, GITR, BAFFR, HVEM (LIGHTR), SLAMF7, NKp80, CD160, CD19, CD4, CD8α, CD8β, IL2Rβ, IL2Rγ, IL7Rα, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1, SLAMF4, CD84, CD96, CEACAM1, CRTAM, Ly9, CD160, PSGL1, CD100, CD69, SLAMF6, SLAM, BLAME, SELPLG, LTBR, LAT, GADS, SLP-76, PAG/Cbp, NKp44, NKp30, NKp46, NKG2D, and DAP10.
9. The chimeric antigen receptor of item 8, wherein the primary signal transduction domain is from or comprises the signal transduction domain of CD3ζ or DAP10, and the co-stimulatory domain is from or comprises one of the signal transduction domains of CD137, CD28, 2B4, or DAP10, or a combination thereof.
10. The chimeric antigen receptor of any one of items 1-9, wherein the intracellular region comprises:
   (i) the signal transduction domains of CD137 and CD3ζ;
   (ii) the signal transduction domains of CD28, CD137, and CD3ζ;
   (iii) the signal transduction domains of CD137, CD3ζ, and DAP10;
   (iv) the signal transduction domains of CD28, DAP10, and CD3ζ;
   (v) the signal transduction domains of 2B4, DAP10, and CD3ζ; or
   (vi) the signal transduction domains of CD137, 2B4, and CD3ζ.
11. The chimeric antigen receptor of any one of items 8 or 9, wherein a linker peptide is also comprised in the intracellular region between the primary signal transduction domain and the co-stimulatory domain.
12. The chimeric antigen receptor of item 11, wherein the sequence of the linker peptide comprises the amino acid sequence of SEQ ID NO: 22.
13. The chimeric antigen receptor of any one of items 1-10, wherein the sequence of intracellular regions comprises the amino acid sequence of any one of SEQ ID NO: 28-34 or the amino acid sequence having at least 85%, 90%, 93%, 95%, 97%, 98%, 99% identity to any one of SEQ ID NO: 28-34.
14. The chimeric antigen receptor of any one of items 1-13, wherein a further signal peptide is linked to the chimeric antigen receptor, and the signal peptide is from or comprises the signal peptide of any secretory proteins or membrane proteins.
15. The chimeric antigen receptor of item 14, wherein the signal peptide is from or comprises the signal peptide of CD4 or CD8 molecule, and preferably, the signal peptide comprises the amino acid sequence of SEQ ID NO: 16 or 17.
16. The chimeric antigen receptor of any one of items 1-15, wherein the chimeric antigen receptor comprises the amino acid sequence of any one of SEQ ID NO: 35, 36, 43-46, or comprises the amino acid sequence having at least 85%, 90%, 93%, 95%, 97%, 98%, 99% identity to any one of SEQ ID NO: 35, 36, 43-46.
17. A polynucleotide encoding the chimeric antigen receptor of any one of items 1-16.
18. A vector comprising the polynucleotide of item 17.
19. The vector of item 18, selected from the group consisting of plasmids, viral vectors, or linear nucleic acid molecules.
20. A recombinant cell comprising the chimeric antigen receptor of any one of items 1-16, or the polynucleotide of item 17, or the vector of item 18 or 19.
21. The recombinant cell of item 20, wherein the recombinant cell is derived from human or primate animal. More preferably, the recombinant cell is derived from patients infected with HIV or from healthy population. More preferably, the recombinant cell is derived from HIV receptor cell, peripheral blood mononuclear cell or lymphocyte; further preferably, the recombinant cell is derived from T cells (e.g., naive T cell, memory T cell, effector T cell, cytotoxic T cell, helper T cell, regulatory T cell, CD4+ T cell, CD8+ T cell, NKT cell, γδ T cells, etc.), NK cell, antigen-presenting cell (e.g., macrophage, dendritic cell, etc.); or the recombinant cell is derived from progenitor cell or stem cell, including but not limited to hematopoietic stem cell, hematopoietic progenitor cell, memory T stem cell (e.g., central memory T cell, effector memory T cell or stem cell-like memory T cell).

Furthermore, the present application also relates to a combination of the above chimeric antigen receptor and at least one shRNA/the nucleic acid coding for shRNA /vector comprising the nucleic acid coding for shRNA, in particular:
22. The combination of chimeric antigen receptor of items 1-16, or the polynucleotide of claim 17, the vector of claim 18 or 19, or the recombinant cell of claim 20 or 21 and at least one shRNA targeting HIV, or the nucleic acid coding therefor, or a vector comprising the nucleic acid thereof.
23. The combination of item 22, wherein the at least one shRNA targets any one or more host genes or HIV genes involved in the HIV life cycle selected from the group consisting of NF-κB, CCR5, TSG101, CXCR4, P-TEFb, tat, rev, nef, env, LTR, and gag.

The combination of item 23, wherein the combination comprises:
(i) shRNA targeting gag and shRNA targeting LTR, or
(ii) shRNA targeting gag and shRNA targeting nef.

25. The combination of item 23 or 24, wherein the shRNA targeting gag comprises the sequence of SEQ ID NO: 1 or 5 or the sequence having at least 85% identity to the sequence of SEQ ID NO: 1 or 5, the shRNA targeting LTR comprises the sequence of SEQ ID NO: 2 or the sequence having at least 85% identity to the sequence of SEQ ID NO: 2, and the shRNA targeting nef comprises the sequence of SEQ ID NO: 6 or the sequence having at least 85% identity to the sequence of SEQ ID NO: 6.
26. The combination of item 25, wherein the combination comprises:
   (i) shRNA targeting gag and shRNA targeting LTR, wherein the shRNA targeting gag comprises the sequence of SEQ ID NO: 1, and the shRNA targeting LTR comprises the sequence of SEQ ID NO: 2.
   (ii) shRNA targeting gag and shRNA targeting nef, wherein the shRNA targeting gag comprises the sequence of SEQ ID NO: 5, and the shRNA targeting nef comprises the sequence of SEQ ID NO: 6.
27. The combination of item 23, wherein the combination comprises:
   (i) a nucleic acid coding for the shRNA targeting gag and a nucleic acid coding for the shRNA targeting LTR, or a vector comprising the above nucleic acid, or
   (ii) a nucleic acid coding for the shRNA targeting gag and a nucleic acid coding for the shRNA targeting nef, or a vector comprising the above nucleic acid.
28. The combination of item 23 or 27, wherein the nucleic acid coding for the shRNA targeting gag comprises the sequence of SEQ ID NO: 3 or 7 or the sequence having at least 85%, 90%, 93%, 95%, 97%, 98%, 99% identity to SEQ ID NO: 3 or 7, and the nucleic acid coding for the shRNA targeting LTR comprises the sequence of SEQ ID NO: 4 or the sequence having at least 85%, 90%, 93%, 95%, 97%, 98%, 99% identity to SEQ ID NO: 4, and the nucleic acid coding for the shRNA targeting nef comprises the sequence of SEQ ID NO: 8 or the sequence having at least 85%, 90%, 93%, 95%, 97%, 98%, 99% identity to SEQ ID NO: 8.
29. The combination of item 28, wherein the combination comprises:
   (i) a nucleic acid coding for the shRNA targeting gag and a nucleic acid coding for the shRNA targeting LTR, wherein the nucleic acid coding for the shRNA targeting gag comprises the sequence of SEQ ID NO: 3, and the nucleic acid coding for the shRNA targeting LTR comprises the sequence of SEQ ID NO: 4, or a vector comprising the above nucleic acid, or
   (ii) a nucleic acid coding for the shRNA targeting gag and a nucleic acid coding for the shRNA targeting nef, wherein the nucleic acid coding for the shRNA targeting gag comprises the sequence of SEQ ID NO: 7, and the nucleic acid coding for the shRNA targeting nef comprises the sequence of SEQ ID NO: 8, or a vector comprising the above nucleic acid.

A pharmaceutical composition, comprising the combination of the recombinant cell of item 20 or 21, or the recombinant cell of any one of items 22-29 and at least one shRNA or the nucleic acid coding therefor or a vector comprising the nucleic acid thereof, and a pharmaceutically acceptable carrier. In a specific embodiment, the pharmaceutical composition further comprises other anti-HIV drugs, including but not limited to reverse transcriptase inhibitors, protease inhibitors, HIV vaccines, broad-spectrum neutralizing antibodies, and/or CAR-T cells. In another specific embodiment, the pharmaceutical composition of the present invention is used for treating and/or preventing HIV infection or AIDS.
31. The use of the combination of the chimeric antigen receptor of items 1-16, or the polynucleotide sequence of item 17, or the vector described in item 18 or 19, or the cell described in item 20 or 21, or any one of items 22-29 in the preparation of a medicament for treating and/or preventing HIV infection or AIDS.
32. A method for treating and/or preventing HIV infection or AIDS, comprising administering to a subject infected with HIV or suffering from AIDS the combination of the cell of claim 20 or 21, or the recombinant cell of any one of items 22-29 and at least one shRNA or the nucleic acid coding therefor or a vector comprising the nucleic acid thereof, or a pharmaceutical composition of item 30. In a specific embodiment of the present invention, when administrating the combination of the recombinant cell of any one of items 22-29 and at least one shRNA or the nucleic acid coding therefor or a vector comprising the nucleic acid thereof, the recombinant cell and the at least one shRNA or the nucleic acid coding therefor or the vector comprising the nucleic acid thereof can be administrated sequentially or simultaneously. In a specific embodiment of the present invention, the method further comprises administering to the subject other anti-HIV drugs, including but not limited to other nucleoside inhibitors, HIV vaccines, broad-spectrum neutralizing antibodies and/or CAR-T cells.

### BRIEF DESCRIPTION OF FIGURES

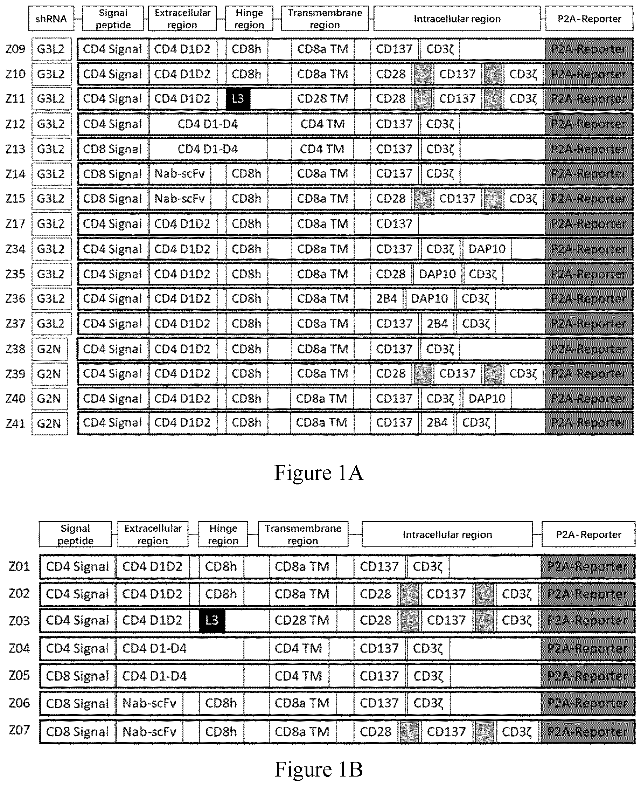

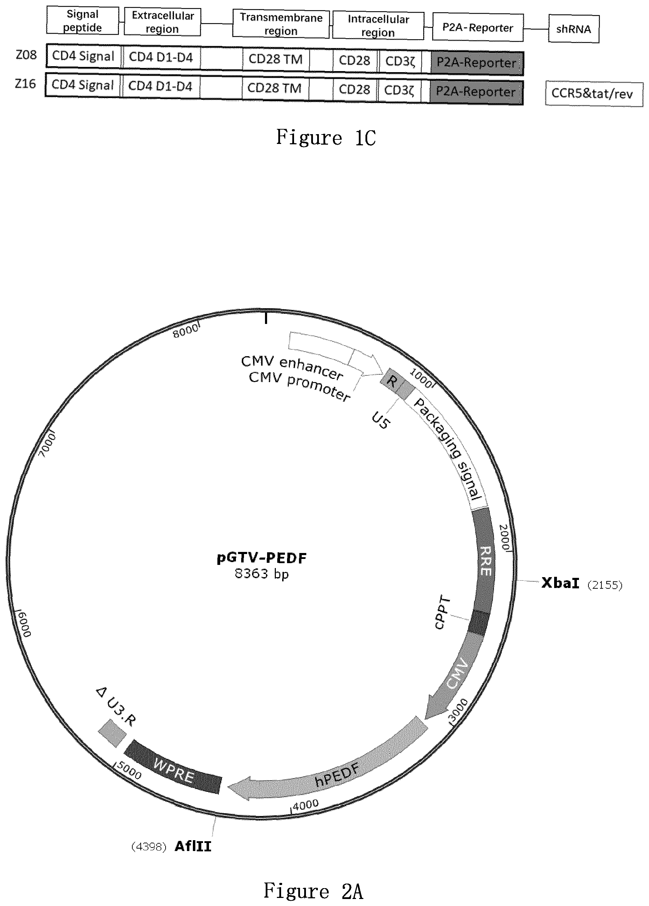

FIGS. 1A-1C show functional structure regions inserted between Xba I and BamH I enzyme digestion sites in each plasmid tested in the examples herein, including functional structures of CAR coding sequences and/or shRNA coding sequences, and/or reporter genes. G3L2 represents the coding sequence of shRNA (whose sequence is SEQ ID NO: 1 and 2, respectively) for Gag and LTR, and G2N represents the coding sequence of shRNA (whose sequence is SEQ ID NO: 5 and 6, respectively) for Gag and Nef.

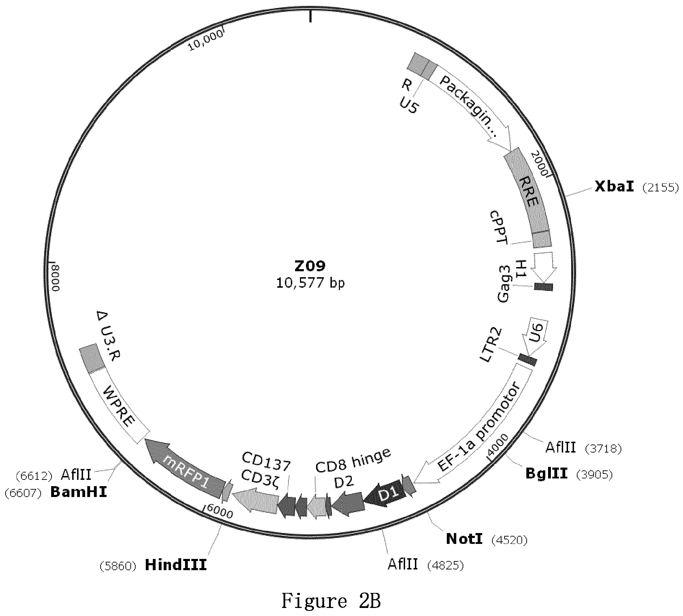

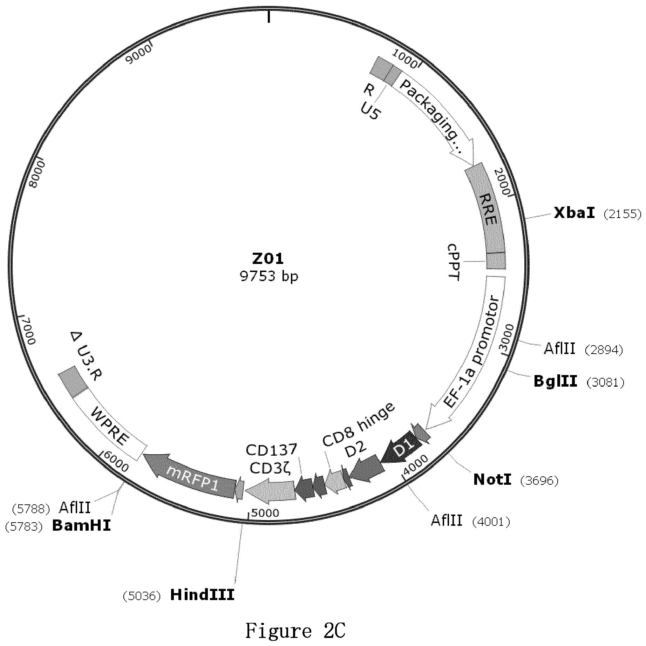

FIGS. 2A-2C show the structure of three representative plasmids herein, wherein FIG. 2A shows the structure of original pGTV-PEDF plasmid, FIG. 2B shows the structure of Z09 plasmid, and FIG. 2C shows the structure Z01 plasmid.

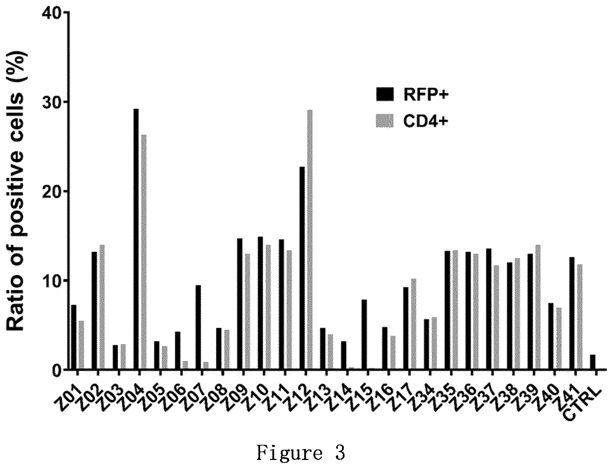

FIG. 3 shows the flow cytometry result based on CAR molecules or intracellular reporter genes after the recombinant lentivirus SIV carrying different CAR molecules is transduced to 293T cells. The CD4+ refers to CD4 positive cells obtained by performing flow cytometric detection and analysis using anti-human CD4 antibody on transduced 293T. RFP+ refers to the cells positive for monomer red fluorescent protein mRFP that is co-expressed with CAR molecules, detected by direct flow cytometry. CTRL is 293T cells not transduced with recombinant lentivirus SIV.

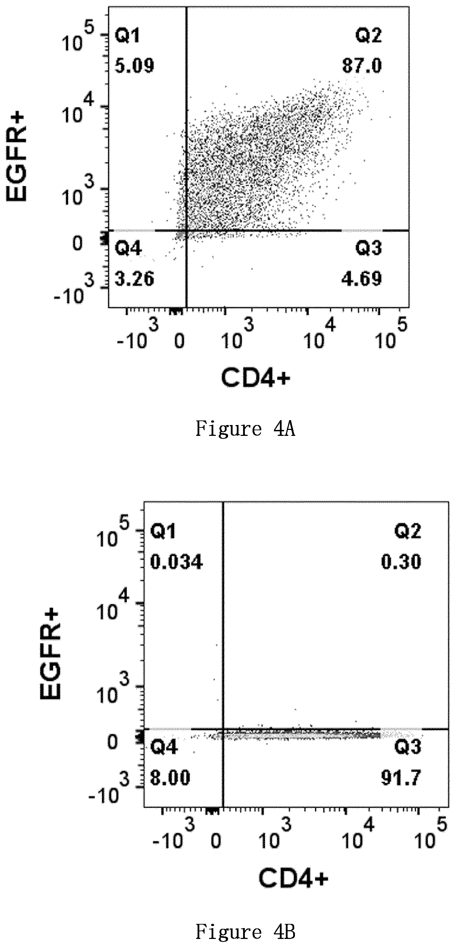

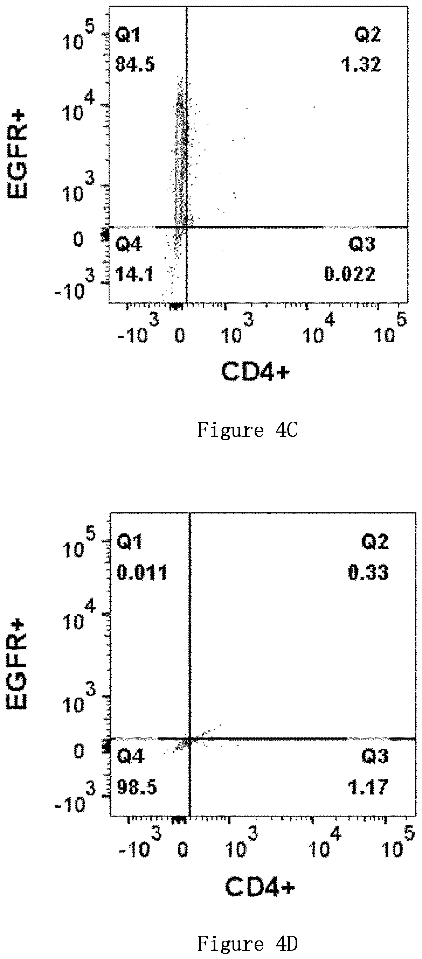

FIG. 4 shows the results of immunofluorescence staining and flow cytometry based on CAR molecules or extracellular reporter gene tEGFR co-expressed with CAR molecules after the recombinant lentivirus SIV-Z09 is transduced to 293T cells, wherein FIG. 4A shows two antibodies double-staining result, FIG. 4B shows anti-CD4 antibody single-staining result, FIG. 4C shows anti-EGFR antibody single-staining result, and FIG. 4D shows a non-staining control. The CD4+ refers to CD4 positive cells obtained by performing immunofluorescence staining and flow cytometry analysis using anti-human CD4 antibody on the transduced 293T, and EGFR+ refers to EGFR positive cells obtained by immunofluorescence staining and flow cytometry analysis using anti-human EGFR antibody on the transduced 293T.

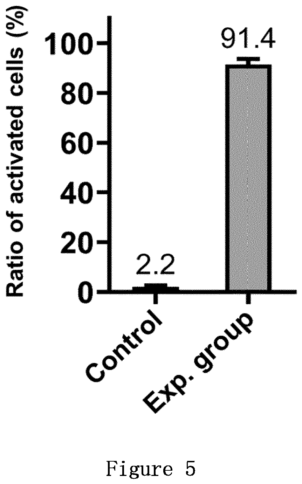

FIG. 5 shows the flow cytometric detection result after the CD3+ T cells are activated for 48 hours through CD3/CD28 magnetic beads and cytokines. The ratio of activated cells refers to the ratio of CD3+CD25+ cells in CD3+ T cells. The control group refers to CD3+ T cells which are not activated by magnetic beads and cytokines, and the experimental (Exp.) group refers to CD3+ T cells activated by magnetic beads and cytokines.

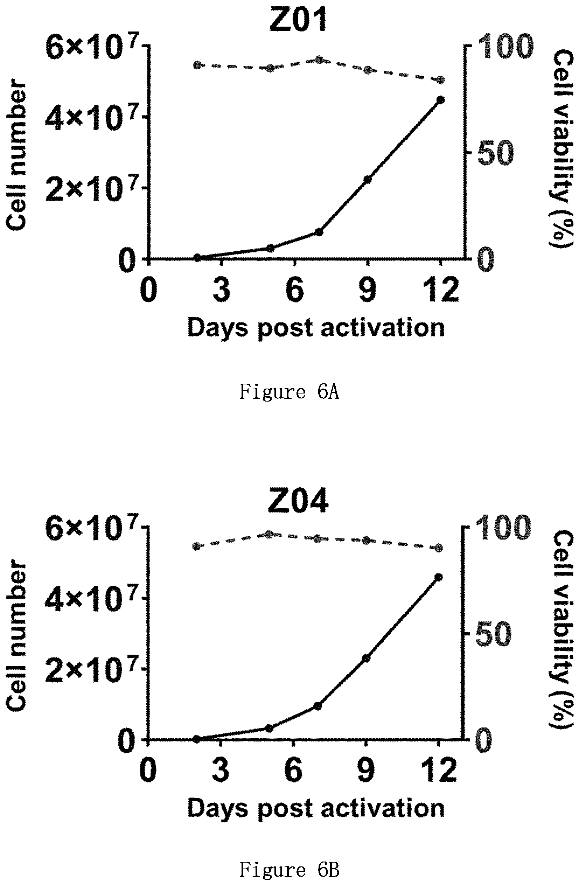

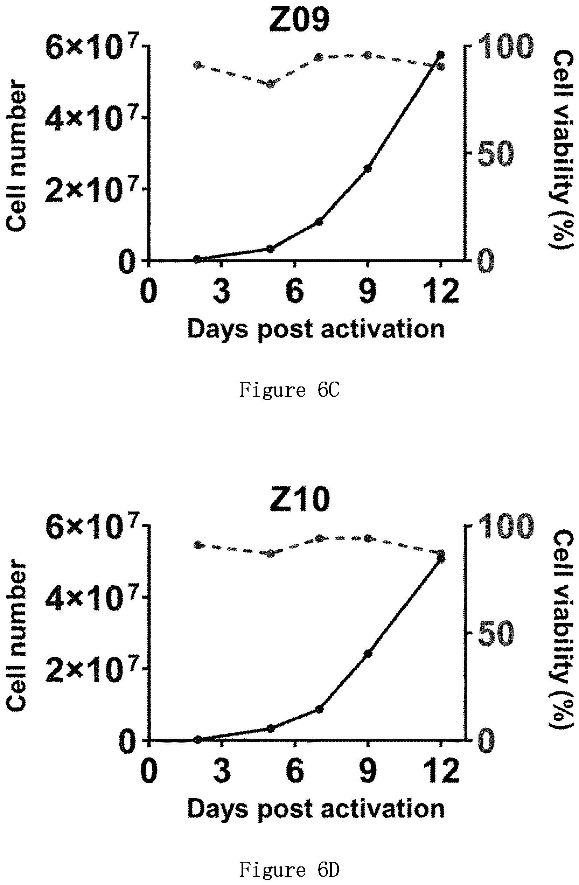

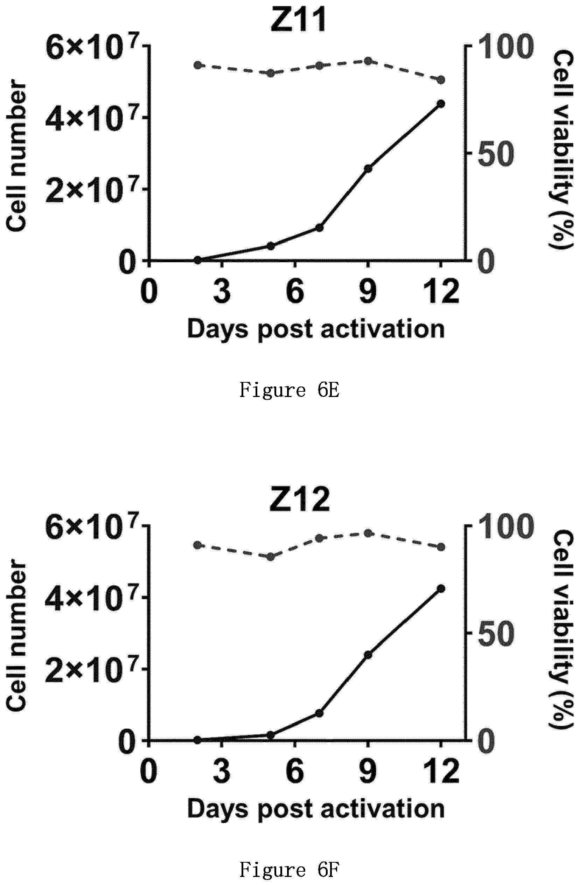

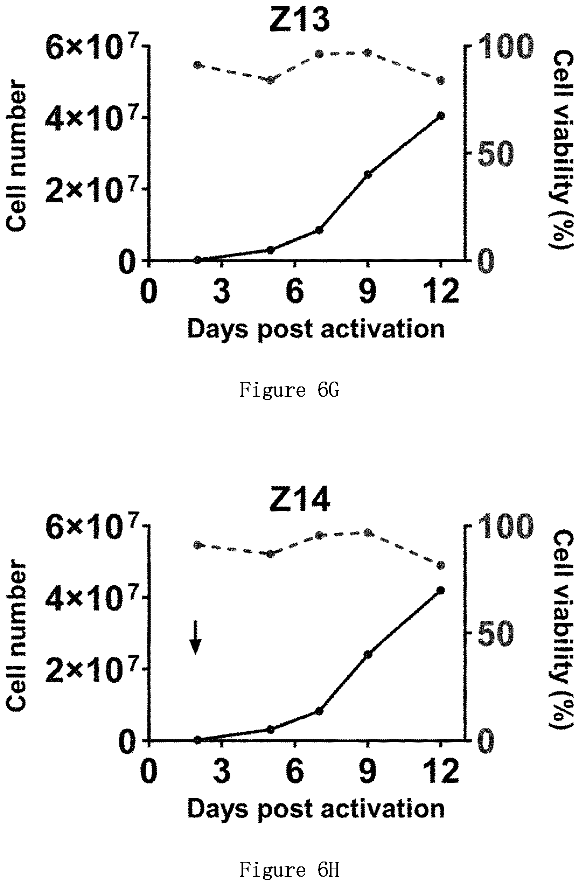

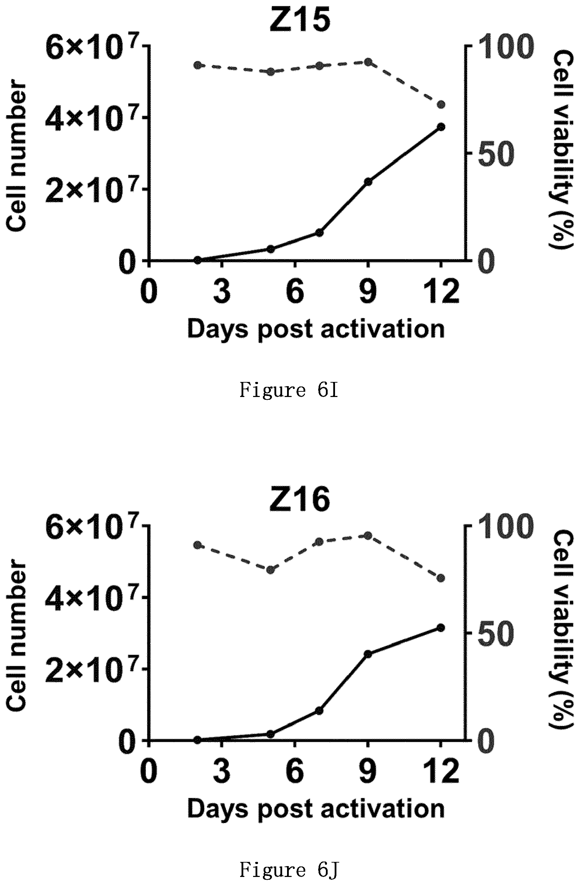

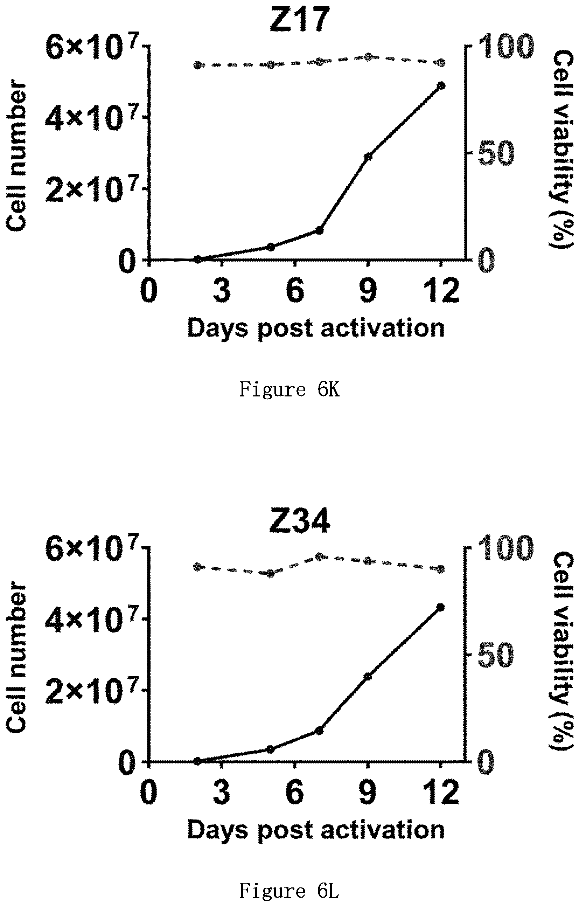

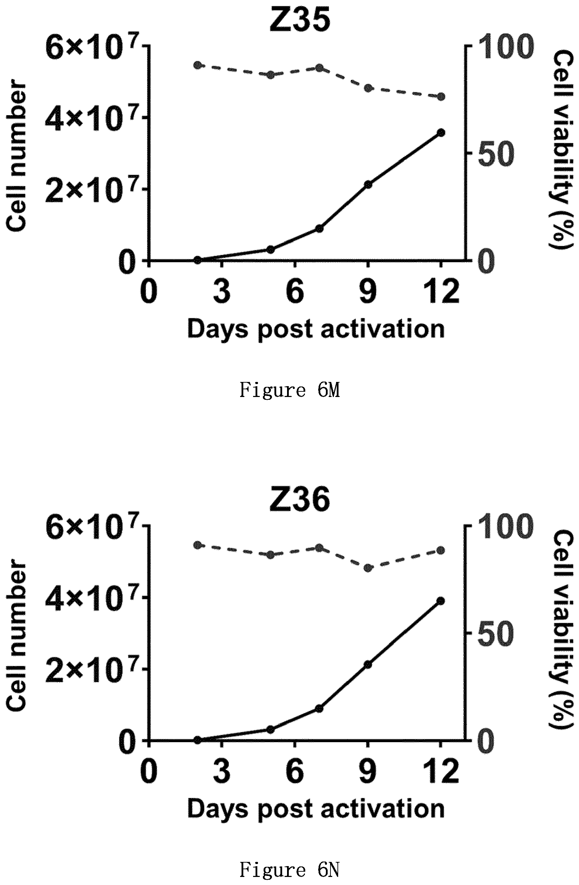

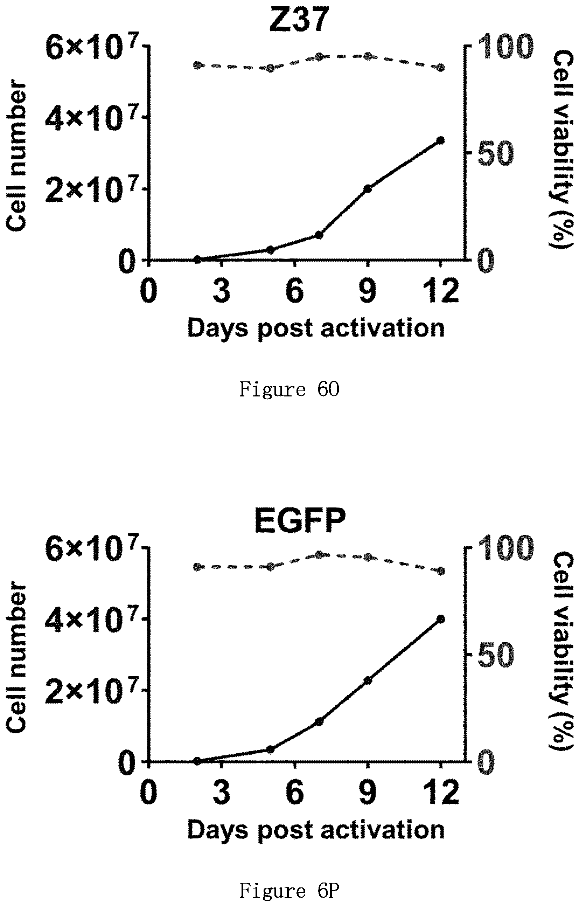

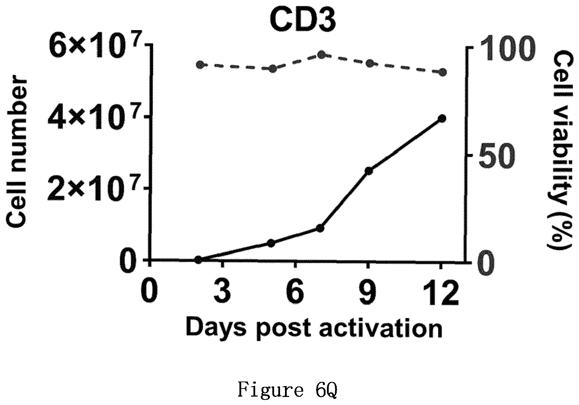

FIG. 6 shows the results of cell number and cell viability of CD3+ T cells during 12 days after activation. 48 hours after activation, CD3+ T cells were transduced with the recombinant lentivirus SIV. The solid lines in the graph represent the cell number and correspond to the left Y-axis. The dotted lines represent the cell viability and correspond to the right Y-axis. EGFP refers to CD3+ T cells transduced with recombinant lentivirus SIV-EGFP, and CD3 represents CD3+ T cells which are not transduced with any recombinant lentivirus SIV. The names of the CAR molecules carried in the recombinant lentivirus SIV are respectively marked above the FIG. 6A-6Q.

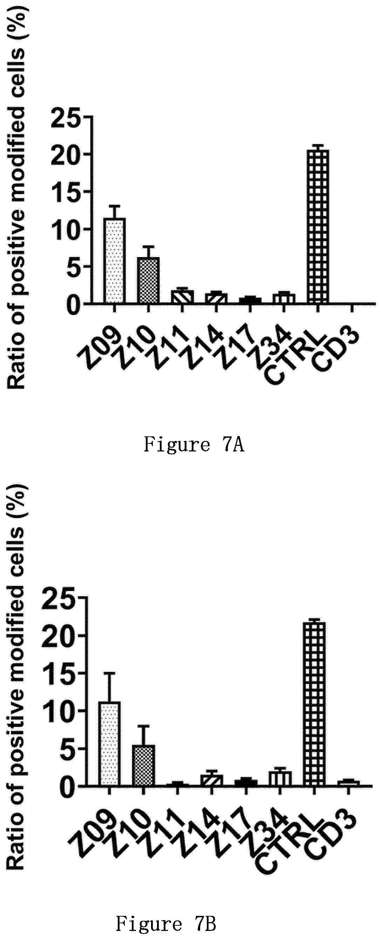

FIG. 7 shows the results of flow cytometric detection and analysis based on the surface marker and intracellular reporter gene of modified CD3+ T cells. FIG. 7A shows the ratio of positive cells in the 13th day after activation, and FIG. 7B shows the ratio of positive cells in the 19th day after activation. The ratio of the modified positive cells is obtained through flow cytometric detection and analysis based on reporter gene; CTRL refers to CD3+ T cells transduced with recombinant lentivirus SIV-EGFP, and CD3 refers to CD3+ T cells which are not transduced with any recombinant lentivirus SIV.

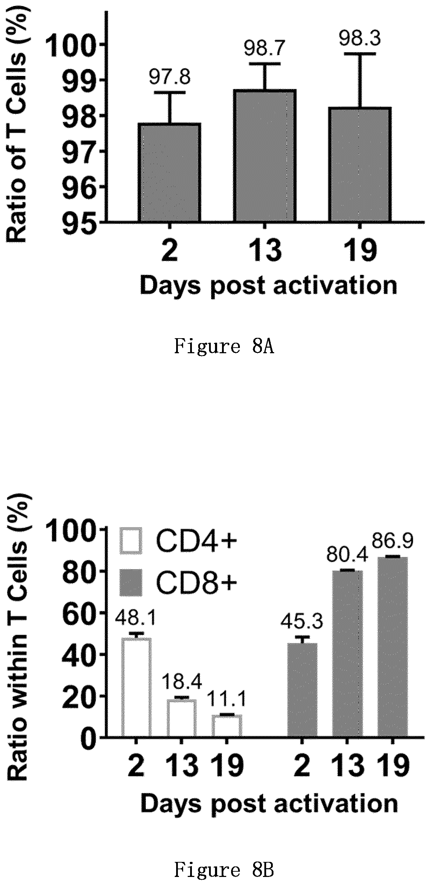

FIG. 8 shows the results of flow cytometric detection and analysis based on the surface markers of non-transduced CD3+ T cells. FIG. 8A shows the ratio of CD3+ T cells at different time points, and FIG. 8B shows the ratio of CD4+ T cells and CD8+ T cells at different time points.

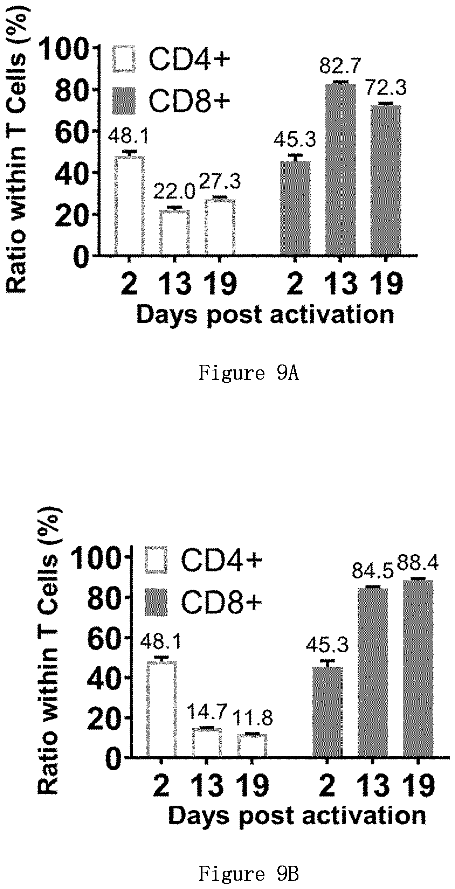

FIG. 9 shows the results of flow cytometric detection and analysis based on the surface markers of transduced CD3+ T cells. FIG. 9A shows the ratio of CD4+ T cells and CD8+ T cells, respectively, after CD3+ T cells was transduced with recombinant lentivirus SIV-Z09 (expressing CD4 extracellular region), and FIG. 9B shows the ratio of CD4+ T cells and CD8+ T cells, respectively, after CD3+ T cells was transduced with recombinant lentivirus SIV-Z14 (not expressing CD4 extracellular region).

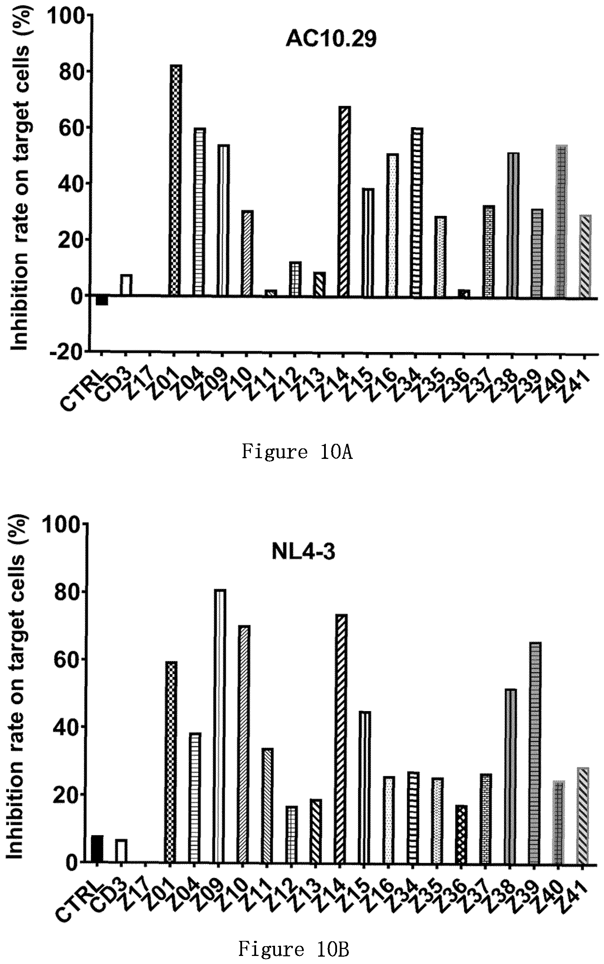

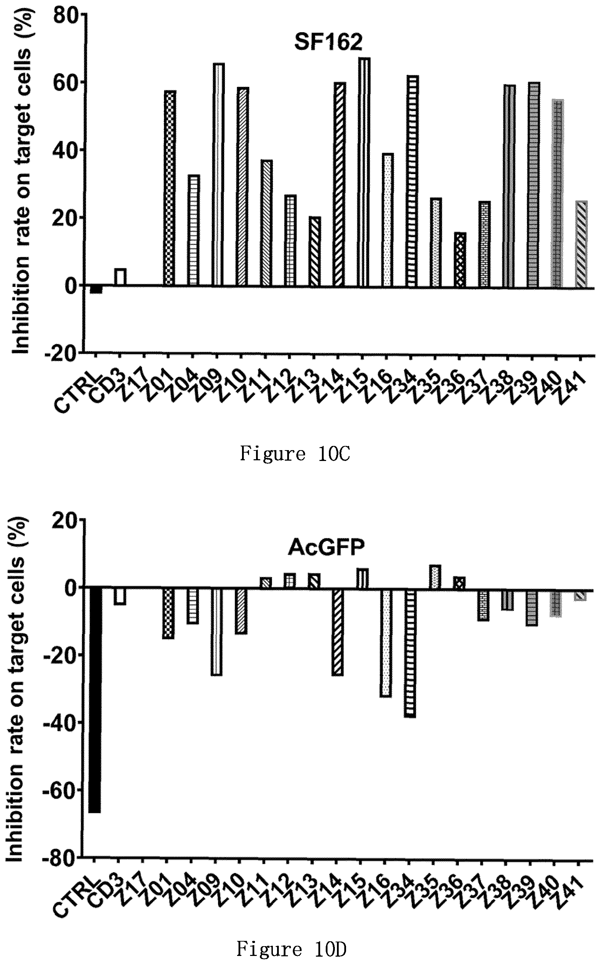

FIG. 10 shows the results of flow cytometric detection and analysis on the modified CD3+ T cells after mixed with target cells in a fixed ratio of 3: 1. All of the target cells are 293F cells, obtained by transfecting with the pAcGFP plasmids expressing the membrane protein gp120 of different HIV virus strains respectively and screening by G418, and the original green fluorescent protein AcGFP in the plasmid was co-expressed with gp120 by using P2A. In FIG. 10A, target cells express gp120 of HIV virus strain AC10.29. In FIG. 10B, target cells express gp120 of HIV virus strain NL4-3. In FIG. 10C, target cells express gp120 of HIV virus strain SF162. In FIG. 10D, target cells express only AcGFP. The X-axis is labeled with corresponding CAR molecular structure in modified CD3+ T cells. CTRL refers to CD3+ T cells transduced with recombinant lentivirus SIV-EGFP, and CD3 refers to CD3+ T cells that are not transduced with any recombinant lentivirus SIV. The number of target cells mixed for 24 hours was calculated and detected based on the result of flow cytometric detection of GFP+ CD3- 293F cells. Then relative reduction of the number of target cells in each experimental group compared to that in the group where CD3+ T cells were transduced with recombinant lentivirus SIV-Z17 was respectively calculated, as the inhibition rate of each sample to the target cells.

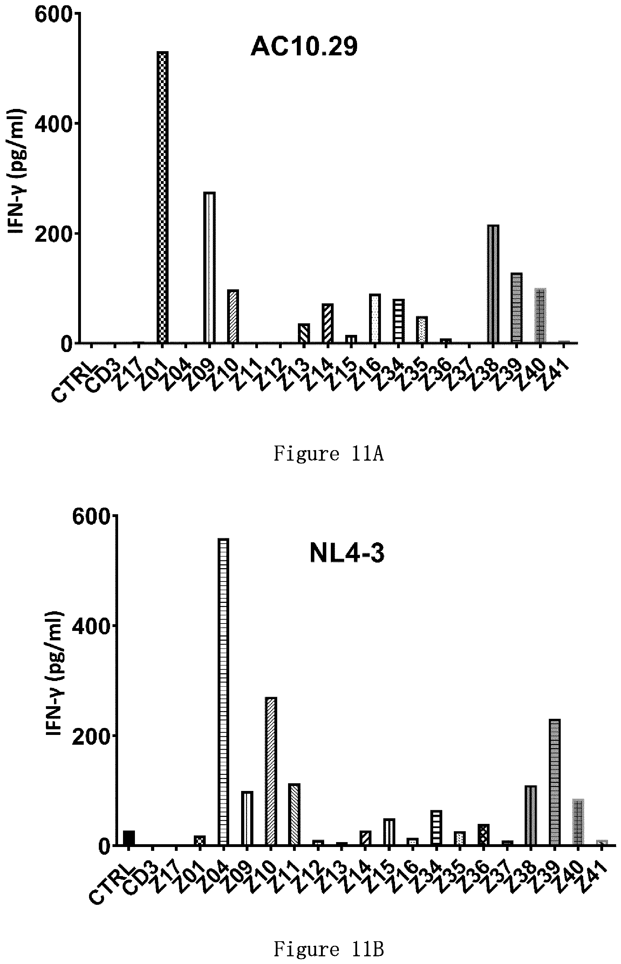

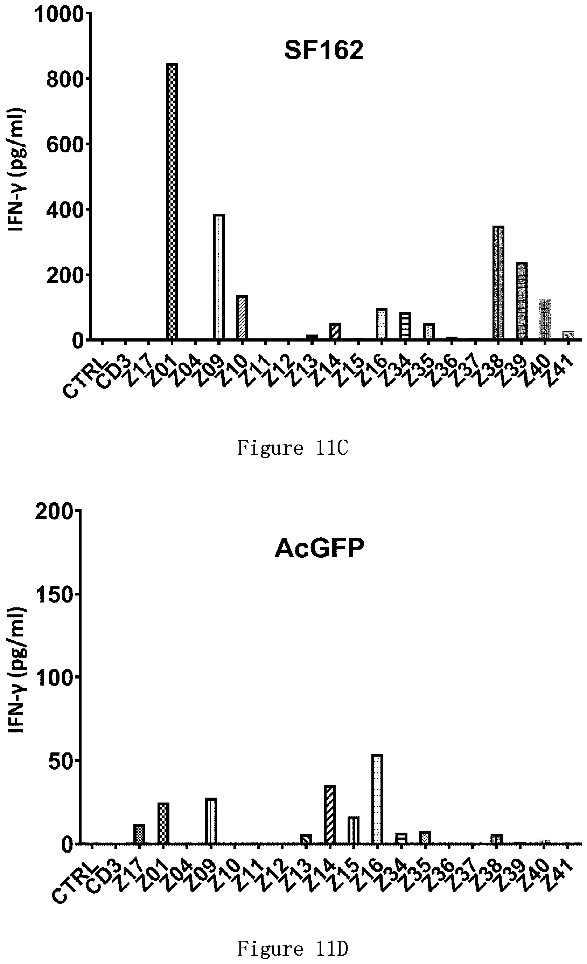

FIG. 11 shows the results of detection and analysis of the cytokine IFN-γ in the supernatant 24 hours after the modified CD3+ T cells were mixed with the target cells at a fixed ratio of 3:1. In FIG. 11A, target cells express gp120 of HIV virus strain AC10.29. In FIG. 11B, target cells express gp120 of HIV virus strain NL4-3. In FIG. 11C, target cells express gp120 of HIV virus strain SF162. In FIG. 11D, target cells only express AcGFP. The X-axis is labeled with corresponding CAR molecular structure in modified CD3+ T cells. CTRL refers to CD3+ T cells transduced with recombinant lentivirus SIV-EGFP, and CD3 refers to CD3+ T cells which are not transduced with any recombinant lentivirus SIV.

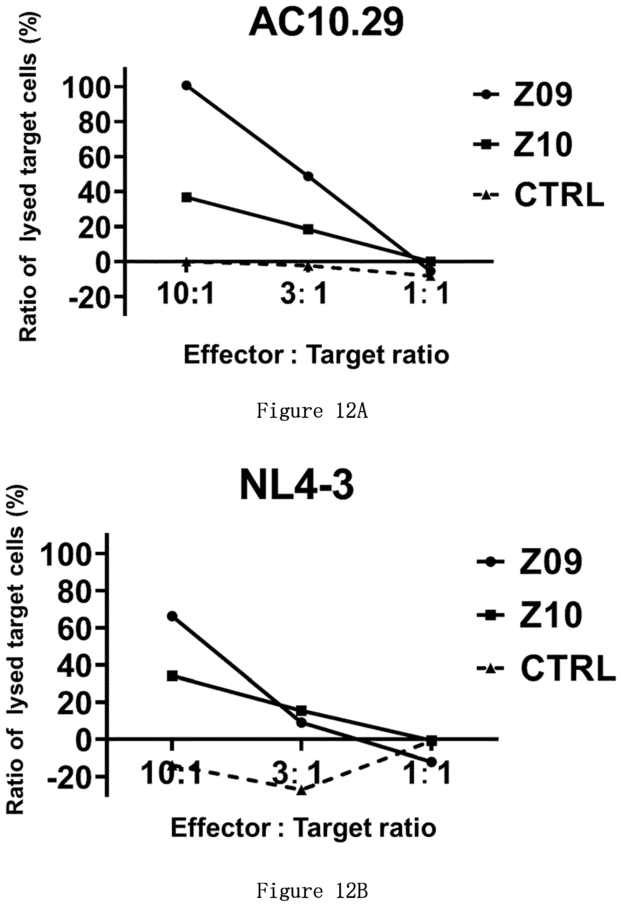

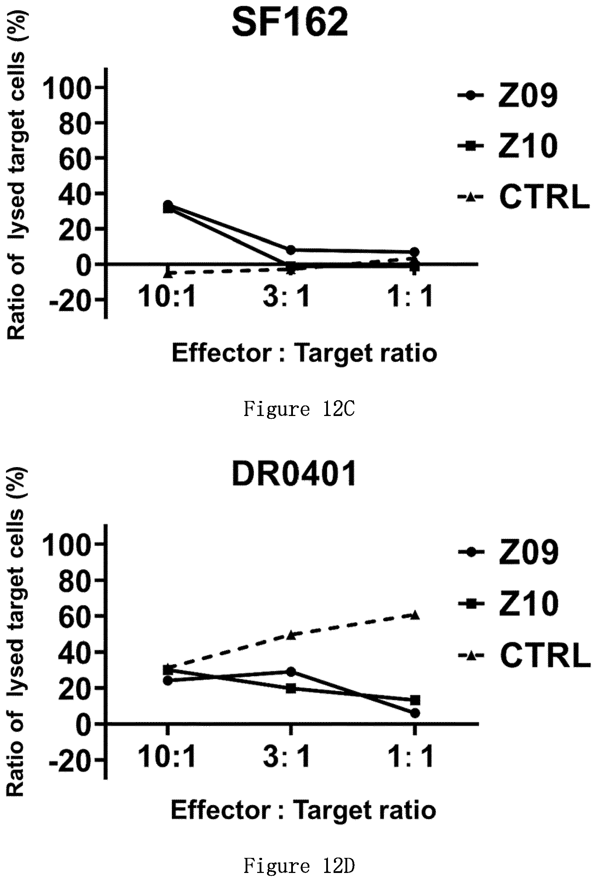

FIG. 12 shows the results of acute killing activity against target cells after the modified CD3+ T cells were mixed with target cells in different ratios. The content of lactate dehydrogenase LDH in the supernatant 4 hours after mixing was detected and calculated by using CytoTox 96 non-radioactive cytotoxicity detection kit. In FIG. 12A, target cells express gp120 of HIV virus strain AC10.29. In FIG. 12B, target cells express gp120 of HIV virus strain NL4-3, In FIG. 12C, target cells express gp120 of HIV virus strain SF162. In FIG. 12D, target cells express human leukocyte antigen DR0401. The icons Z09, Z10 and CTRL indicate structures used in corresponding modified cells, wherein Z09 and Z10 indicate CD3+ T cells transduced with recombinant lentivirus SIV-Z09 and SIV-Z10 respectively, and CTRL is CD3+ T cells transduced with recombinant lentivirus SIV-EGFP.

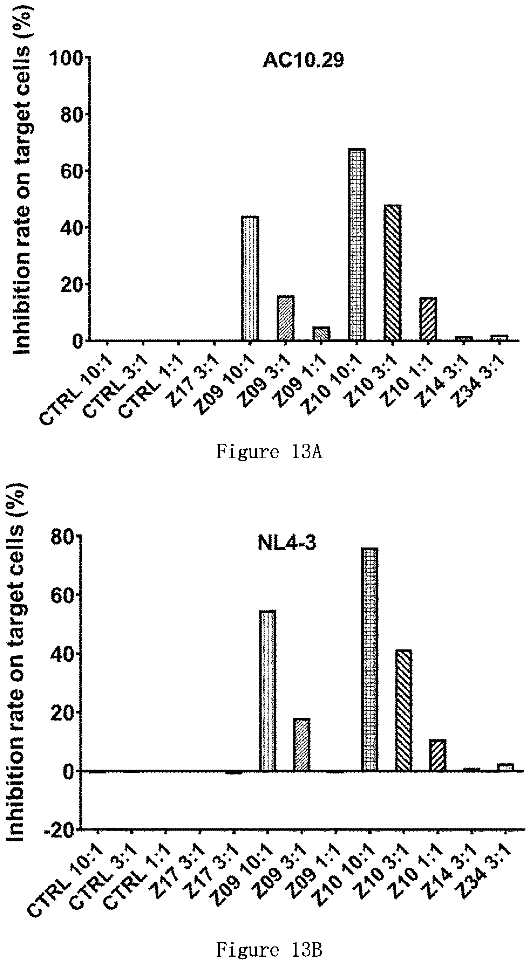

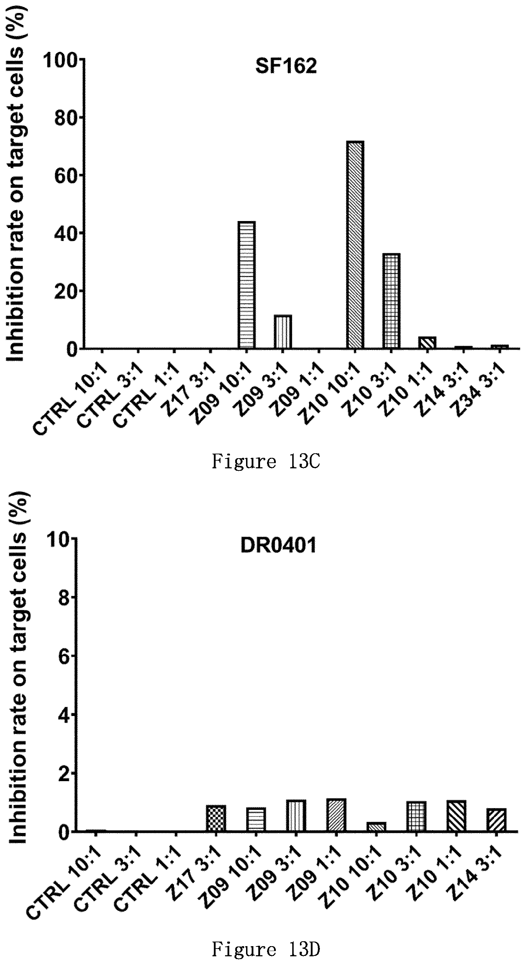

FIG. 13 shows the results of the inhibitory effect on target cells 24 hours after the modified CD3+ T cells were mixed with target cells in different ratios. 24 hours after mixing, the results of flow cytometric detection and analysis on GFP+CD3- 293F cells were used to calculate the number of target cells mixed for 24 hours, and then relative reduction of the number of target cells in each experimental group compared to that in CTRL control with 1: 1 effector: target ratio was respectively calculated as the inhibition rate of each sample to the target cells. In FIG.13A, target cells express gp120 of HIV virus strain AC10.29. In FIG. 13B, target cells express gp120 of HIV virus strain NL4-3. In FIG. 13C, target cells express gp120 of HIV virus strain SF162. In FIG. 13D, target cells express human leukocyte antigen DR0401. CTRL is CD3+ T cells transduced with recombinant lentivirus SIV-EGFP.

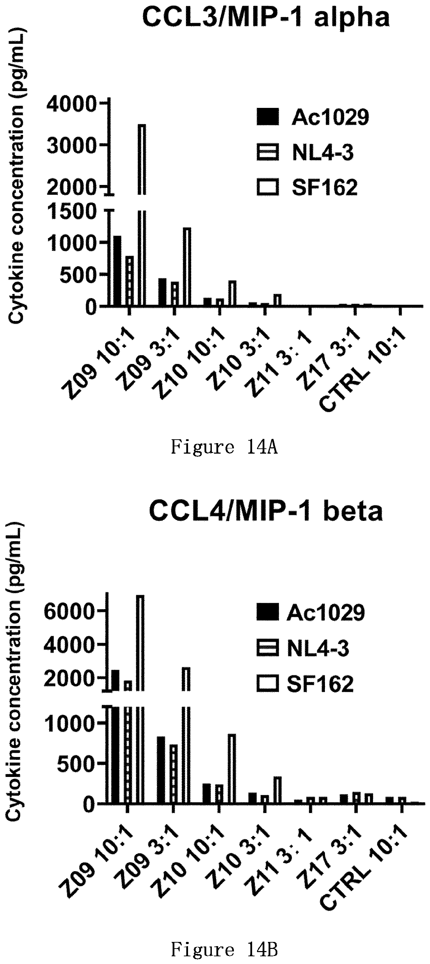

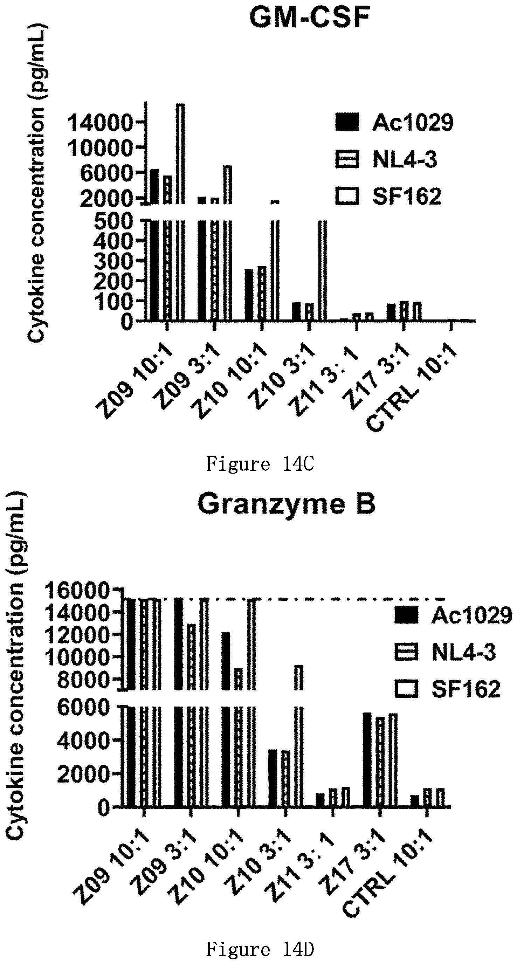

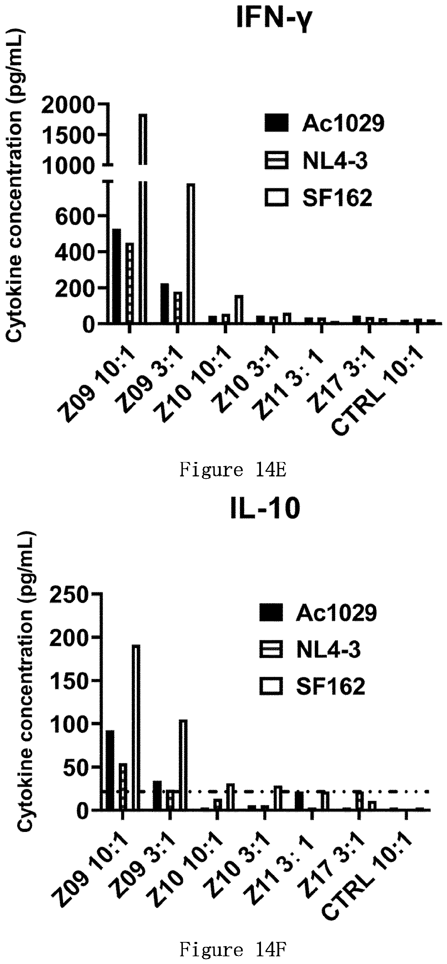

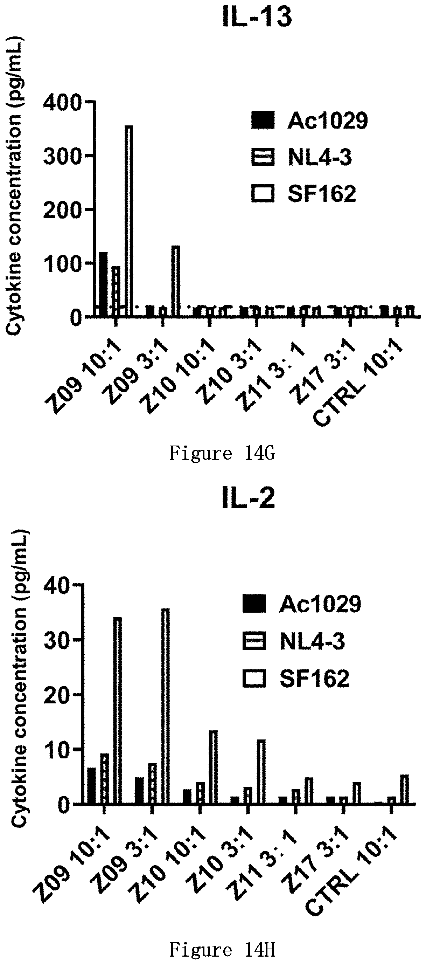

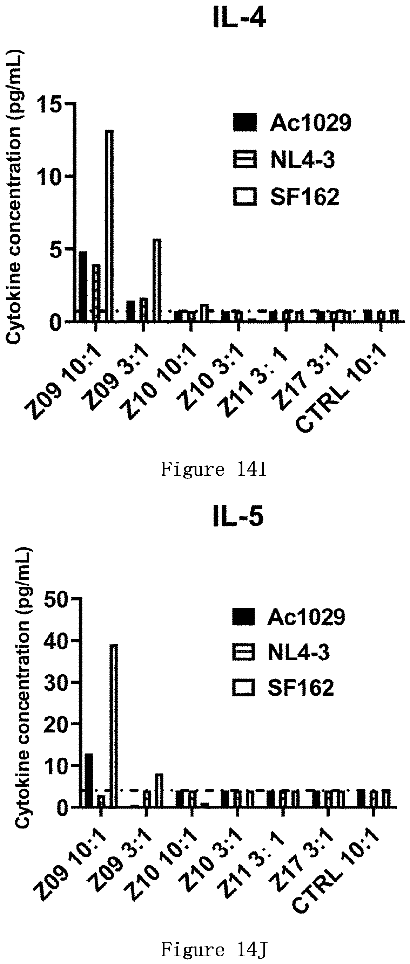

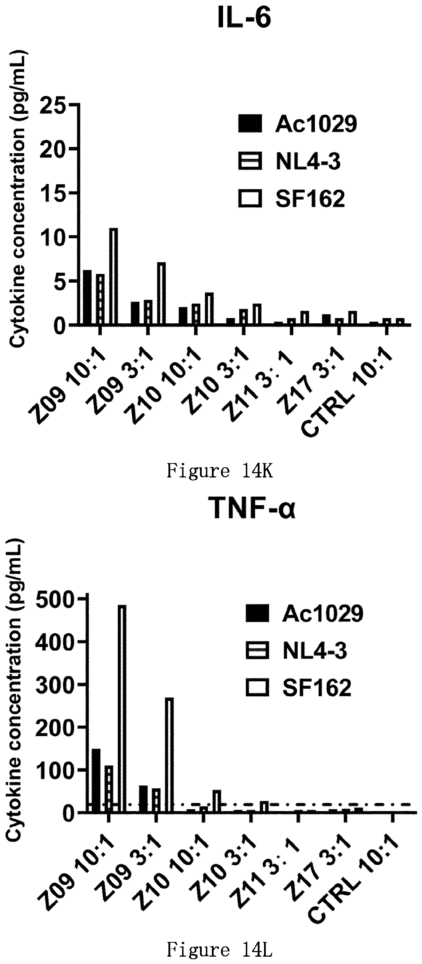

FIG. 14 shows the results of the detection of multiplex cytokines in the culture supernatant after the modified CD3+ T cells were mixed with target cells in different ratios. 24 hours after mixing, the levels of 12 cytokines in supernatant were detected simultaneously by Luminex (FIGS. 14A-14L). The HIV virus gp120 of corresponding target cell is shown in the figure, and the corresponding structure and effector: target ratio of the modified cell are shown on the X-axis. CTRL refers to the CD3+ T cell transduced with recombinant lentivirus SIV-EGFP. The dotted line parallel to the X-axis is the detection threshold.

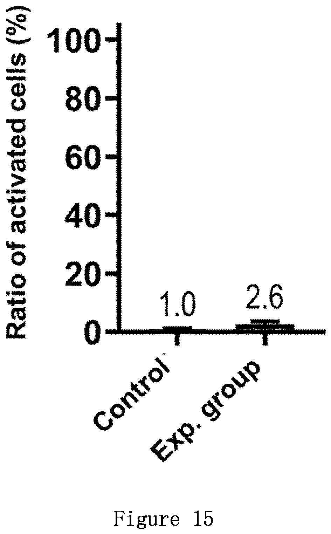

FIG. 15 shows the results of flow cytometry of CD3+ T cells on day 19 after activation. The ratio of activated cells refers to the ratio of CD3+CD25+ cells in CD3+ T cells. The control group refers to unstained CD3+ T cells, and the experimental group refers to CD3+ T cells detected after staining.

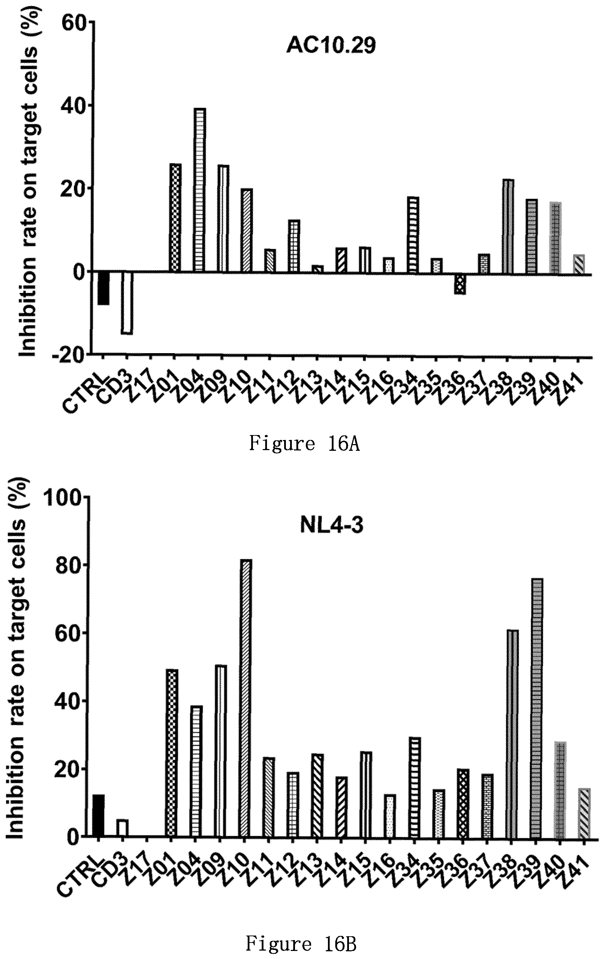

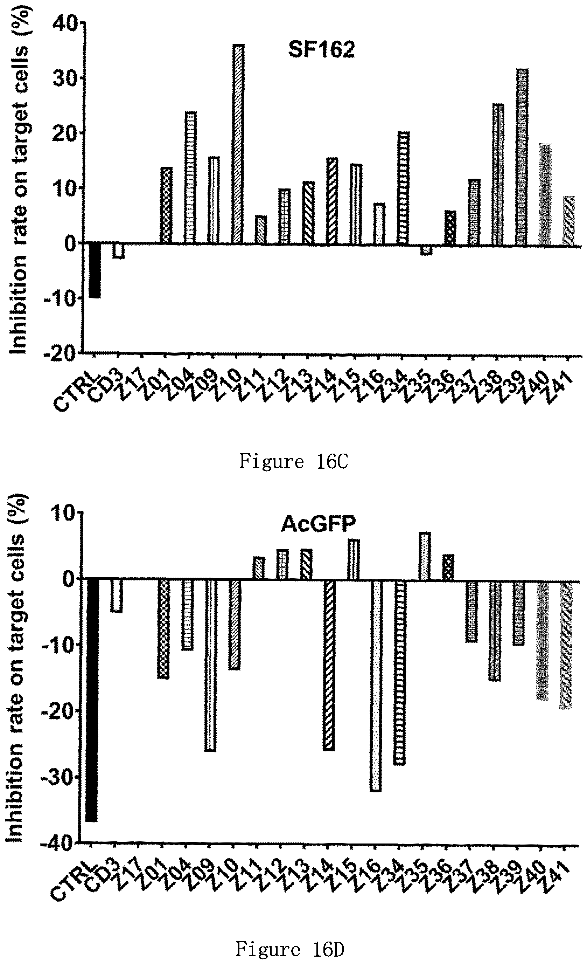

FIG. 16 shows the results of flow cytometric detection and analysis on the modified CD3+ T cells after mixed with target cells at a fixed ratio of 3:1 and extended culture. In FIG. 16A, target cells express gp120 of HIV virus strain AC10.29. In FIG. 16B, target cells express gp120 of HIV virus strain NL4-3. In FIG. 16C, target cells express gp120 of HIV virus strain SF162. In FIG. 16D, target cells express only AcGFP. The X-axis is labeled with the CAR molecular structure in corresponding modified CD3+ T cells. CTRL refers to CD3+ T cells transduced with recombinant lentivirus SIV-EGFP, and CD3 refers to CD3+ T cells which are not transduced with any recombinant lentivirus SIV. The number of target cells mixed for 24 hours was calculated and detected based on the result of flow cytometric detection of GFP+CD3- 293F cells. Then relative reduction of the number of target cells in each experimental group sample compared to that in the group where CD3+ T cells were transduced with recombinant lentivirus SIV-Z17 was respectively calculated as the inhibition rate of each sample to the target cells.

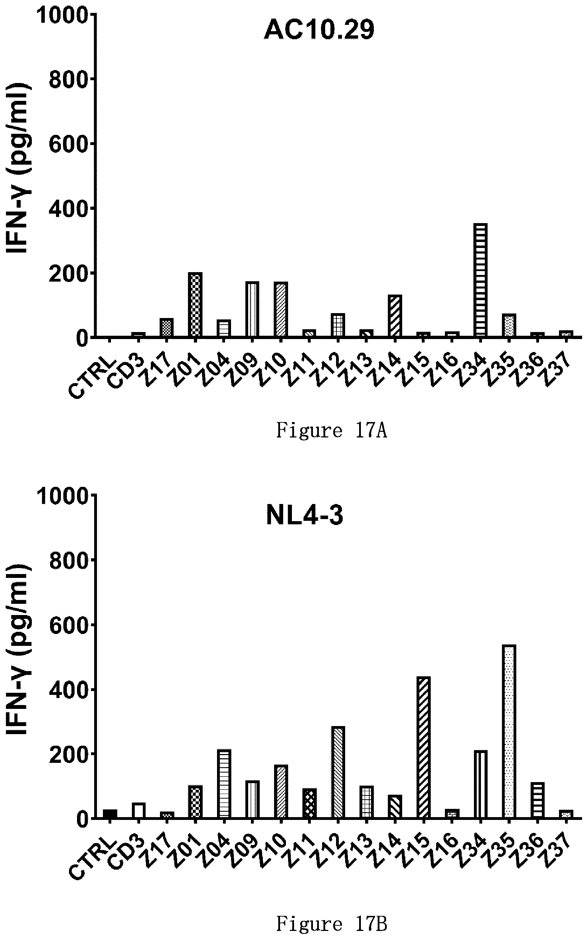

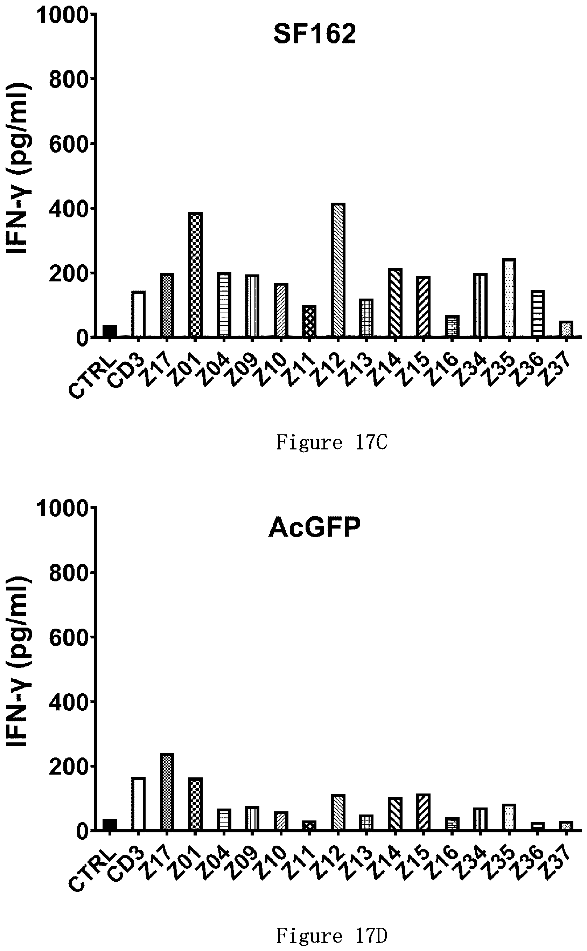

FIG. 17 shows the results of detection and analysis on the culture supernatant of the modified CD3+ T cells after mixed with target cells at a fixed ratio of 3:1 and extended culture. The cytokine IFN-γ in the supernatant was detected and analyzed by ELISA 24 hours after mixing. In FIG. 17A, target cells express gp120 of HIV virus strain AC10.29. In FIG. 17B, target cells express gp120 of HIV virus strain NL4-3. In FIG. 17C, target cells express gp120 of HIV virus strain SF162. In FIG. 17D, target cells express only AcGFP. The X-axis is labeled with corresponding CAR molecular structure in modified CD3+ T cells, CTRL refers to the CD3+ T cells transduced with recombinant lentivirus SIV-EGFP, and CD3 refers to the CD3+ T cells which are not transduced with any recombinant lentivirus SIV.

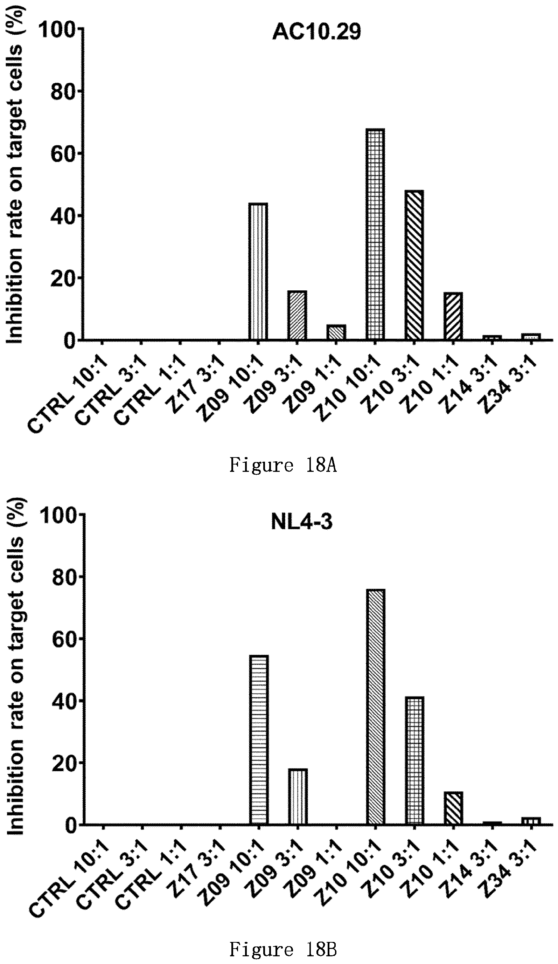

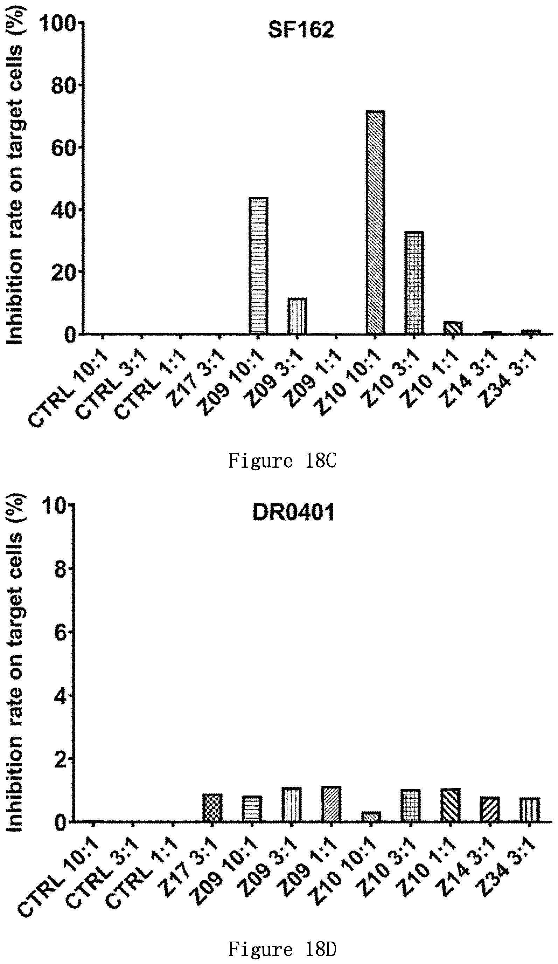

FIG. 18 shows the results of flow cytometric detection and analysis on the modified CD3+ T cells 24 hours after extended culture and then mixed with target cells in different ratios. The results of flow cytometric detection and analysis on GFP+CD3- 293F cells were used to calculate the number of target cells mixed for 24 hours, and then relative reduction of the number of target cells in each experimental group compared to that in CD3+ T cell sample transduced with recombinant lentivirus SIV-EGFP with 1: 1 effector: target ratio was respectively calculated as the inhibition rate of each sample to the target cells. In FIG. 18A, target cells express gp120 of HIV virus strain AC10.29. In FIG. 18B, target cells express gp120 of HIV virus strain NL4-3. In FIG. 18C, target cells express gp120 of HIV virus strain SF162. In FIG. 18D, target cells express human leukocyte antigen DR0401 (MHCII). CTRL refers to CD3+ T cells transduced with recombinant lentivirus SIV-EGFP, and CD3 refers to CD3+ T cells not transduced with recombinant lentivirus SIV.

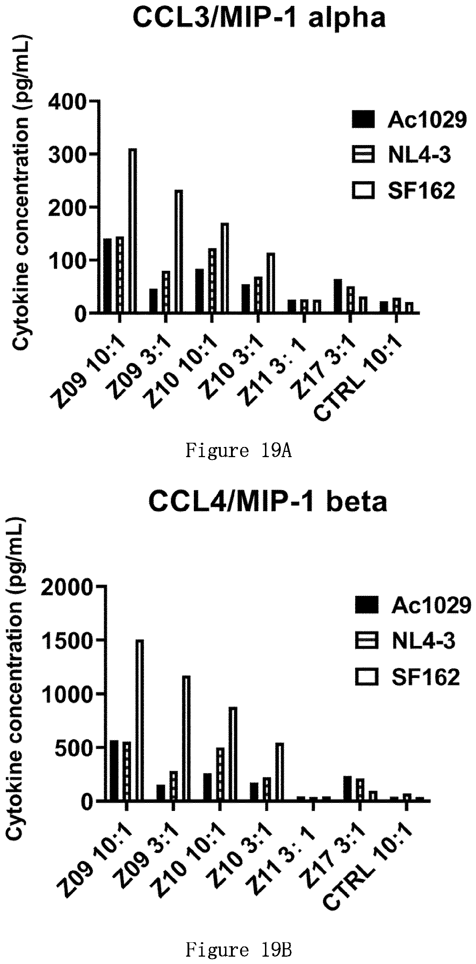

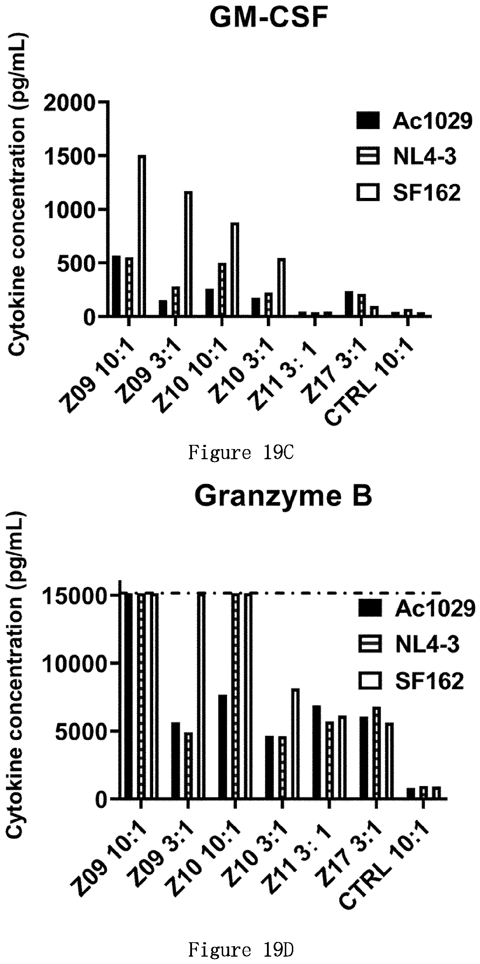

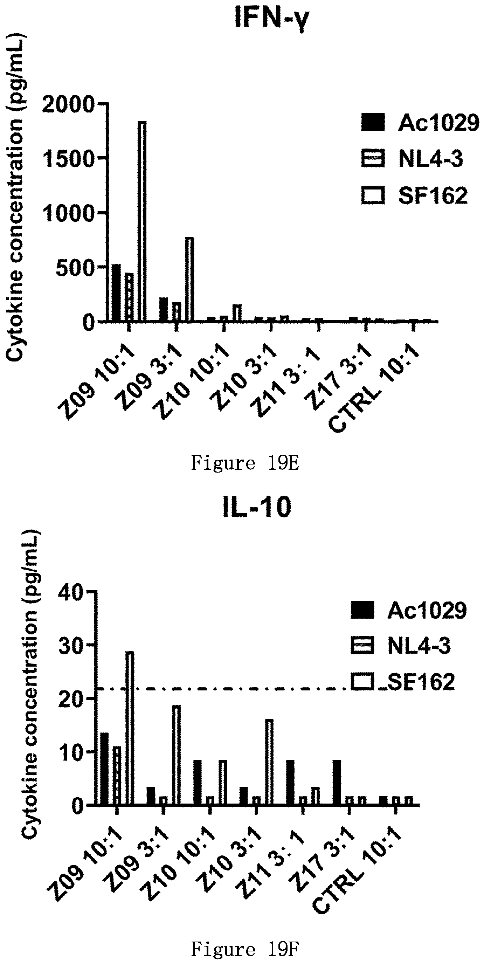

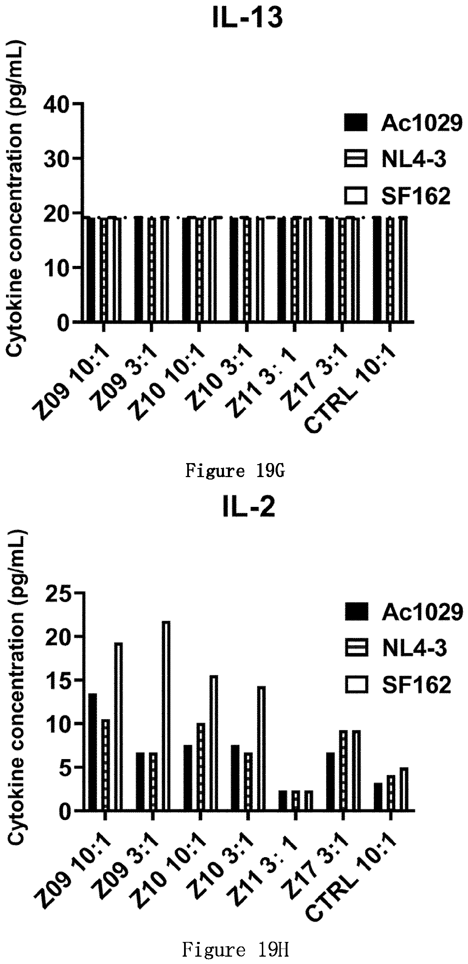

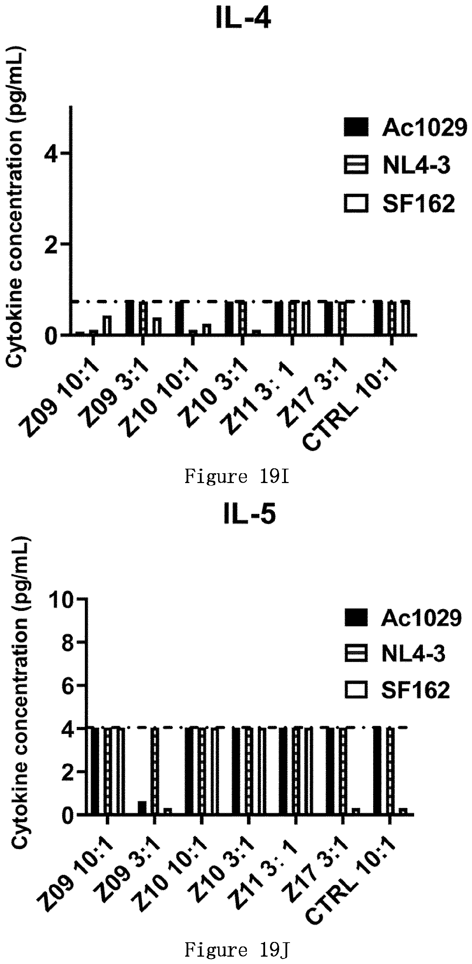

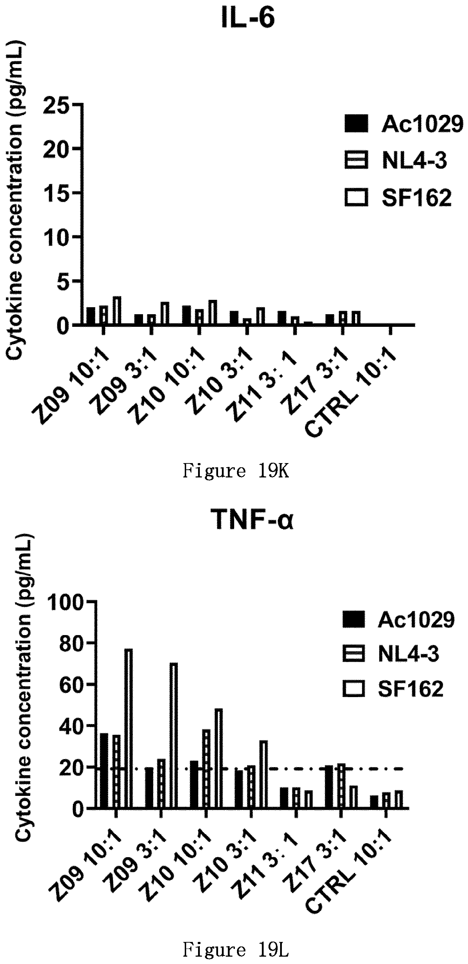

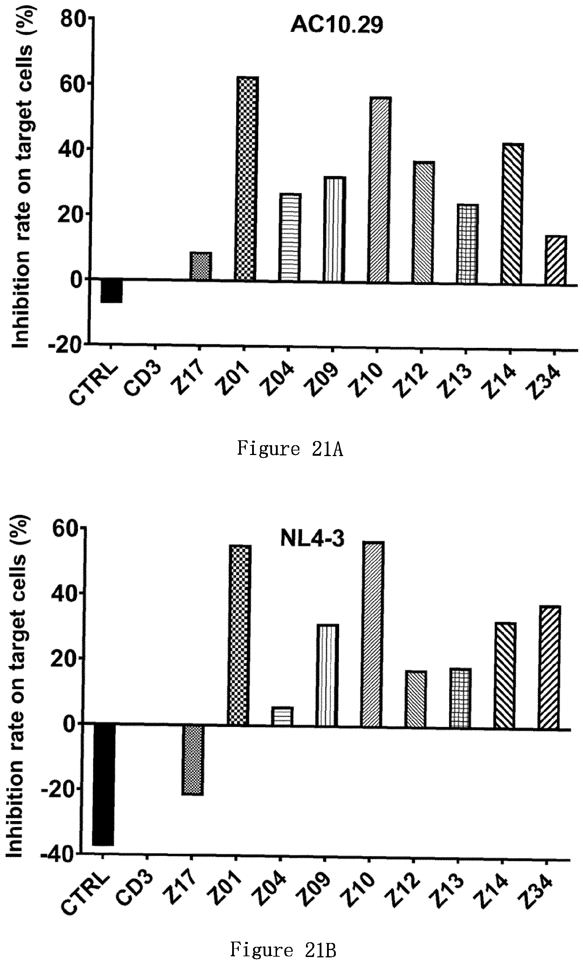

FIG. 19 shows the results of detection of multiplex cytokine in the culture supernatant after the modified CD3+ T cells were mixed with target cells at a fixed ratio of 3:1. 24 hours after mixing, the levels of 12 cytokines in supernatant were detected simultaneously by Luminex (FIGS. 21A-21L). The HIV virus gp120 of corresponding target cell is shown in the figure, and the corresponding structure and the effector: target ratio of the modified cell are shown on the X-axis. CTRL refers to the CD3+ T cell transduced with recombinant lentivirus SIV-EGFP. The dotted line parallel to the X-axis is the detection threshold.

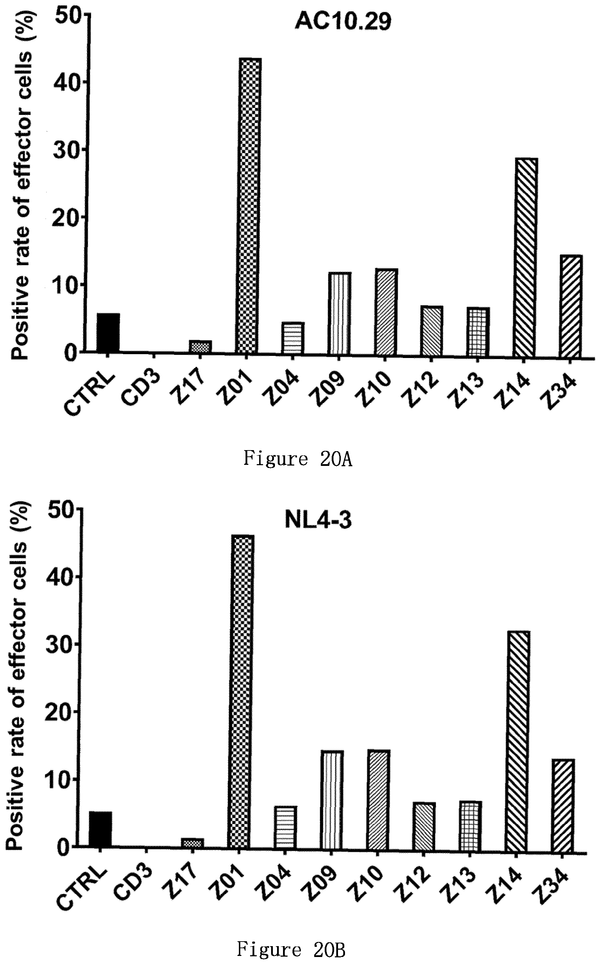

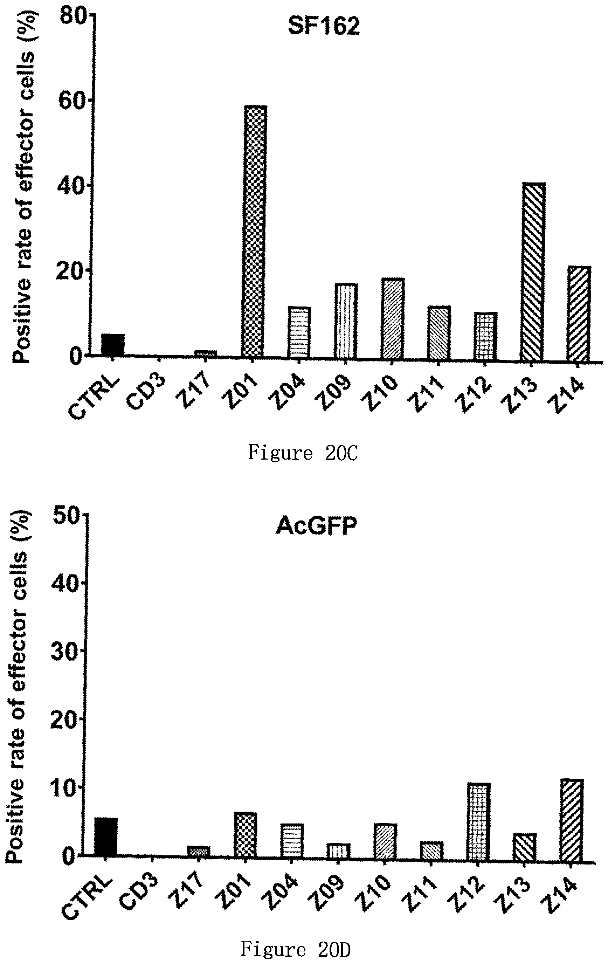

FIG. 20 shows the results of flow cytometric detection and analysis on the modified CD3+ T cells after mixed with target cells at a fixed ratio of 3:1 and extended culture. The modified CD3+ T cells were distinguished from target cells based on CD3+, and CAR expression was indicated by fluorescent protein reporter gene. In FIG. 20A, target cells express gp120 of HIV virus strain AC10.29. In FIG. 20B, target cells express gp120 of HIV virus strain NL4-3. In FIG. 20C, target cells express gp120 of HIV virus strain SF162. In FIG. 20D, target cells express only AcGFP. The X-axis is labeled with corresponding CAR molecular structure in modified CD3+ T cells, CTRL refers to the CD3+ T cells transduced with recombinant lentivirus SIV-EGFP, and CD3 refers to the CD3+ T cells which are not transduced with any recombinant lentivirus SIV.

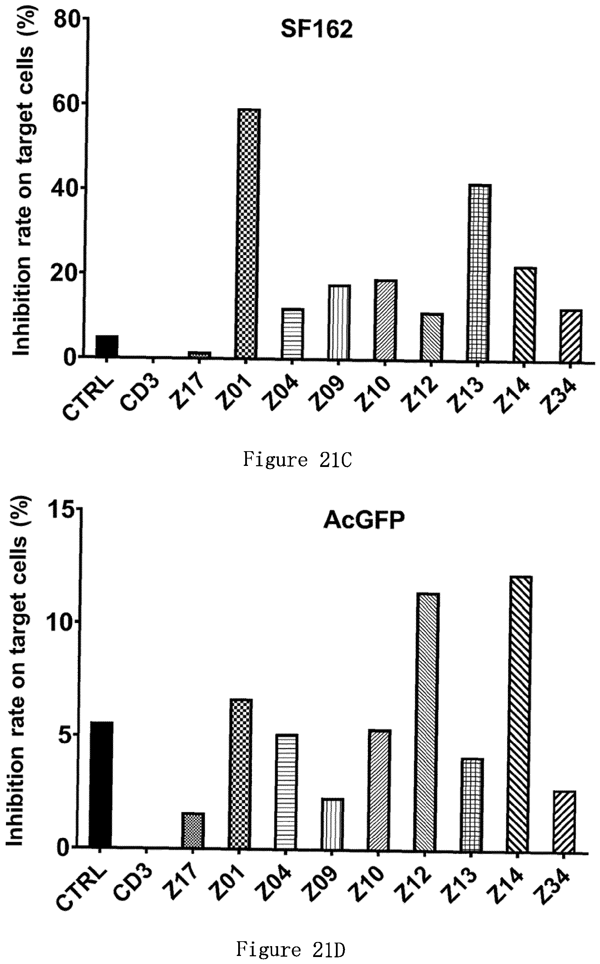

FIG. 21 shows the results of flow cytometric detection and analysis on target cells after the modified CD3+ T cells were mixed with target cells at a fixed ratio of 3:1 and extended culture. Target cells were distinguished from modified CD3+ T cells based on CD3-. In FIG. 21A, target cells express gp120 of HIV virus strain AC10.29. In FIG. 21B, target cells express gp120 of HIV virus strain NL4-3. In FIG. 21C, target cells express gp120 of HIV virus strain SF162. In FIG. 21D, target cells express only AcGFP. The X-axis is labeled with corresponding CAR molecular structure in modified CD3+ T cells, CTRL refers to the CD3+ T cells transduced with recombinant lentivirus SIV-EGFP, and CD3 refers to the CD3+ T cells which are not transduced with any recombinant lentivirus SIV. the number of target cells mixed for 24 hours was calculated and detected based on the result of flow cytometric detection of GFP+CD3- 293F cells. Then relative reduction of the number of target cells in each experimental group compared to that in the CD3 control group was respectively calculated, as the inhibition rate of each sample to the target cells.

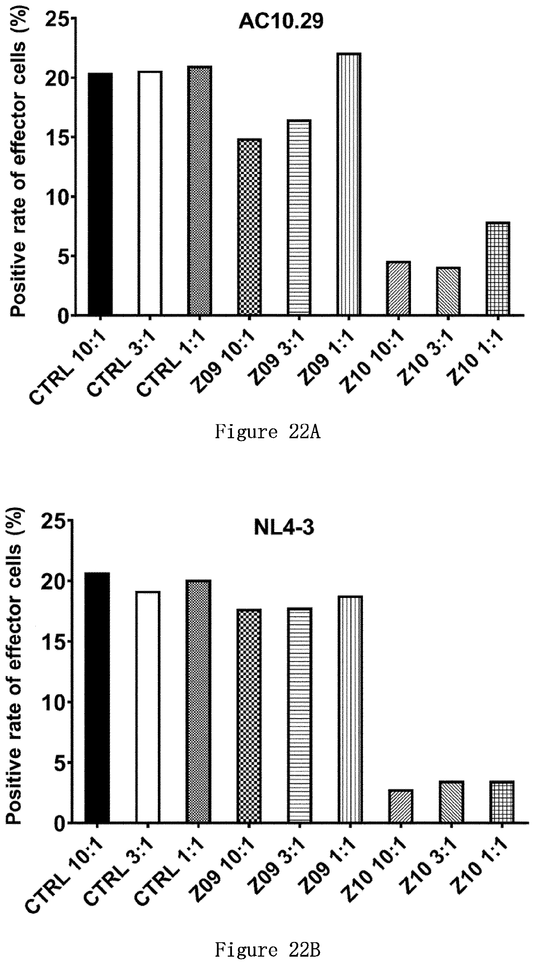

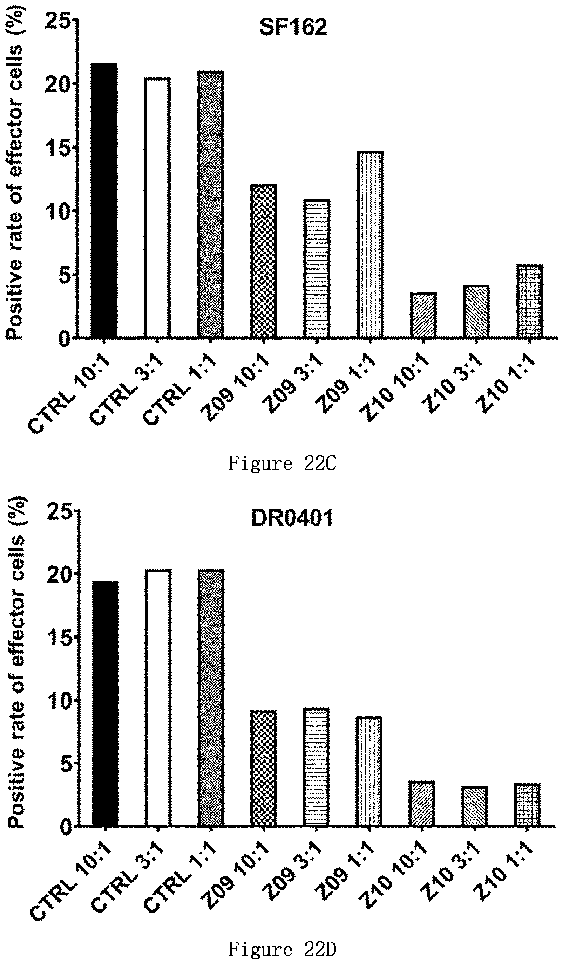

FIG. 22 shows the results of flow cytometric detection and analysis on the modified CD3+ T cells after mixed with target cells in different ratios and extended culture. The different structures and effector: target ratios are labeled on the X-axis, wherein CTRL refers to the CD3+ T cells transduced with recombinant lentivirus SIV-EGFP. The modified CD3+ T cells were distinguished from target cells based on CD3+, and CAR expression was indicated by fluorescent protein reporter gene. In FIG. 22A, target cells express gp120 of HIV virus strain AC10.29. In FIG. 22B, target cells express gp120 of HIV virus strain NL4-3. In FIG. 22C, target cells express gp120 of HIV virus strain SF162. In FIG. 22D, target cells express human leukocyte antigen DR0401.

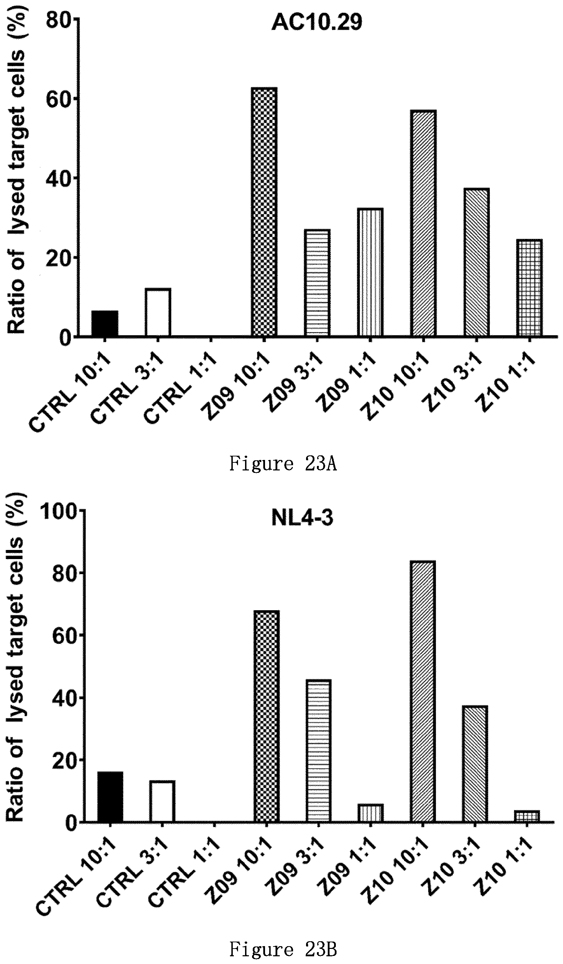

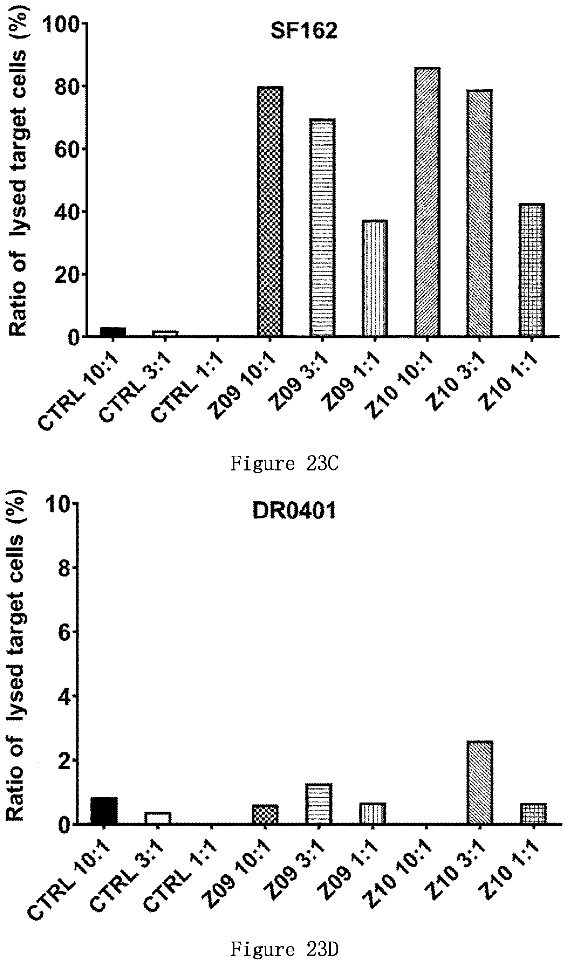

FIG. 23 shows the results of flow cytometric detection and analysis on the target cells after mixed with the modified CD3+ T cells in different ratios and extended culture. The different structures and effector: target ratios are labeled on the X-axis. Target cells were distinguished from modified CD3+ T cells based on CD3-. In FIG. 23A, target cells express gp120 of HIV virus strain AC10.29. In FIG. 23B, target cells express gp120 of HIV virus strain NL4-3. In FIG. 23C, target cells express gp120 of HIV virus strain SF162. In FIG. 23D, target cells express human leukocyte antigen DR0401. The icons Z09, Z10 and CTRL indicate the CAR molecular structures corresponding to modified cells, wherein CTRL refers to CD3+ T cells transduced with recombinant lentivirus SIV-EGFP. The number of target cells mixed for 24 hours were calculated based on the results of flow cytometry detection of GFP+CD3- 293F cells. Then relative reduction of the number of target cells in each experimental group compared to that in CTRL sample with 1: 1 effector: target ratio was respectively calculated as the inhibition rate of each sample to the target cells.

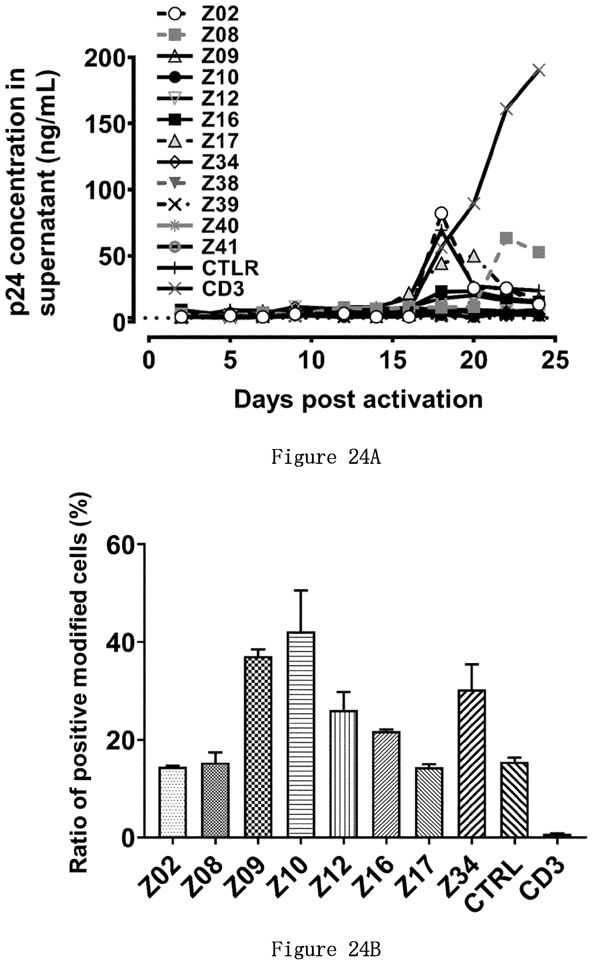

FIG. 24 shows the results of ELISA detection of HIV viral products collected at different time points in the culture supernatant of HIV patient modified cells, and the results of flow cytometry detection and analysis of cells at the final time point. FIG. 24A shows the concentration of virus p24 in the culture supernatant. CTRL is the control of modified cells transfected with plasmid encoding G3L2 shRNA, CD3 is the control of modified cells which are not transduced with any recombinant lentivirus SIV. FIG. 24B is the result of flow cytometry detection and analysis of the cells at 24 days after activation.

### DETAILED DESCRIPTION

In the prior art, there are many limitations on cell therapy (e.g., CAR-T cells) for HIV-infected cells. On one hand, its specific killing activity is not sufficient; on the other hand, it is liable to HIV infection and be exhausted, therefore the duration of efficacy is shortened. In order to solve the above issues, in one aspect, the present application provides a recombinant cell (for example, CAR-T cells) that has stronger killing activity for HIV-infected cells, maintains for a longer period of time in HIV-infected environment, and has a longer duration of efficacy, and nucleic acids and vectors encoding the CAR and other functional structures of the recombinant cells, pharmaceutical compositions and therapies for treating HIV infection. Another aspect of the present application provides a chimeric antigen receptor molecule (CAR), and cells containing the CAR molecule have efficient and specific killing activity against HIV-infected cells.

### DEFINITIONS

For the purpose of explanation of the specification, the following definitions will be applied and, when appropriate, the terminology used in the singular will also include the plural forms and vice versa. If any of the definitions set forth below conflict with any of the documents incorporated herein by reference, the proposed definition in the specification will prevail.

As used herein, "shRNA" is a short hairpin RNA or a small hairpin RNA, which is an artificial RNA molecule with a hairpin structure, and can be used for silencing the expression of a target gene through RNA interference.

As used herein, "Chimeric Antigen Receptor (CAR)" is a class of engineered cell surface receptors, generally expressed on immune effector cells (such as effector T cells), which mediate the killing of cells with specific targets (in the present application, referred to as "target cells", such as cells expressing HIV envelope protein gp120 on the cell surface) by engineered immune effector cells. CAR usually includes an extracellular region, an intracellular region, and a transmembrane region between them. The "extracellular region" contains an antigen-specific recognition domain, which can specifically recognize and bind to the antigen. In some embodiments, the antigen recognition region and the transmembrane domain are connected by a hinge region. An "intracellular region" may comprise one or more primary signaling domains and one or more co-stimulatory domains. After the antigen-specific recognition domain binding to the antigen to form a binary complex and then conformational change, the intracellular region can initiate a series of biochemical reactions, causing the immune effector cells where the CAR is to generate biological effects, such as secreting cytokines or killing target cells directly. Herein, unless otherwise specified particularly, the "primary signal transduction domain" provides the first signal of activating lymphocytes (such as effector T cells), while the "co-stimulatory domain" provides the second signal of activating lymphocytes. Both the "primary signal transduction domain" and the "co-stimulatory domain" are derived from the "signal transduction domain" of the receptor molecule, which refers to the domain of a receptor molecule that is partially or completely located inside the cell and plays a role in signal transduction after the receptor specifically binds to the ligand.

As used herein, the "co-stimulatory domain" is mainly used to provide co-stimulatory signals to enhance the activity of immune cells, including, for example, enhancing the proliferation, survival and/or development of memory cells. In some embodiments, the "co-stimulatory domain" is selected from the signal transduction domains of CD28, 4-1BB (CD137), OX40 (CD134), and the like.

As used herein, the "transmembrane region" refers to a protein structural region that is anchored into the cell membrane and thermodynamically stable. Transmembrane domains can be obtained from natural proteins, such as those derived from the T cell receptor (TCR). In some embodiments, the transmembrane domain is selected from the transmembrane domains of CD4, CD8α, CD28, and CD3ζ.

As used herein, the "hinge region" is a segment of peptide chain that connects the antigen-specific recognition domain or the extracellular region and the transmembrane domain, usually is elastic. In some embodiments, the hinge region is derived from the hinge of IgG or the hinge region of CD8α/CD28. The "hinge" of IgG refers to the region between CH1 and CH2 functional regions of IgG, usually containing a large amount of prolines.

As used herein, " CAR-T ", i.e., chimeric antigen receptor-T cell, is a T cell that expresses a chimeric antigen receptor molecule on the cell surface, and can recognize the antigen on the surface of target cell. The CAR-T has now been developed to the fourth generation. The CAR molecule of the first-generation CAR-T was formed by connecting the signal transduction domain of CD3ζ chain or FcεRlγ with an antigen recognition region, and lacked co-stimulatory domain. The first-generation CAR-T had limited proliferation ability in vivo and was prone to apoptosis. In the second-generation CAR, a co-stimulatory domain was added into the CAR, such as CD28 or 4-1 BB (CD137). CD28 has strong anti-tumor activity, and the advantages of 4-1BB are that it can prolong the survival time of T lymphocytes and maintain its anti-tumor effect. The second-generation CAR had stronger proliferation ability than the first-generation CAR, and could secrete more cytokines and anti-apoptotic proteins. The third-generation CAR-T could not only express two co-stimulatory signal molecules at the same time, but also secrete more IFN-γ, which had even higher anti-tumor cytotoxicity. The fourth-generation CAR-T can also secrete specific cytokines (such as IL-12) in tumors, thereby changing the tumor microenvironment and influencing and activating other immune cells to generate immune response.

As used herein, the term "linker peptide" refers to an oligopeptide or polypeptide region of about 1 to 100 amino acids in length that links together any domains/regions of the CAR of the present invention. The linker peptide may be composed of flexible residues like glycine and serine so that the adjacent protein domains are free to move relative to one another. Longer linker peptides may be used when it is desirable to ensure that two adjacent domains do not sterically interfere with one another.

In the present application, "GS linker", as a kind of linker peptide, refers to a flexible linker peptide composed of glycine (G) and serine (S). The most common GS linker is (GGGGS) n. The distance between domains can be increased or reduced by changing the number of n.

As used herein, " CD4 " is a cluster of differentiated antigens, which is the member of immunoglobulin superfamily expressed on surfaces of helper T (Th) cells, regulatory T cells, monocytes, macrophages and dendritic cells, and plays an important role in the development and activation of T cells. CD4 comprises an amino-terminal extracellular domain (containing four immunoglobulin domains exposed on the outer surface of cell: D1, D2, D3 and D4, presenting an Ig-like structure), a transmembrane domain, and a short cytoplasmic tail region. D1 and D3 are similar to immunoglobulin variable regions. D2 and D4 are similar to the constant region of immunoglobulin. CD4 is also the main receptor of HIV, and CD4 positive (CD4+) T cell is the target of HIV (human immunodeficiency virus) attack.

As used herein, "gp120" is a glycoprotein exposed on the surface of HIV envelope. The 120 in its name comes from its molecular weight of 120 kDa. On helper T cells, gp120 can bind to CD4, inducing conformational changes in gp120 and gp41 and enabling HIV-1 to bind to co-receptors (such as CCR5 or CXCR4) expressed on host cells, and eventually the envelope of HIV is fused with the cell membrane leading to the entry of the virus into the cell.

As described herein, the term "antibody" includes full-length antibodies and antigen-binding fragments thereof. Full-length antibodies comprise two heavy chains and two light chains. The variable regions of the light chain and heavy chain are responsible for antigen binding. Variable regions of the two chains typically comprise three hypervariable loops, referred to as complementarity determining regions (CDRs) (light chain (LC) CDRs comprising LC-CDR1, LC-CDR2 and LC-CDR3, heavy chain (HC) CDRs comprising HC-CDR1, HC-CDR2 and HC-CDR3). The three CDR regions of the heavy chain or light chain are inserted between flanking segments called framework regions (FRs), which are more conserved than the CDR regions and form a scaffold to support the hypervariable loops. The constant regions of the heavy and light chains are not involved in antigen binding, but can exhibit various effector functions. Antibodies are classified based on the amino acid sequences of their heavy chain constant regions. The five major classes or isotypes of antibodies are IgA, IgD, IgE, IgG and IgM, characterized by heavy chains of α, δ, ε, γ and µ type, respectively. Several major antibody classes are divided into subclasses such as IgG1 (γ1 heavy chain), IgG2 (γ2 heavy chain), IgG3 (γ3 heavy chain), IgG4 (γ4 heavy chain), IgA1 (α1 heavy chain) or IgA2 (α2 heavy chain).

As described herein, the term " antigen-binding fragment" includes an antibody fragment, such as diabody, Fab, Fab', F (ab') 2, Fv fragment, disulfide-stabilized Fv antibody fragment (dsFv), (dsFv) 2, bispecific dsFv (dsFv-dsFv'), disulfide-stabilized diabody (ds-Diabody), single-chain Fv (scFv), scFv dimer (bivalent diabody), multispecific antibody composed of antibody fragments containing one or more CDRs, single domain antibody, nanobody, domain antibody, bivalent domain antibody, or any other antibody fragment capable of binding to an antigen but not containing a complete antibody structure. The antigen-binding fragment can bind the same antigen as the parental antibody or parental antibody fragment (for example, parental scFv). Antigen-binding fragments also include fusion proteins comprising the antibody fragments described above.

As used herein, the term "specific binding" refers to the contact between an antibody and an antigen, a receptor and a ligand, or a specific antigen recognition domain and an antigen, with a binding affinity of at least 10⁻⁶M. In certain aspects, the binding affinity is at least about 10⁻⁷M, preferably 10⁻⁸M, 10⁻⁹M, 10⁻¹⁰M, 10⁻¹¹M, or 10⁻¹²M.

In the present application, the terms "polynucleotide" or "nucleic acid" and "nucleic acid molecule" may be used interchangeably, including but not limited to DNA, RNA, cDNA (complementary DNA), mRNA (messenger RNA), rRNA (ribosomal RNA), shRNA (small hairpin RNA), snRNA (small nuclear RNA), snoRNA (short nucleolar RNA), miRNA (microRNA), genomic DNA, synthetic DNA, synthetic RNA and/or tRNA.

As used herein, " vectors", " cloning vectors", and " expression vectors " refer to vectors through which polynucleotide sequences (e.g., foreign genes) can be introduced into a host cell to transform the host and facilitate the expression of the introduced sequence (e.g., transcription and translation). Vectors include plasmids, phages, viruses, and the like.

As used herein, "immune effector cells" refer to cells capable of achieving immune effects and immune responses against target antigens or target cells, such as cells with immune killing effects and immune response effects, such as T cells and the like.

As used herein, the "signal peptide" refers to a short peptide chain, usually 5 to 30 amino acids in length, which directs the transfer of newly synthesized proteins to secretory pathway. In some embodiments, the signal peptide is a membrane localization signal peptide, that is, an amino acid sequence used to direct transmembrane transfer and/or localization of a protein. In most cases, the signal peptide is located at the N-terminus of the amino acid sequence. In mRNA, the coding sequence of the signal peptide is usually located after the initiation codon, which is an RNA region encoding a hydrophobic amino acid sequence. After the signal peptide guides the protein to complete its positioning, it is usually excised under the action of signal peptidase.

As used herein, the "safety switch" refers to an engineered protein designed to prevent potential toxicity or adverse effects of cell therapy in other ways. In some embodiments, the expression of the safety switch is conditionally controlled to address the safety issues of the transplanted engineered cells/recombinant cells. In some embodiments, the gene encoding the safety switch may be incorporated permanently into cell genome. In some embodiments, the safety switch is capable of mediating induction of apoptosis, inhibition of protein synthesis, DNA replication, growth arrest, transcriptional and post-transcriptional gene regulation, and/or antibody-mediated depletion (e.g., by ADCC, CDC, etc.). In some embodiments, activation of the safety switch results in the death of the cell in which the safety switch has been activated. In some embodiments, activation of the safety switch results in downregulation of the activity of the cell in which the safety switch has been activated, wherein the cell can be reactivated in the future. In other embodiments, the default activity of the cell is down-regulated and the activation of the safety switch results in an up-regulation of the cell activity, wherein the cell can be deactivated in the future.

In some embodiments, the safety switches described herein include receptors capable of being targeted by antibodies or antibody fragments, examples of such receptors including EPCAM, VEGFR, integrins (e.g., integrin αvβ3, α4, αl3/4β3, α4β7, α5β1, αvβ3, αv), TNF receptor superfamily members (e.g. TRAIL-R1, TRAIL-R2), PDGF receptor, interferon receptor, folate receptor, GPNMB, ICAM-1, HLA-DR, CEA, CA-125, MUC1, TAG-72, IL-6 receptor, 5T4, GD2, GDB, CD2, CD3, CD4, CD5, CD11, CD11a/LFA-1, CD15, CD18/ITGB2, CD19, CD20, CD22, CD23/IgE receptor, CD25, CD28, CD30, CD33, CD38, CD40, CD41, CD44, CD51, CD52, CD62L, CD74, CD80, CD125, CD147/basigin, CD152/CTLA-4, CD154/ CD40L, CD195/CCR5, CD319/SLAMF7, and EGFR and truncated forms thereof (e.g., forms that retain one or more extracellular epitopes but lack one or more regions within the cytoplasmic domain), etc. Safety switches suitable for use in the present application are known in the art, and the preferred examples thereof include, but are not limited to, e.g., caspase 9 (iCaspase-9), iCaspase-1, iCaspase-8, thymidine kinase (e.g., HSV-TK, VZV-TK), cytosine deaminase (CD), CD20, truncated EGFR (tEGFR), FR806, RQP8, or any combination thereof.

As is known in the art, different types of safety switches can be activated and/or recognized by different exogenous molecules. In some embodiments, the safety switch is activated by an exogenous molecule (e.g., a prodrug), which triggers apoptosis and/or cell death in the therapeutic cell upon activation. In other embodiments, the safety switch is recognized by a molecule (e.g., an antibody) capable of inducing cell death (e.g., ADCC or complement-induced cell death). In some embodiments, when the safety switch is iCaspase-9, a dimerization drug that results in iCaspase-9 activation and apoptosis can be administered. The iCaspase-9 molecule contains a chemically induced dimerizers (CID) binding domain that mediates dimerization in the presence of a CID. This results in inducible and selective depletion of CAR-expressing cells. In other embodiments, for recombinant cells comprising truncated EGFR, although tEGFR lacks signal transduction ability, it retains the epitope recognized by molecules capable of inducing ADCC (e.g., cetuximab), thereby administrating cetuximab to the recombinant cells results in ADCC and subsequent depletion of the recombinant cells. For recombinant cells comprising CD20, the administration of rituximab can destroy the recombinant cells. In other embodiments, the recombinant cells comprise HSV-TK that will cause cell death when contacted with gancyclovir. In some other embodiments, exogenous molecules capable of activating and/or recognizing safety switches include ganciclovir (GCV), acyclovir (ACV), bromovinyl deoxyuridine (BVDU), 6-Methoxypurine arabinoside (AraTP), 5-fluorocytosine (5-Fc), AP20187, AP1903, tamoxifen, tacrolimus, etc.

As used herein, the term "reporter gene" refers to a nucleotide sequence encoding a reporter molecule. "Reporter molecule" refers to a substance detectable in any one of a variety of detection systems, which can be used to identify potentially transfected cells. In one embodiment, reporter molecules include, but are not limited to, luciferase, β-galactosidase, chloramphenicol acetyltransferase (CAT), alkaline phosphatase (AKP), fluorescent protein such as green fluorescent protein (GFP or EGFP), yellow fluorescent protein (YFP), red fluorescent protein (RFP), or other reporter molecules known in the art. In another embodiment, tEGFR may also be used as a reporter molecule.

As used herein, " identity " refers to the degree of similarity between amino acid sequences or between nucleotide sequences through sequence alignment software such as BLAST. For example, in the present application, at least 85% sequence identity includes, for example: at least 90%, 93%, 95%, 97%, 98%, 99% sequence identity.

As used herein, the term "effective amount" or "therapeutically effective amount" refers to the amount of a pharmaceutical composition comprising one or more of the peptide, protein, nucleic acid, or mutant, variant, analog or derivative thereof disclosed in the present application, and the patient or subject receiving the "effective amount" or "therapeutically effective amount" of the pharmaceutical composition can obtain a reasonable benefit/risk ratio of medical treatment, thereby alleviating or preventing at least one or more symptoms of the diseases or conditions, achieving a desired therapeutic or preventive effect.

As used herein, the term "about" refers to the normal error range for various values readily known to those skilled in the art. Reference to "about" a value or parameter includes (and describe) embodiment for that value or parameter itself. As used herein, the term "about" being ahead of a numerical value means within 10% above or below the numerical value. For example, "about 100" encompasses 90 and 110.

As used herein, the singular forms " a, " " an, " and " the " include plural forms unless otherwise indicated.

Unless otherwise defined herein, all technical and scientific terms used herein have the same meaning as commonly understood by one of ordinary skill in the art to which this invention belongs.

### Engineered cells or recombinant cells

In one aspect, the present application provides a recombinant cell comprising the following functional structure:
1) a chimeric antigen receptor (CAR) or a nucleic acid coding therefor, wherein the CAR comprises an extracellular region, a transmembrane region and an intracellular region, wherein the extracellular region comprises D1 and D2 structural domain of human CD4 molecule;
2) an shRNA or a nucleic acid coding therefor targets one or more host genes or HIV genes involved in the HIV life cycle selected from the group consisting of NF-κB, CCR5, TSG101, CXCR4, P-TEFb, tat, rev, nef, env, LTR, and gag.

In some embodiments, the recombinant cells are derived from HIV receptor cells or peripheral blood mononuclear cells. In other embodiments, the recombinant cells are derived from lymphocytes. In some embodiments, the recombinant cells are derived from T cells. In other embodiments, the recombinant cells are derived from naive T cells, memory T cells, or effector T cells. In other embodiments, the recombinant cells are derived from cytotoxic T cells, helper T cells, or regulatory T cells. In other embodiments, the T cells are derived from CD4+ T cells or CD8+ T cells. In other embodiments, the recombinant cells are derived from NKT cells. In other embodiments, the recombinant cells are derived from γδ T cells. In other embodiments, the recombinant cells are derived from NK cells. In other embodiments, the recombinant cells are derived from antigen-presenting cells, such as macrophages and dendritic cells.

In some embodiments, the recombinant cells are derived from progenitor cells or stem cells. In some embodiments of the invention, the progenitor cells or stem cells include hematopoietic stem cells (e.g., CD34+ cells) or hematopoietic progenitor cells. In other embodiments of the present invention, the stem cells include memory T stem cells, such as central memory T cells, effector memory T cells, or stem cell memory T cells. In another embodiment of the invention, the stem cells are induced to differentiate into any of the T cells as previously described. In some embodiments, the recombinant cells are derived from patients infected with HIV. In some embodiments, the recombinant cells are derived from healthy populations.

Those skilled in the art should know that the genes involved in the HIV life cycle include its structural and regulatory genes, regulatory elements, and some genes of the host cell. Interfering or inhibiting any step in the HIV life cycle can affect its replication and proliferation. However, it is difficult to predict which gene or genes among them should be interfered or inhibited in order to achieve this effect well. and to make the balance between the anti-HIV effect and the normal physiological function of the host cell. For example, it is difficult to be obtained from the prior art how to minimize the infection of HIV to the cells, reduce and delay the depletion of recombinant cells while maintain the recombinant cell killing ability of the present application as possible.

The HIV genome is known to consists of at least seven regulatory elements (LTR, TAR, RRE, PE, SLIP, CRS, and INS) and nine genes (gag, pol, env, tat, rev, nef, vif, vpr, vpu, and sometimes there is a tenth gene, tev, which is a fusion of tat, env, and rev) and encodes a total of 19 proteins. Three of these genes, gag, pol and env, contain the information required to make the structural proteins of new virus particles, tat, rev, nef, vif, vpr, and vpu (vpx in the case of HIV-2) are regulatory genes for proteins, which control HIV's ability to infect cells, the production of new viral copies, the replication of virus, or cause disease. Regulatory elements interact with the gene expression regulatory proteins, participate in genome transcription, translation and other processes, or interfere with normal physiological activities of host cells. In addition, genes involved in HIV life cycle also include some host genes, such as NF-κB, CCR5, TSG101, CXCR4, P-TEFb and the like. Therefore, the introduction of shRNA that can target and inhibit these genes has the opportunity to interfere with the replication and proliferation of HIV and reduce its damage to host cells.

Therefore, in some embodiments, the shRNAs which inhibit one or more host genes or HIV genes involved in the HIV life cycle and are comprised in the recombinant cell target the gag gene and the LTR gene of HIV. In some embodiments, the shRNAs target gag and nef of HIV.

In some embodiments, the nucleic acid sequence of shRNA targeting gag comprises the sequence of SEQ ID NO: 1, or the nucleic acid sequence having at least 85%, 90%, 93%, 95%, 97%, 98%, 99% identity to the sequence of SEQ ID NO: 1. In some embodiments, the nucleic acid sequence of shRNA targeting LTR comprises the sequence of SEQ ID NO: 2, or the nucleic acid sequence having at least 85%, 90%, 93%, 95%, 97%, 98%, 99% identity to the sequence of SEQ ID NO: 2. In some embodiments, the nucleic acid sequence encoding shRNA targeting gag comprises the sequence of SEQ ID NO: 3, or the nucleic acid sequence having at least 85%, 90%, 93%, 95%, 97%, 98%, 99% identity to the sequence of SEQ ID NO: 3. In some embodiments, the nucleic acid sequence of shRNA targeting LTR comprises the sequence of SEQ ID NO: 2, or the nucleic acid sequence having at least 85%, 90%, 93%, 95%, 97%, 98%, 99% identity to the sequence of SEQ ID NO: 2. In some embodiments, the nucleic acid sequence encoding shRNA targeting LTR comprises the sequence of SEQ ID NO: 4, or the nucleic acid sequence having at least 85%, 90%, 93%, 95%, 97%, 98%, 99% identity to the sequence of SEQ ID NO: 4.

In some embodiments, the nucleic acid sequence of shRNA targeting gag comprises the sequence of SEQ ID NO: 5, or the nucleic acid sequence having at least 85%, 90%, 93%, 95%, 97%, 98%, 99% identity to the sequence of SEQ ID NO: 5. In some embodiments, the nucleic acid sequence of shRNA targeting nef comprises the sequence of SEQ ID NO: 6, or the nucleic acid sequence having at least 85%, 90%, 93%, 95%, 97%, 98%, 99% identity to the sequence of SEQ ID NO: 6. In some embodiments, the nucleic acid sequence encoding shRNA targeting gag comprises the sequence of SEQ ID NO: 7, or the nucleic acid sequence having at least 85%, 90%, 93%, 95%, 97%, 98%, 99% identity to the sequence of SEQ ID NO: 7. In some embodiments, the nucleic acid sequence of shRNA targeting nef comprises the sequence of SEQ ID NO: 6, or the nucleic acid sequence having at least 85%, 90%, 93%, 95%, 97%, 98%, 99% identity to the sequence of SEQ ID NO: 6. In some embodiments, the nucleic acid sequence encoding the shRNA targeting nef comprises the sequence of SEQ ID NO: 8, or the nucleic acid sequence having at least 85%, 90%, 93%, 95%, 97%, 98%, 99% identity to the sequence of SEQ ID NO: 8.

In some embodiments of the present application, an H1 promoter is selected as the promoter of shRNA targeting gag, and a U6 promoter is selected as the promoter of shRNA targeting LTR or nef, and favorable transcription and stability of each shRNA are achieved. In some embodiments, the nucleotide sequences of the H1 promoter and the U6 promoter are shown in SEQ ID NO: 9 and 10, respectively, or have at least 85% sequence identity to SEQ ID NO: 9 and 10, respectively.

In some embodiments, the extracellular region of the CAR in the recombinant cell comprises a structure selected from the following structures: D1 domain of human CD4 molecule, D1 and D2 domain of human CD4 molecule, D1-D3 domain of human CD4 molecule, D1-D4 domain of human CD4 molecule, antibody specifically binding to HIV, or antigen-binding fragment thereof. As used herein, the term "antigen-binding fragment" refers to one or more fragments of an antibody that retain the ability to specifically bind to an antigen. Examples of antigen-binding fragments include Fab, Fab', F(ab')2, Fv fragment, diabody, linear antibody, and scFv. In the present application, examples of the HIV-specific antibody or antigen-binding fragment thereof include gp120-binding specific antibody or antigen-binding fragment.

As used herein, the term "scFv" (single-chain antibody variable fragment) refers to a protein domain or protein comprising only the variable regions of the heavy (VH) and light (VL) chains, wherein VH and VL can be connected via a linker peptide. scFv can be expressed as a single chain polypeptide. scFvs retain the specificity of the intact antibody from which they are derived. The light chain and the heavy chain may be in any order, for example, VH-linker-VL or VL-linker-VH, as long as the specificity of the scFv to the target antigen is retained. In some special embodiments, linkers can be omitted. In some embodiments, the scFv form of the anti-gp120 monoclonal antibody is NAb-scFv (the construction method thereof is shown in patent CN103797029B). In some embodiments, the NAb-scFv comprises the sequence of SEQ ID NO:15.

In some preferred embodiments of the present application, the extracellular region comprises D1 and D2 domain of human CD4 molecule but does not comprise D3 and D4 domain. In some specific embodiments, the extracellular region comprises the amino acid sequence of SEQ ID NO: 13 or the amino acid sequence having at least 85% identity to the amino acid sequence. HIV can enter host T cells through the specific binding of its envelope protein gp120 and CD4. By utilizing the specific binding, CD4 or the part thereof that has specific binding ability to gp120 can be used as the specific antigen recognition domain of CAR.

In some embodiments, the transmembrane region comprised in the recombinant cell is derived from or comprises the transmembrane domain of one or more of the protein molecules selected from the group consisting of α, β or ζ chains of T cell receptors, CD28, CD3ε, CD45, CD4, CD5, CD8, CD9, CD16, CD22, CD33, CD37, CD64, CD80, CD86, CD134, CD154, KIRDS2, OX40, CD2, CD27 , LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2Rβ, IL2Rγ, IL7Rα, ITGA1, VLA1, CD49a, ITGA4, IA4, CD49D4, ITGA6, VLA-6, CD49f, ITGAD, CD11d, ITGAE, CD103, ITGAL, CD11a, ITGAM, CD11b, ITGAX, CD11c, ITGB1, CD29, ITGB2, CD18, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKp44, NKp30, NKp46, NKG2D, NKG2C. In some specific embodiments, the transmembrane region is derived from or comprises the transmembrane domain of CD8α protein molecule. In some specific embodiments, the transmembrane region comprises the amino acid sequence of SEQ ID NO: 18 or comprises the amino acid sequence having at least 85% identity to SEQ ID NO: 18.

In some embodiments, the intracellular region of the recombinant cell comprises a primary signaling domain. In some embodiments, the primary signal transduction domain comprises immune-receptor tyrosine-based activation motifs (ITAM, the basic composition thereof is: YXXUV, wherein Y is tyrosine, L/V refers to leucine or valine, and X can be any amino acid). In some embodiments, the primary signal transduction domain is derived from or comprises the signal transduction domain of one or more protein molecules selected from the group consisting of: CD3ζ, CD3γ, CD3δ, CD3ε, FCER1G, FcεR1b, CD79a, CD79b, FcγRlla, DAP10, and DAP12. In some embodiments, the primary signaling domain is derived from or comprises the signaling domain of CD3ζ. In some embodiments, the primary signaling domain comprises the amino acid sequence of SEQ ID NO:23.

In some embodiments, the intracellular region further comprises a co-stimulatory domain derived from or comprising the signal transduction domain of one or more of the protein molecules selected from the group consisting of: CD27, CD28, 4-1BB, OX40, CD30, CD40, PD-1, ICOS, LFA-1, CD2, CD7, LIGHT, NKG2C, B7-H3, ligands that specifically bind to CD83, CDS, ICAM-1, GITR, BAFFR, HVEM (LIGHTR), SLAMF7, NKp80, CD160, CD19, CD4, CD8α, CD8β, IL2Rβ, IL2Rγ, IL7Rα, VLA1, CD49a, ITGA4, IA4, CD49D, ITGA6, VLA-6, CD49f, ITGAD, CD11d , ITGAE, CD103, ITGAL, CD11a, ITGAM, CD11b, ITGAX, CD11c, ITGB1, CD29, ITGB2, CD18, ITGB7, TNFR2, TRANCE/RANKL, DNAM1, SLAMF4, CD84, CD96, CEACAM1 , CRTAM, Ly9, CD160, PSGL1, CD100, CD69, SLAMF6, SLAM, BLAME, SELPLG, LTBR, LAT, GADS, SLP-76, PAG/Cbp, NKp44, NKp30, NKp46, NKG2D, DAP10. In some embodiments, the co-stimulatory domain comprises the signaling domain of CD137, CD28+CD137, or CD137+DAP10.

In some embodiments, the intracellular region comprises the signaling domains of CD3ζ and CD137. In some embodiments, the intracellular region comprises the signaling domains of CD3ζ, CD28, and CD137. In some embodiments, the intracellular region comprises the signaling domains of CD3ζ, CD137, and DAP10. In some embodiments, the intracellular region comprises the signaling domains of CD28, DAP10, and CD3ζ. In some embodiments, the intracellular region comprises the signaling domains of 2B4, DAP10, and CD3ζ. In some embodiments, the intracellular region comprises the signaling domains of CD137, 2B4, and CD3ζ.

In some embodiments, a linker peptide is also comprised in the intracellular region between the primary signal transduction domain and the co-stimulatory domain, as well as between each co-stimulatory domain. The linker peptide can be selected from any natural or synthetic peptides that can connect the various structural domains in the intracellular region and maintain a certain relative spatial distance between the structural domains to ensure their functional integrity and signal transduction between them. Optional linker peptides are known to those skilled in the art and may be used in combination with alternative embodiments of the invention. In some specific embodiments, the linker peptide is a GS linker, such as (GGGGS)n, wherein n is selected from any natural number. In some specific embodiments, the linker peptide is GGGGS.

In some specific embodiments of the present application, the intracellular region comprises the amino acid sequence shown in any one of SEQ ID NO: 28-34 or comprises the amino acid sequence having at least 85%, 90%, 93%, 95%, 97%, 98% and 99% identity with any one of SEQ ID NO: 28-34.

In some embodiments, the extracellular region is connected to the transmembrane region by the hinge region. The hinge region described in the present application can be selected from hinges contained in natural proteins, and can also be selected from artificially synthesized hinge regions, such as GS linkers. In some embodiments of the present application, the hinge region is from or comprises human Ig hinge region, GS linker, KIR2DS2 hinge, or the hinge region of CD8. In some embodiments, the hinge region is the hinge region of CD8α. In some specific embodiments, the hinge region comprises the amino acid sequence of SEQ ID NO: 20 or comprises the amino acid sequence having at least 85%, 90%, 93%, 95%, 97%, 98%, 99% identity to SEQ ID NO: 20.

In some embodiments, the CAR in the recombinant cell of the present application further comprises a signal peptide that locates the CAR on the cell membrane of the recombinant cell. In some embodiments, after the signal peptide directs the protein to complete its localization, the signal peptide is excised under the action of signal peptidase. Therefore, the linking of the signal peptide described in the present application includes the linking of the signal peptide to the CAR at any time of the cell life cycle. In some embodiments, the signal peptide is from or comprises the signal peptide of any secretory proteins or membrane proteins. In some embodiments, the signal peptide is from or comprises the signal peptide of CD4 or CD8. In some embodiments, the signal peptide comprises the amino acid sequence of SEQ ID NO: 16 or 17 or comprises the amino acid sequence having at least 85%, 90%, 93%, 95%, 97%, 98%, 99% identity to SEQ ID NO: 16 or 17.

In some specific embodiments, the CAR intracellular region comprises the amino acid sequence selected from any one of SEQ ID NO: 28-34 or comprises the amino acid sequence having at least 85%, 90%, 93%, 95%, 97%, 98%, 99% identity to any one of SEQ ID NO: 28-34.

In some specific embodiments, the CAR comprises the amino acid sequence selected from any one of SEQ ID NO: 35-46 or comprises the amino acid sequence having at least 85%, 90%, 93%, 95%, 97%, 98%, 99% identity to any one of SEQ ID NO: 35-46.

In some specific embodiments, the recombinant cell comprises shRNA targeting gag and LTR respectively or nucleic acid coding therefor, signal peptide of CD4, D1 and D2 domain of CD4, hinge region of CD8α, transmembrane region of CD8α, signal transduction domain of CD137, and signal transduction domain of CD3ζ.

In some specific embodiments, the recombinant cell comprises shRNA targeting gag and LTR respectively or nucleic acid coding therefor, signal peptide of CD4, D1 and D2 domain of CD4, hinge region of CD8α, transmembrane region of CD8α, signal transduction domain of CD28, signal transduction domain of CD137, and signal transduction domain of CD3ζ.

In some specific embodiments, the recombinant cell comprises shRNA targeting gag and LTR respectively or nucleic acid coding therefor, signal peptide of CD4, D1 and D2 domain of CD4, hinge region of CD8α, transmembrane region of CD8α, signal transduction domain of CD28, signal transduction domain of CD137, and signal transduction domain of CD3ζ. Moreover, the signal transduction domain of CD28, the signal transduction domain of CD137, and the signal transduction domain of CD3ζ are connected via the GS linker peptide.

In some specific embodiments, the recombinant cell comprises shRNA targeting gag and LTR respectively or nucleic acid coding therefor, signal peptide of CD4, D1 and D2 domain of CD4, hinge region of CD8α, transmembrane region of CD8α, signal transduction domain of CD28, signal transduction domain of CD137, signal transduction domain of SLAMF4 (2B4) and/or DAP10, and signal transduction domain of CD3ζ.

In some embodiments, the recombinant cell further comprises a reporter molecule and/or a safety switch.

In some specific embodiments, the safety switch is selected from one or more of the following: iCaspase-9, iCaspase-1, iCaspase-8, thymidine kinase (e.g., HSV-TK, VZV-TK), cytosine deaminase (CD), CD20, tEGFR, FR806, and RQP8. In another preferred embodiment, the safety switch is tEGFR, preferably, the amino acid sequence of the tEGFR is shown in SEQ ID NO: 49 or 50.

In some specific embodiments, the reporter molecule is selected from one or more of the following: luciferase, β-galactosidase, CAT, AKP, GFP or EGFP, YFP, RFP, and tEGFR. In another preferred embodiment, the reporter molecule is preferably RFP or tEGFR, preferably, the amino acid sequence of the RFP is shown in SEQ ID NO: 48, and the amino acid sequence of the tEGFR is shown in SEQ ID NO: 49 or 50.

### Chimeric Antigen Receptor (CAR) and Combinations

In another aspect, the present application provides a chimeric antigen receptor comprising an extracellular region, a transmembrane region and an intracellular region, wherein the extracellular region is from or comprises the extracellular region of human CD4 molecule, and the transmembrane region is from or comprising the transmembrane domain of CD8α.

In a preferred embodiment of the present application, the chimeric antigen receptor further comprises a hinge region, and the extracellular region is connected to the transmembrane region through the hinge region. Preferably, the hinge region is from or comprises human Ig hinge region, GS linker, KIR2DS2 hinge or the hinge region of CD8α, more preferably the hinge region of CD8α. In a specific embodiment of the present application, the hinge region comprises the amino acid sequence of SEQ ID NO: 20 or comprises the amino acid sequence having at least 85% identity to SEQ ID NO: 20.

In some preferred embodiments of the present application, the extracellular region comprises D1 and D2 domain of human CD4 molecule but does not comprise D3 and D4 domains. In some specific embodiments, the extracellular region comprises the amino acid sequence shown in SEQ ID NO: 13 or the amino acid sequence having at least 85% identity to SEQ ID NO: 13.

In some embodiments, the transmembrane region of the CAR comprises the amino acid sequence shown in SEQ ID NO: 18.

In some embodiments, the intracellular region of the CAR comprises a primary signaling domain. In some embodiments, the primary signal transduction domain comprises immune-receptor tyrosine-based activation motifs (ITAM, the basic composition thereof is: YXXUV, wherein Y is tyrosine, UV refers to leucine or valine, and X can be any amino acid). In some embodiments, the primary signal transduction domain is derived from or comprises the signal transduction domain of one or more of the protein molecules selected from the group consisting of: CD3ζ, CD3γ, CD3δ, CD3ε, FCER1G, FcεR1b, CD79a, CD79b, FcγRlla, DAP10, and DAP12. In some embodiments, the primary signaling domain is derived from or comprises the signaling domain of CD3ζ. In some embodiments, the primary signaling domain comprises the amino acid sequence of SEQ ID NO:23.

In some embodiments, the intracellular region further comprises a co-stimulatory domain derived from or comprising the signal transduction domain of one or more of the protein molecules selected from the group consisting of: CD27, CD28, 4-1BB, OX40, CD30, CD40, PD-1, ICOS, LFA-1, CD2, CD7, LIGHT, NKG2C, B7-H3, ligands that specifically bind to CD83, CDS, ICAM-1, GITR, BAFFR, HVEM (LIGHTR), SLAMF7, NKp80, CD160, CD19, CD4, CD8α, CD8β, IL2Rβ, IL2Rγ, IL7Rα, VLA1, CD49a, ITGA4, IA4, CD49D, ITGA6, VLA-6, CD49f, ITGAD, CD11d , ITGAE, CD103, ITGAL, CD11a, ITGAM, CD11b, ITGAX, CD11c, ITGB1, CD29, ITGB2, CD18, ITGB7, TNFR2, TRANCE/RANKL, DNAM1, SLAMF4, CD84, CD96, CEACAM1 , CRTAM, Ly9, CD160, PSGL1, CD100, CD69, SLAMF6, SLAM, BLAME, SELPLG, LTBR, LAT, GADS, SLP-76, PAG/Cbp, NKp44, NKp30, NKp46, NKG2D, DAP10. In some embodiments, the co-stimulatory domain comprises the signaling domain of CD137, CD28+CD137, or CD137+DAP10.

In some embodiments, the intracellular region comprises the signaling domains of CD3ζ and CD137. In some embodiments, the intracellular region comprises the signaling domains of CD3ζ, CD28, and CD137. In some embodiments, the intracellular region comprises the signaling domains of CD3ζ, CD137, and DAP10. In some embodiments, the intracellular region comprises the signaling domains of CD28, DAP10, and CD3ζ. In some embodiments, the intracellular region comprises the signaling domains of 2B4, DAP10, and CD3ζ. In some embodiments, the intracellular region comprises the signaling domains of CD137, 2B4, and CD3ζ.

In some embodiments, a linker peptide is also comprised in the intracellular region between the primary signal transduction domain and co-stimulatory domain, as well as between each co-stimulatory domain. The linker peptide can be selected from any natural or synthetic peptides that can connect the various structural domains in the intracellular region and maintain a certain relative spatial distance between the structural domains to ensure their functional integrity and signal transduction between them. Optional linker peptides are known to those skilled in the art and may be used in combination with alternative embodiments of the invention. In some specific embodiments, the linker peptide is a GS linker, such as (GGGGS)n, wherein n is selected from any natural number. In some specific embodiments, the linker peptide is GGGGS.

In some specific embodiments of the present application, the intracellular region comprises the amino acid sequence shown in any one of SEQ ID NO: 28-34 or comprises the amino acid sequence having at least 85%, 90%, 93%, 95%, 97%, 98% and 99% identity to any one of SEQ ID NO: 28-34.

In some specific embodiments, the CAR comprises the amino acid sequence selected from any one of SEQ ID NO: 35, 36, 43-46 or comprises the amino acid sequence having at least 85%, 90%, 93 %, 95%, 97%, 98%, 99% identity to any one of SEQ ID NO: 35, 36, 43-46.

In some specific embodiments, a further signal peptide is linked to the CAR, and the signal peptide is derived from or comprises a signal peptide of any secretory proteins or membrane proteins, preferably, the signal peptide is derived from or comprises the signal peptide of CD4 or CD8.

Furthermore, the present application also provides a recombinant cell comprising the chimeric antigen receptor as described above.

The present application further provides a combination of the chimeric antigen receptor or a recombinant cell comprising the chimeric antigen receptor and at least one HIV-targeting shRNA or the nucleic acid coding therefor or a vector comprising the nucleic acid thereof described in the present application.

### Nuclear acids, vectors and compositions

The present application also provides a nucleic acid or vector, comprising a polynucleotide encoding the CAR and/or shRNA comprised in the recombinant cell as described above, or the CAR molecule as described above. In some embodiments, the vector is selected from plasmids, viral vectors, or linear nucleic acid molecules. In some particular embodiments, the vector is a retroviral shuttle plasmid vector, such as a Simian Immunodeficiency Virus (SIV) shuttle plasmid vector. In some embodiments, the vector is a packaged SIV viral vector. In some embodiments, the expression and transcription of the CAR and shRNA are driven by a single promoter. In some embodiments, the expression and transcription of the CAR and shRNA are driven by different promoters. In some embodiments, the polynucleotides encoding the CAR and shRNA are contained in different vectors. In some embodiments, the polynucleotide encoding the CAR and shRNA are contained in the same vector. In some embodiments, the polynucleotide encoding shRNA are contained in the same or different vectors.

The present application also provides a composition comprising the nucleic acids or vectors described in the present application. In some embodiments, the present application also provides liposomes comprising the nucleic acids, vectors or compositions.

### Pharmaceutical compositions and therapeutic methods

The present application also provides a pharmaceutical composition comprising the recombinant cell, CAR molecule, nucleic acid or vector, or a combination as described above. The pharmaceutical composition can be used for treating patients infected with HIV. In some embodiments, the HIV-infected patient has been treated with long-term anti-retroviral therapy. In some embodiments, the pharmaceutical composition further comprises a pharmaceutically acceptable excipient, carrier, and/or stabilizer. In some embodiments, suitable excipients, carriers, and/or stabilizers are selected from the group consisting of antioxidants, preservatives, pyrogen-free water, isotonic saline solutions, phosphate buffers, etc., or combinations thereof. The pharmaceutically acceptable carrier, excipient or stabilizer is non-toxic to recipients at the dosage and concentration employed, and includes, for example, a buffer, such as phosphate, citrate or acetate, having pH generally from 5.0 to 8.0, optionally from 6.0 to 7.0; isotonic salts such as sodium chloride, potassium chloride, etc.; antioxidants; preservatives; low molecular weight polypeptides; proteins; hydrophilic polymers such as polysorbate 80; amino acids such as glycine; carbohydrates; chelating agents; sugars; and other standard components known to those skilled in the art (Remington: The Science and Practice of Pharmacy, 22th ed., Loyd V. Allen et al., Pharmaceutical Press (2012)).

The pharmaceutical composition of the present application can be made into various dosage forms as required, and can be administered by the doctor at the amount beneficial to the patient that can be determined according to the patient's type, age, body weight and general disease condition, administration method and other factors. Administration can be, such as parenteral administration (e.g., injection) or other treatment approaches. The present application further provides a method for treating HIV infection, comprising administering to a subject infected with HIV an effective amount of the recombinant cell, the nucleic acid or vector as described above, or the combination as described above, or the pharmaceutical composition as described above. In some embodiments, the administration is intravenous injection or arterial infusion. In some embodiments, the effective amount is determined according to the disease severity, age and physical condition of the patient. In some embodiments, the method for treating HIV infection further comprises administering to an HIV-infected subject an effective amount of the recombinant cells, nucleic acids or vectors as described above, the combinations as described above, or the pharmaceutical compositions as described above, and coadministration of other nucleoside inhibitors, HIV vaccines, broad-spectrum neutralizing antibodies and/or CAR-T cells against HIV.

### Preparation method

In addition, the present application also provides a method for preparing the recombinant cell as described above, which comprises introducing the vectors into the cell described above for expression. In some embodiments, the vector is a SIV shuttle plasmid, and the preparation method comprises packaging the SIV shuttle plasmid into SIV virus particles, and infecting the cells with the virus particles. In some embodiments, the preparation method comprises packaging the SIV shuttle plasmid into SIV virus particles, infecting the progenitor cells or stem cells with the virus particles, and further differentiating the progenitor cells or stem cells into mature effector T cells through stimulation by cytokine and the like.

The preferred embodiments of the present invention have been described in detail above. However, the present invention is not limited thereto. Within the scope of the technical concept of the present invention, various simple modifications can be made to the technical solution of the present invention, including the combination of various technical features in any other suitable manners, and these simple modifications and combinations should also be regarded as the content disclosed in the present invention. All belong to the protection scope of the present invention.

It should be understood that the above description and the following examples are intended to illustrate rather than limit the scope of the invention. Other aspects, advantages and modifications within the scope of the invention will be apparent to those skilled in the art to which the invention pertains.

### EXAMPLES

### Example 1: Vector Construction

The plasmids carrying exogenous gene expression cassette used in the examples of the present application were constructed based on the shuttle plasmid in the SIV third-generation lentiviral vector packaging system. The schematic diagrams of the important functional structures inserted into this series of plasmids relative to shuttle plasmid in the SIV third-generation lentiviral vector packaging system are shown in Fig. 1A-1C. The meanings of the structures in Fig. 1A-1C are as follows: G3L2 represents the coding sequences of shRNA (sequences are shown in SEQ ID NO: 1 and 2, respectively) targeting the conserved sequences of Gag and LTR in the HIV genome (each contains an independent promoter); G2N represents the coding sequence of shRNA (sequences shown in SEQ ID NO: 5 and 6) targeting the conserved sequences of Gag and Nef in the HIV genome (each contains an independent promoter); CD4 signal represents the signal peptide region of CD4; CD4 D1D2 represents the D1 and D2 domains of CD4; CD8h indicates the hinge region of CD8α; CD8a TM and CD28TM represent the transmembrane regions of CD8α and CD28, respectively; CD28, CD137, 2B4, CD3ζ and DAP10 represent the signal transduction domains of CD28, CD137, 2B4, CD3ζ and DAP10, respectively; Nab-scFv represents the neutralizing antibody scFv (3BNC117 was used in this example); CCR5&tat/rev indicates the shRNA coding sequence targeting CCR5 and tat/rev. Reporter represents a reporter gene, and the reporter gene used in the examples of the present application is enhanced green fluorescent protein (EGFP), red fluorescent protein (RFP) or truncated epidermal growth factor receptor (tEGFR). L represents the linker peptide GGGGS; L3 represents the linker peptide (GGGGS)3. Related sequences are shown in Table 2.

First, based on the pGTV-PEDF vector (Fig. 2A), according to the combination shown in Fig. 1A-1C and the sequences in Table 2, the plasmid carrying the Z09 structure was obtained by nucleic acid fragment synthesis, seamless ligation, and transformation (Fig. 2B). After that, plasmids carrying Z10-Z15, Z17, Z34-Z37 and other structures were constructed on the basis of the plasmid said above. The series of plasmids carrying the Z01-Z07 structure were constructed based on the series of plasmids carrying the Z09-Z15 structure, wherein Z01 corresponds to Z09, Z02 corresponds to Z10, Z03 corresponds to Z11, Z04 corresponds to Z12, Z05 corresponds to Z13, Z06 corresponds to Z14, and Z07 corresponds to Z15. The difference between the plasmids carrying the Z01-Z07 structure and their respective corresponding plasmids is that the former does not contain polynucleotides encoding shRNA. In addition, the difference between Z01/Z02/Z09/Z10 and Z11-Z15/Z03-Z07 is that the extracellular region, hinge region, and transmembrane region of the former are the D1D2 domain of CD4, the hinge region of CD8α, and the transmembrane region of CD8α, respectively. In order to show the difference between the plasmid and its corresponding plasmid more visually, Fig. 2C shows the map of the plasmid carrying the Z01 structure. The construction of the plasmid carrying the Z08 structure and the Z16 structure refers to the US patent US 9,833,480 B2.

Nucleic acid fragment synthesis, seamless ligation, transformation and other steps or methods involved in plasmid construction are conventional techniques in the art, and can be operated with reference to, for example, J Sambrook et al., Molecular Cloning: A Laboratory Manual, 3rd ed., Cold Spring Harbor Laboratory Press, 2001, or according to the specification of the manufacturer.

### Example 2: Preparation of recombinant lentivirus based on SIV vector

### 2.1 Packaging and purification of recombinant lentivirus SIV

The methods for the construction of packaging vector, rev expression vector and VSV-G expression vector are described in details in Example 1 of patent ZL200680012905.4.

The cell line 293T cells (ATCC, CRL-11268) were seeded with 9×10⁶ cells per T225 culture flask (Coring, Cat#431082), and cultured in 20 mL of D-MEM medium (Thermofisher, Cat#11995-065) containing 10% fetal bovine serum for 48 hours. Then the culture medium was replaced with 10 mL of pre-warmed OPTI-MEM medium (Thermofisher, Cat#31985-070), and the cells as the ones to be transfected and ready for use.

Transfection system was prepared for each T225 culture bottle respectively. First, 60 µg of the shuttle plasmid obtained in Example 1, 30 µg of the packaging vector, 12 µg of the rev expression vector, and 12 µg of the VSV-G expression vector were dissolved in 2.25 mL of HBSS buffer to form a plasmid mixture. Then, after 167 nM CaCh solution was prepared, the 2.25 mL plasmid mixtures were added to 2.25 mL of the CaCh solution prepared above. The solution was mixed by vortex immediately for 5 seconds, and incubated at room temperature for 10-15 minutes to obtain about 4.5 mL solutions containing plasmid DNA-CaCh complex. Then, the solution was added to the culture flask inoculated with cells, and cultured in a 37°C, 5% CO₂ incubator for 3 hours. Then each bottle was supplemented with DMEM medium containing 20% FBS, with the final concentration of FBS being 10%, and incubated overnight in a 37°C, 5% CO₂ incubator.

20 hours after transfection, the culture medium in each T225 culture bottle was replaced with 20 mL of fresh pre-warmed DMEM medium. 48 hours after transfection, the supernatant was recovered and filtered with a 0.45 µm filter to obtain a primary filtrate, which was then concentrated using a high-speed centrifuge. Specifically, the primary filtrate was placed in a high-speed centrifuge tube and centrifuged at 4°C under 40,000 g for 2 hours. After the supernatant was completely removed from the centrifuge tube, DPBS was added into each centrifuge tube to cover the precipitate, and then the pellet was resuspended to obtain the recombinant lentivirus SIV concentrate. The virus concentrate can be used directly or cryopreserved at -80°C for later use after aliquoting.

### 2.2 Infectious titer of recombinant virus SIV

293T cells was infected with the recombinant lentivirus SIV obtained in section 2.1, and then the ratio of cells expressing the reporter gene or the extracellular region of the CAR structure in the 293T cells was detected by flow cytometry, so that the infectious titer of the recombinant lentivirus SIV was obtained (for example, see Kunter et al., Nature Protocol, 2009).

Specifically, 2×10⁴/well 293T cells were seeded in 24-well plates 1 day before infection. On the day of infection, the medium in each well was replaced with 0.5 mL of pre-warmed DMEM medium (Thermofisher, Cat#11995-065). For each recombinant lentivirus SIV, three wells were prepared, then 0.5, 5, or 50 µL of the same recombinant lentivirus SIV concentrate diluted 100 times in DMEM medium was added into each well respectively, cultured in a 37°C, 5% CO₂ Incubator overnight. The culture was supplemented with fresh medium on the second day after infection: each well was supplemented with a complete medium containing 20% serum at a volume of 1:1, and was cultured in a 37°C, 5% CO₂ incubator.

On the 3rd day post-infection, the medium in the well was removed, each well was washed once with 0.5 mL DPBS, and then 0.1 mL/well of TrypLE Express Enzyme (Thermofisher, Cat# 12605028) was added to digest the cells. 1 mL of DPBS was added into each well. The cells were repeatedly pipetted to suspend in DPBS, transferred to flow tubes, and centrifuged at 300 g for 5 minutes. The supernatant was discarded, and the cells were resuspended in DPBS. When the reporter gene is EGFP or RFP, the signal of fluorescent protein was detected directly by flow cytometry (BD Celesta) to obtain the ratio of positive cells. When the reporter gene is tEGFR, immunofluorescent staining was performed using anti-tEGFR antibody (R&D, FAB9577G-100). Then, the immunofluorescence signal was detected by flow cytometry, and the ratio of positive cells was obtained.

Meanwhile, since the CAR molecule containing CD4 and the reporter gene was connected through P2A in the CAR molecule, it can also be confirmed by detecting CD4. Using the anti-human CD4 antibody (BD, Cat#562424) to perform immunofluorescence staining on the DPBS resuspended cells according to relevant instructions, the ratio of positive cells was then obtained by using flow cytometry to detect the fluorescent signal conjugated to the human CD4 antibody.

The infectious titer of the recombinant lentivirus SIV was calculated by using the formula IU/ml=(F×N×D×1000)/V. F represents the ratio of positive cells; N represents the number of cells in each well during infection; D represents the dilution factor of stock solution; and V represents the volume of the diluted virus actually added to each well. For the same recombinant lentivirus, the virus titers obtained in each well were calculated combinedly to obtain an average value, which was used as the infection titer of the recombinant lentivirus. Recombinant lentivirus SIV-EGFP obtained by packaging shuttle plasmid pGTV-EGFP carrying EGFP was used as a control. Table 1 shows the results of infectious titers detected by using reporter genes.

**Table 1. Infectious titers of recombinant lentivirus SIV**

| Plasmid name | Recombinant lentivirus SIV name | Infectious titer (IU/ml) |
|---|---|---|
| Z01 | SIV-Z01 | 2.70E+07 |
| Z02 | SIV-Z02 | 3.95E+07 |
| Z03 | SIV-Z03 | 1.25E+07 |
| Z04 | SIV-Z04 | 9.05E+06 |
| Z05 | SIV-Z05 | 3.30E+07 |
| Z06 | SIV-Z06 | 2.43E+07 |
| Z07 | SIV-Z07 | 1.25E+07 |
| Z08 | SIV-Z08 | 3.23E+07 |
| Z09 | SIV-Z09 | 5.41 E+07 |
| Z10 | SIV-Z10 | 1.35E+07 |
| Z11 | SIV-Z11 | 8.93E+06 |
| Z12 | SIV-Z12 | 3.70E+07 |
| Z13 | SIV-Z13 | 3.18E+07 |
| Z14 | SIV-Z14 | 6.78E+06 |
| Z15 | SIV-Z15 | 2.99E+07 |
| Z16 | SIV-Z16 | 2.31 E+07 |
| Z17 | SIV-Z17 | 2.54E+07 |
| Z34 | SIV-Z34 | 3.96E+07 |
| Z35 | SIV-Z35 | 2.54E+07 |
| Z36 | SIV-Z36 | 2.52E+07 |
| Z37 | SIV-Z37 | 1.34E+07 |
| Z38 | SIV-Z38 | 5.72E+07 |
| Z39 | SIV-Z39 | 1.78E+07 |
| Z40 | SIV-Z40 | 4.23E+07 |
| Z41 | SIV-Z41 | 1.15E+07 |
| pGTV-EGFP | SIV-EGFP | 3.09E+08 |

### Example 3: Expression of recombinant proteins

### 3.1 Detection of target proteins using Western blot method

Each plasmid in Example 1 was directly transfected into 293T cells, and the corresponding cells were harvested 48 hours after transfection. Cells were lysed by adding lysis buffer. After centrifugation, the supernatant was obtained. The protein concentration in the supernatant was quantitatively detected using BCA method. After appropriately diluting the supernatant according to the required concentration, the target protein was detected by Western blot. In the Western blot detection method, anti-CD3ζ antibody (Santacruz sc-166275) was used as the primary antibody, and horseradish peroxide-labeled goat anti-mouse IgG (H+L) (Beyotime A0216) was used as the secondary antibody.

Except for the plasmid carrying the Z17 structure without CD3ζ and the control pGTV-EGFP, positive spots at the position of target band could be detected for all cell lysates transfected with other plasmids (data not shown). This indicated that all plasmid expression cassettes were constructed successfully.

### 3.2 Flow cytometric detection of proteins on the surface or inside of cells

Each recombinant lentivirus SIV obtained in Example 2 was used to infect 293T cells in the same volume with original infectious titer. After 48 hours, the ratio of positive cells was detected with reference to the method in Example 2. That is, after immunofluorescent staining of 293T cells with the anti-human CD4 antibody (BD, Cat#562424), the conjugated fluorescent signal was detected by flow cytometry to obtain the ratio of positive cells. Or the signal of the fluorescent protein was directly detected by using a flow cytometer (BD Celesta) to obtain the ratio of the positive cells.

The infected 293T cells were immunofluorescence stained by using the anti-human CD4 antibody and then subjected to flow cytometric detection. The results are shown in the gray columns of Fig. 3: positive signals were detected in cells carrying the structure of Z09-Z13, Z17, Z34-Z37, Z01-Z05 or Z38-Z41 because these structures comprised the extracellular region of CD4. However, Z06-07 and Z14-Z15 did not contain the structure of the extracellular region of CD4, no positive signal was detected when stained with the anti-human CD4 antibody.

The results of the detection through reporter gene-red fluorescent protein (RFP) expressed thereby are shown in the black columns of Fig. 3. Positive signals of the reporter gene could be detected in all 293T cells infected with these recombinant lentivirus SIV. And as shown in Fig. 3, it can be found that in the cells carrying the Z09-Z13, Z16-Z17, Z34-Z37, Z01-Z05, Z38-Z41 structures, the ratios of positive cells shown by the two colors were consistent. The results showed that under the given detection condition, cells with two positive signals (CD4 and reporter gene expression) which can be detected at the same time belonged to the same group of cells, and their ratios were also equivalent. Therefore, the expression of fluorescent protein can indicate the expression of the CAR structure on the cell surface.

When tEGFR was selected as the reporter gene, the anti-human CD4 antibody and/or anti-tEGFR antibody were used to perform flow cytometry detection after staining. Taking Z09 as an example, positive signals were also detected in cells carrying the above structures (Fig. 4). Fig. 4A shows the result of double-staining with two antibodies, Fig. 4B shows the result of single-staining with anti-CD4 antibody, Fig. 4C shows the result of single-staining with anti-EGFR antibody, and Fig. 4D shows the result of unstained recombinant lentivirus SIV-Z09 transduced 293T cells as a control. CD4+ refers to the ratio of positive cells obtained by flow cytometry detection and analysis of 293T transduced with the recombinant lentivirus SIV-Z09 using a fluorescence-conjugated anti-human CD4 antibody, and EGFR+ refers to the ratio of positive cells obtained by flow cytometry detection and analysis of 293T transduced by the recombinant lentivirus SIV-Z09 using a fluorescence-conjugated anti-human EGFR antibody. The results showed that under the given detection conditions, the double-stained positive cells belonged to the same group of cells. The above results indicated that both the extracellular region of CD4 and tEGFR were correctly expressed on the cell surface, so the expression of tEGFR could indicate the expression of the CAR structure on the cell surface.

### Example 4: Functional validation of modified primary cells

### 4.1 Preparation of primary cells

### 4.1.1 Obtaining PBMC cells

Ficoll-Paque PLUS (GE, Cat#17-1440-02) was used to separate PBMC cells from EDTA anticoagulated whole blood (blood donated by healthy volunteers) according to the kit instructions. Specifically, the blood was centrifuged at 800 g for 30 minutes by using a highspeed refrigerated centrifuge (Thermo Scientific Sorvall ST40R), the centrifugal speed increasing factor was set to be 3, the speed reduction factor was 0, and the temperature was 20°C. After centrifugation, the PBMC cell layer was gently sucked out and placed in a new centrifuge tube. The PBMCs were washed with 3-5 times volume of DPBS, and then centrifuged at 20°C and 300 g for 10 minutes. After centrifugation, the supernatant was removed, and pre-cooled erythrocyte lysate (Tianjin Haoyang NH4CL2009) was added to the cell pellet, and the cell pellet was placed at 4°C for 2 minutes to lyse the erythrocytes. Then, pre-cooled DPBS was added to make the total volume as 40-45 mL, the cells were mixed by pipetting, and centrifuged at 300 g, 4°C for 10 minutes to obtain PBMC cells.

### 4.1.2 Sorting CD3+ T cells:

CD3+ T cells were purified using Dynabeads untouched Human T cells kit (Thermofisher 11344D) according to the manufacturer's instructions. Specifically, the PBMC cells obtained in the previous step were resuspended in a 4°C pre-cooled separation buffer (DPBS buffer containing 0.1% BSA and 2 mM EDTA) with the final volume of 300 µL, mixed by pipetting several times, and placed on ice. 10 µL solution was used for live cell counting. According to the count of viable cells, the number of PBMC cells were adjusted to 1×10⁸ cells/mL with 4°C pre-cooled separation buffer, and the corresponding volume of the required reagents was calculated and the reagents were added with reference to the instructions of the Dynabeads kit

Taking 5×10⁷ PBMC cells as an example: First, 0.5 mL of the PBMC cells with the above concentration was added in a 15 mL centrifuge tube. 100 µL of pre-cooled FBS was also added and mixed. Then 100 µL of pre-cooled Antibody Mix was added, mixed by pipetting several times, and incubated at 4°C for 20 minutes in the dark. Then, 4 mL of pre-cooled separation buffer was added, mixed by pipetting several times, centrifuged at 350 g for 8 minutes at 4°C, and the supernatant was discarded. 500 µL of separation buffer was added to resuspend the cells. 500 µL of pre-washed Dynabeads was also added and incubated for 15 minutes on a Hula mixer (18-25°C, angle 89, speed 10, vibration 2-3). After incubation, 5 mL separation buffer was added and mixed by gently pipetting 5 times. The 15 mL centrifuge tube containing the mixture was placed on the magnetic stand, and after standing for 2 minutes, the supernatant was aspirated into a new 15 mL centrifuge tube.
5 mL of separation buffer was added again into the original 15 mL centrifuge tube which had contained Dynabeads, repeating the above operation. After mixing thoroughly, the 15 mL centrifuge tube was placed on the magnetic stand and stood for 2 minutes. Then, the supernatant was aspirated and mixed with the supernatant obtained in the previous step at room temperature to obtain CD3+ T cells and the cell number was counted. CD3+ T cells were centrifuged and resuspended in X-vivo 15 medium (Lonza BE02-060F).

### 4.1.3 Activation of T cells

T cells were activated using magnetic beads coated with anti-CD3 antibody and anti-CD28 antibody following the kit instructions of Dynabeads Human T-Activator CD3/CD28 for T-Cell Expansion and Activation (Thermofisher, 11131D). First, according to the counting results of CD3+ T cells, the cells were mixed with the pre-washed CD3/CD28 magnetic beads with each 1×10⁶ cells per 25 uL magnetic beads with original concentration. The cells were supplemented with IL-2 (R&D 202-IL- 010), IL-7 (R&D 207-IL-025) and IL-15 (R&D 247-ILB-025), and human serum albumin with the final concentration of 3-5% or human AB serum or autologous serum from which the CD3 + T cells were isolated was added and thoroughly mixed (recorded as day 0 after activation), and cultured at 37°C for 48 hours.

The mixture of cells and magnetic beads was taken into a 15 mL centrifuge tube, shaken at 2000rpm for 30 seconds, and placed on a magnetic stand for 1 minute. The supernatant was aspirated into a new centrifuge tube, and the cells were resuspended with X-vivo 15 medium, and centrifuged at 300 g for 5 minutes. After centrifugation, the supernatant was discarded and the cell pellet was resuspended with fresh X-vivo 15 medium, part of which were taken for counting and flow cytometric detection (day 2 after activation).

### 4.1.4 Determination of cell ratio:

The subsets of CD3+, CD3+CD4+ double-positive, CD3+CD8+ double-positive, CD3+CD25+ double-positive cell and ratios thereof were determined by immunofluorescence staining and flow cytometry. Specifically, according to the counting results, 1×10⁶ cells were taken and centrifuged at 300 g for 5 minutes at room temperature, and then resuspended in DPBS. The multicolor staining buffer (BD Horizon Brilliant Stain Buffer Plus, 566385) was added, and a combination of mouse anti-human CD3 antibody conjugated to PE-CY7 (BD, 557851), mouse anti-human CD4 antibody conjugated to BV421 (BD, 562424) and mouse anti-human CD8 antibody conjugated to PE (BD, 555367), or a combination of mouse anti-human CD3 antibody conjugated to PE-CY7 (BD, 557851) and mouse anti-human CD25 antibody conjugated to BV421 (BD, 562442) were also added. Flow cytometry detection was carried out, and the ratios of CD3+, CD3+CD4+ double-positive, CD3+CD8+ double-positive, CD3+CD25+ double-positive cell subsets were counted. The ratio of activated cells refers to the ratio of CD3+CD25+ cells in CD3+ T cells, and CD3+ T cells not activated by magnetic beads and cytokines were used as a control.

According to the results of detection and statistical analysis, the ratio of activated cells in the living CD3+ T cells was more than 91.4% (Fig. 5).

### 4.2 Viral transduction and cell maintenance

The above-mentioned activated CD3+ T cells whose viability was verified were inoculated into culture plates or flasks coated with RetroNectin (Takara T100B, 5 µg/cm²). The CD3+ T cells were infected with a series of recombinant lentiviruses SIV (SIV-Z01, SIV-Z04, SIV09-SIV17, SIV34-SIV37) obtained in Example 2 (with MOI not exceeding 5). After 48 or 72 hours of infection, the cultures were centrifuged to remove the original culture medium. Fresh X-vivo 15 medium (Lonza BE02-060F) was used to resuspend the cells, and the cell density was adjusted to 1×10⁶/mL. The cells were supplemented with IL-2 (R&D 202-IL- 010), IL-7 (R&D 207-IL-025) and IL-15 (R&D 247-ILB-025) and human serum albumin with the final concentration of 3-5% or human AB serum or autologous serum corresponding to CD3 + T cells. The concentration of cytokines was adjusted according to cell density and growth time, usually the final concentration of IL-2 is 30 IU/mL, the final concentration of IL-7 is 5 or 10 ng/mL, and the final concentration of IL-15 is 5 or 10 ng/mL. The cells were counted and passaged every other day, and the cell density was adjusted to 1×10⁶/mL with fresh X-vivo 15 medium (containing IL-2, IL-7 and IL-15, the final concentration was as above). From then on to 12 days after activation, the density and viability of the cells were detected and recorded, and the total number of cells was calculated. CD3+ T cells (EGFP) transduced with recombinant lentivirus SIV-EGFP, and CD3+ T cells (CD3) not transduced with recombinant lentivirus were used as controls.

The results show that: under the current culture conditions, on the 12th day after activation, the number of cells was 140-440 times as much as the initial number, with the viability greater than 80% (Fig. 6A-6Q).

### 4.3 Transduction efficiency and identification of cell subpopulations

On days 13 and 19 after activation, the transduced cells (as used herein, "modified cells", "transduced cells" and "modified T cells" were used interchangeably to refer to CD3 + T cells or mixtures thereof that successfully express a CAR structure after recombinant lentivirus SIV transduction) were stained using the same antibody combination as described in section 4.1.4, and flow cytometry was used to detect the expression of CD3, CD4, CD8, and CD25. The expression of the reporter gene that was expressed simultaneously with the CAR was also detected.

Fig. 7 shows the ratio of CAR-positive cells in CD3+ cells at two time points after activation, 13 days (Fig. 7A) and 19 days (Fig. 7B). This result suggests that the ratio of CAR-positive cells remained relatively stable over time.

Fig. 8 shows the ratio of CD3+ cells in total cells (Fig. 8A) and the ratio of each cell subpopulation in CD3+ cells (Fig. 8B), without recombinant lentivirus SIV transduction. Fig. 8A is the ratio of T cells at 3 different time points during 19 days of continuous culture, which was calculated based on CD3+ T positive cells. Fig. 8B is the ratio of each subpopulation of T cells at 3 different time points during 19 days of continuous culture, wherein the ratio of CD4+ T cells decreased, while the ratio of CD8+ cells increased, which was consistent with the results reported in the literature, i.e., under the condition that favored the growth of CD8+ T cells, the number of CD8+ T cells would constantly increase.

Fig. 9 shows the ratio of each cell subpopulation at different time points after CD3+ T cells were transduced with two recombinant lentiviruses (SIV-Z09 and SIV-Z14) carrying different CAR structures. As can be seen from the figures, over time, the ratio of CD4+ T cells in CD3+ cells transduced with recombinant lentivirus SIV-Z09 (Fig. 9A) was higher than the ratio of CD4+ T cells in CD3+ cells transduced with recombinant lentivirus SIV-Z14 (Fig. 9B), and was also higher than the ratio in CD3+ cells without any transduction (Fig. 8B). The results further proved that the introduced CAR molecules were correctly expressed on the surface of CD3 + cell membrane.

### 4.4 Effects of modified T cells mixed with target cells

Recombinant plasmids, in which gp120 protein of different HIV strains (AC10.29, NL4-3 or SF162) or human MHCII (DR0401) were co-expressed with AcGFP, were constructed. The series of plasmids were transfected into 293F cells and the cells were obtained by G418 screening. Wherein, the green fluorescent protein AcGFP in the plasmid was co-expressed with gp120 or MHCII by using P2A. 293F cells transfected with AcGFP empty vector were used as negative control. The transfected 293F cells co-expressing MHCII (DR0401) and green fluorescent protein AcGFP served as a functional control for binding to CD4. Since CD4 can naturally bind to MHCII, the functional control can be used to evaluate the effect of modified T cells in the examples of this application on the cells expressing MHCII.

According to the experiment, a series of modified T cells were mixed with the 293F target cells described above, and a series of indicators were detected to determine the effect of the modified T cells. Specifically, the target cells were seeded in a 24-well plate with the density of 1×10⁴ cells/well. The stimulation of cytokines to the modified T cells was stopped 24 hours before the experiment. On the 14th day after T cell activation, the modified T cells were added to different wells at a given effector to target ratio (that is, the ratio of the number of effector cells to target cells, 10:1, 3:1 or 1:1), mixed with the target cells, and the final volume of the liquid was 200 µL/well.

4 hours after mixing, part of the supernatant was collected, with which the release of lactate dehydrogenase LDH was detected by using CytoTox 96 non-radioactive cytotoxicity detection kit (Promega G1780) to determine the acute killing activity of CD8+ T lymphocytes in the modified T cells of each experimental group to target cells.

24 hours after mixing, the supernatant was collected, with which ELISA kits (BioLegend 430204, 431804 and 430104) were used to detect the release of cytokines TNF-α, IL2 or IFN-y, respectively. In addition, the expression levels of multiple factors were simultaneously detected by using R&D Luminex Performance Human XL Cytokine Discovery Magnetic Panel (R&D FSCTM18-12) on the luminex200 instrument.

At the same time, i.e., 24 hours after mixing, cells were also collected. According to the protocol in section 4.3, the ratio of cells expressing gp120 or MHCII in CD3- cells (mainly target cells) were calculated by antibody staining and flow cytometry to determine the inhibitory activity on the target cells.

The results of the acute killing activity of CD8+ T lymphocytes in the modified T cells of each experimental group to target cells showed that 4 hours after the effector cells and target cells were mixed, the modified T cells (the modified T cells carrying Z09 or Z10 structure was taken as an example) had specific killing effect on the target cells expressing gp120 protein of different HIV strains (AC10.29, NL4-3, SF162), in a concentration- dependent manner (Fig. 12A-12C).

For the release of cytokines, IFN-γ was taken as an example. The results showed that specific stimulation could allow the modified T cells to release cytokines involved in killing (Fig. 11A-Fig. 11D). The results of multiplex cytokine detection (Fig. 14A-14J and Fig. 14L) further confirmed that when the modified T cells of the present application were co-cultured with target cells expressing gp120, the modified T cells were specifically activated and secreted a large amount of cytotoxicity-related cytokines, thereby strongly mediating the lysis of target cells. And for the modified T cells carrying Z09 or Z10 structure, for example, no significant release of factors such as IL-6 (Fig. 14K), which is significantly associated with cytokine storm, was detected, indicating that the side effects in vivo such as cytokine storms was less likely to occur in the future.

The results of the inhibitory activity of the modified T cells on the target cells showed that the modified T cells using CD4 as part of the CAR could specifically inhibit the target cells expressing gp120 when the effector to target ratio was 3:1 (Fig. 10A-10C), while had no obvious effect (Fig. 10D) on the target cells transfected with AcGFP empty vector (cells not expressing gp120). Fig. 13A-Fig. 13D show the results of the inhibitory activity of the modified T cells on the target cells at different effector to target ratios (10:1, 3:1 and 1:1), in a concentration -dependent manner. The results further confirmed that target cells expressing gp120 could be specifically inhibited by CAR-T cells that specifically recognize the antigen using the extracellular region of CD4, while modified T cells without intracellular signal transduction domain CD3ζ (that was, Z17, without a complete CAR structure) had no inhibitory effect on the target cells. In comparison, the killing effect of modified T cells carrying Z14 or Z34 structure was weaker than that of the modified T cells carrying Z09 or Z10 structure; the killing effect of the modified T cells carrying Z12 and Z13 structures was also weaker than that of the modified T cells carrying Z09 structure. The killing effect of the modified T cells carrying Z11 or Z15 structure was weaker than that of the modified T cells carrying Z10 structure; the killing effect of the modified T cells carrying Z04 structure was weaker than that of the modified T cells carrying Z01 structure. It was worth noting that under the same effector to target ratio, the modified T cells carrying Z09 structure had higher inhibitory effect on the target cells expressing gp120 than those carrying Z14 structure had (Fig. 13A-C). The difference between the Z14 and Z09 structures was that the antigen-specific recognition domain contained in the extracellular region of Z14 structure was the scFv of 3BNC117, while the antigen-specific recognition domain of Z09 was the D1-D2 segment of CD4, suggesting that the CAR-T cells using the D1-D2 segment of CD4 to recognize the antigen had a stronger inhibitory effect on the target cells expressing gp120 compared with the CAR-T cells using the scFv of 3BNC117 to recognize the antigen.

In addition, it can be seen from the above results that none of the modified T cells of the present application significantly inhibit or kill the target cells expressing MHCII (DR0401) (Fig. 12D, 13D). That is, although CD4 can naturally bind to MHCII, the original MHCII-expressing cells in vivo will not be massively killed by the modified T cells expressing CD4. It is speculated that the reason may be the affinity of CD4 to several typical HIV-1 gp120 is stronger than that to MHCII.

The above results showed the specific effect of the CAR structure in the technical solution of the present application, and it was expected that the CAR structure had a relatively low risk of cytokine storm when applied in vivo in the future, and would not kill or inhibit the original MHCII-expressing cells in vivo.

### 4.5 The in vitro simulation of the long-lasting suppression Effects of T cells

The T cells were activated, modified and cultured using the methods described above, and the cytokine stimulation was stopped on the 13th day after activation, for 24 hours. The cell density was counted and adjusted. Then the cells were continuously cultured with the presence of cytokines to the 18th day, and the cytokine stimulation was stopped again for another 24 hours. The expression level of CD25 on the surface of T cells was measured (day 19) according to the method in Section 4.1.4, and it was confirmed that the T cell was recovered to a nonactivated state after this stage of culture (Fig. 15).

On the 20th day after activation, the modified T cells were mixed with the target cells using the same method as in Section 4.4, so as to detect and evaluate the inhibitory effect of the modified T cells on the target cells. 24 hours after the modified T cells were mixed with the target cells (that is, the first day after mixing), part of the cells were collected, and antibody immunostaining and cell flow cytometry were used to analyze the ratio of cells expressing gp120 of different HIV strains (AC10.29, NL4-3, SF162) or control cells expressing AcGFP empty vector in the CD3- cells (mostly target cells), and the ratio of modified T cells in CD3+ T cells, to determine the inhibitory effect of modified T cells on target cells and the release level of cytokines.

The inhibition result of the modified T cell on the target cell showed that modified T cells using CD4 or its fragments as the antigen-specific recognition domain of CAR could specifically inhibit target cells expressing gp120 at an effector to target ratio of 3:1 (Fig. 16A-16C), and the target cells introduced with AcGFP empty vector were not significantly affected (Fig. 16D). Fig. 18A-Fig. 18D showed that the inhibitory effect of modified T cells on target cells at different effector-to-target ratios (10:1, 3:1 and 1:1), and the modified T cells (carrying Z09 or Z10 structure) had an inhibition effect on target cells expressing HIV virus gp120 (Fig. 18A-18C) in a concentration- dependent manner, while there was no obvious inhibitory effect on target cells expressing MHCII even at the highest effector-to-target ratio of 10:1 (Fig. 18D).

The ELISA assay results of cytokine release, in which IFN-γ was used as an example, showed that the specific stimulation from the antigen can make the modified T cells release cytokines related to killing (Fig. 17). The results of multiplex cytokine detection further confirmed the release of killing-related cytokines (Fig. 19A-19J and 19L), and for the modified T cells carrying Z09 or Z10 structure, no obvious release of IL-6 and other factors related to cytokine storm was detected (Fig. 19K).

These results proved the long-term effect of the CAR structure of the present application, and it was expected that the CAR structure had a relatively low risk of cytokine storm when applied in vivo for a long time in the future, and it would not kill or inhibit the original MHCII-expressing cells in vivo.

After the above-mentioned mixed cells were continuously cultured for 9 days (that is, the 9th day after mixing), the ratio of T cells (that is, percentage of the T cells in CD3+ T cells) carrying the CAR structure that had a specific effect on gp120 and the Inhibitory effect of these cells on target cells were detected.

The results show that 9 days after being mixed with target cells (the effector-to-target ratio was 3:1), the ratio of modified T cells in CD3+ T cells was maintained at a high level (Fig. 20A-C), which could continue to maintain stable or even increased compared with the first day of mixing (results not shown). At the same time, i.e., 9 days after being mixed with target cells, the modified T cells still showed continuous inhibition on target cells expressing different HIV virus gp120 (Fig. 21A-C), and showed no obvious inhibition on target cells transduced only with AcGFP empty vector (Fig. 21D).

Furthermore, for modified T cells carrying CAR structures (such as Z09 or Z10 structures), regardless of the effector-to-target ratio (10:1, 3:1 or 1:1), their ratios maintained or even increased 9 days after being mixed with target cells (Fig. 22), and they exhibited continuously suppression on target cells expressing gp120 of different HIV viruses (Fig. 23A-C), while showed no significant inhibition on target cells expressing MHCII with the effector-to-target ratio up to 10:1 (Fig. 23D).

The above results further confirmed the potential of the modified T cells based on the CAR structure of the present application to inhibit infected cells in vivo for long time, without significantly interfering with the function of the original CD4+ T cells.

### Example 5: Detection on the activity of the modified T cells by using cells from patients

In order to further prove the effectiveness of the modified T cells carrying the structure of the present application for treating HIV virus infection, the modified T cells derived from HIV patients were prepared in the presence of protease inhibitors. Specifically, T cells were isolated from the blood of HIV patients, and within 12 days thereafter, protease inhibitor Darunavir ethanolate (SelleckChem s1620) or Saquinavir mesylate (Sigma s8451) was added with a final concentration of 10 µM to the medium, and the cells were cultured under the conditions as described in section 4.2. After 12 days, the addition of the protease inhibitor was stopped and the culture was continued. The day when patient T cells were isolated, cultured and activated was regarded as day 0, and the patient-derived CD3+ T cells were modified on the 2nd day according to the method described above. From the 2nd day to the 24th day, the culture supernatant was collected at different time points, and the concentration of p24 in the supernatant was detected respectively by using the Lenti-p24 rapid titer kit (Takara 632200) according to kit instructions to quantify the HIV virus particles indirectly. The killing and inhibitory effects of patient T cells modified with complete structures (such as Z09, Z10, Z34, Z38-Z41, etc.) on HIV-infected CD4+ T cells in patients were evaluated. Meanwhile, on the 24th day, by detecting the reporter gene, the ratio of the modified T cells was determined to evaluate the protective effect of the introduced shRNA on the modified T cells.

The result in Fig. 24A showed that, for the modified T cells carrying both shRNA and CAR structures (Z09, Z10, Z12, Z16, Z34, Z38-Z41), virus p24 in the supernatant had not been detected or the concentration of virus p24 was near the detection threshold during the whole preparation process and subsequent culture without protease inhibitors. And the modified T cells carrying the Z10, Z12, Z34, Z38-Z41 structure had better inhibitory effect on p24 than the modified T cells carrying the Z16 structure did, which indicated that the modified T cells with vectors in the present application have better killing and inhibitory effect on HIV than the modified T cells with Z16, with longer duration of efficacy.

During subsequent culture in the absence of protease inhibitor, the concentration of virus p24 in the culture supernatant of modified T cells (Z02 or Z08) carrying only the CAR structure but not shRNA increased since 16th day after activation and then decreased (Fig. 24A). For modified T cells only carrying G3L2 shRNA structure (CTRL) and modified T cells carrying Z17 structure with partially incomplete CAR, the concentration of virus p24 in the culture supernatant first increased and then also decreased (Fig. 24A).

For modified T cells based on these structures (CTRL and Z17), the peak concentration of p24 in the culture supernatant appeared at 18 to 22 days after cell activation, and the peak concentration was lower than that of the unmodified CD3+ T cells (i.e., CD3 in the figures) at the same time point, while the concentration of viral p24 in the unmodified CD3+ T cell group was detectable since 16th day after activation and continued to increase in the following days (Fig. 24A). On the basis that the modified T cells derived from healthy people can inhibit and kill target cells expressing HIV virus gp120 protein, which was verified in Example 4, the above results further proved that the modified T cells from the HIV patient also had killing and inhibitory effects on autologous infected cells.

The results in Fig. 24B show that, compared with the unmodified CD3+ cell control (CD3), 24 days after being cultured with T cells from HIV patients, the ratio of the modified T cells of the present application was still maintained at a high level, indicating that the introduction shRNA had a protective effect on modified T cells.

Combining the results of the above two sections, it is confirmed that the modified T cells carrying the CAR structure of the present application could permanently and specifically kill and inhibit HIV-infected cells, and the shRNA in the present application had the advantage of protecting and maintaining the modified T cells. In particular, when compared with the modified T cells carrying the Z16 structure, the CAR-T cells of the present application had a stronger specific killing effect on HIV-infected cells, and would sustain for a longer period of time in the case of HIV infection.

Based on the above examples, the killing ability of T cells under the conditions of activation and long-term period, their resistance to HIV virus infection, and the risk of cytokine storm were comprehensively compared, and it can be found that the modified T cells carrying Z09, Z10, Z34, Z38-Z41 structure in the present invention had a relatively better comprehensive efficacy, and remarkable advantages. By simultaneously introducing the shRNA that can specifically inhibit the conserved sequence of the HIV genome and the CAR structure that targets HIV viral proteins into effector T cells, more durable and efficient CAR-T cells that target and kill HIV-infected cells can be constructed. Comparing such CAR-T cells, when the same shRNA was used, the D1 and D2 domains of CD4 had a better effect when selected as the antigen-specific recognition domain of CAR, compared with other antigen-specific recognition domains in the prior art or the D1-D4 domain of CD4. In the case that the antigen-specific recognition domains of CAR was the same, selected shRNA targeting G3L2 (for gag and LTR respectively) or G2N (for gag and nef respectively), compared with other shRNA options in the prior art, could make CAR-T cells have better comprehensive efficacy.

### Sequence list

**Table 2: All sequences mentioned herein were as follows**

| SEQ ID NO: | Sequence description | Sequences |
|---|---|---|
| 1 | Nucleotide sequence of shRNA for Gag in G3L2 (RNA) | AUAGUAAGAAUGUAUAGCCUUCAAGAGAGGCUAUACAUUCUUA CUAU |
| 2 | Nucleotide sequence of shRNA for LTR in G3L2 (RNA) | UGCUUCAAGCUAGUACCAGUUCAAGAGACUGGUACUAGCUUG AAGCA |
| 3 | Nucleotide sequence encoding shRNA for Gag in G3L2 (DNA) | ATAGTAAGAATGTATAGCCTTCAAGAGAGGCTATACATTCTTACTA T |
| 4 | Nucleotide sequence encoding shRNA for LTR in G3L2 (DNA) | TGCTTCAAGCTAGTACCAGTTCAAGAGACTGGTACTAGCTTGAA GCA |
| 5 | Nucleotide sequence of shRNA for Gag in G2N (RNA) | GUGACAUAGCAGGAACUACUUCAAGAGAGUAGUUCCUGCUAU GUCAC |
| 6 | Nucleotide sequence of shRNA for Nef in G2N (RNA) | CACAAGGCUACUUCCCUGAUUCAAGAGAUCAGGGAAGUAGCC UUGUG |
| 7 | Nucleotide sequence encoding shRNA for Gag in G2N (DNA) | GTGACATAGCAGGAACTACTTCAAGAGAGTAGTTCCTGCTATGTC AC |
| 8 | Nucleotide sequence encoding shRNA for Nef in G2N (DNA) | CACAAGGCTACTTCCCTGATTCAAGAGATCAGGGAAGTAGCCTT GTG |
| 9 | H1 promote sequence (DNA) | GAACGCTGACGTCATCGACCCGCTCCAAGGAATCGCGGGCCCA GTGTCACTAGGCGGGAACACCCAGCGCGCGTGCGCCCTGGCA GGAAGATGGCTGTGAGGGACAGGGGAGTGGCGCCCTGCAATAT TTGCATGTCGCTATGTGTTCTGGGAAATCACCATAAACGTGAAAT GTCTTTGGATTTGGGAATCTTATAAGTTCTGTATGAGACCAC |
| 10 | U6 promote sequence (DNA) | GATCCTACCAAGGTCGGGCAGGAAGAGGGCCTATTTCCCATGAT TCCTTCATATTTGCATATACGATACAAGGCTGTTAGAGAGATAATT AGAATTAATTTGACTGTAAACACAAAGATATTAGTACAAAATACGT GACGTAGAAAGTAATAATTTCTTGGGTAGTTTGCAGTTTTAAAATT |
| | | ATGTTTTAAAATGGACTATCATATGCTTACCGTAACTTGAAAGTATT TCGATTTCTTGGCTTTATATATCTTGTGGAAAGGACGAAACACCG |
| 11 | Promoter sequence of shRNA targeting CCR5 and shRNA targeting tat/rev (DNA) | GCCCGGATAGCTCAGTcGGTAGAGCACAGACTTTAATCTGAGGG TCCAGGGTCAAGTCCCTGTTCGGGCGCCA |
| 12 | shRNA targeting CCR5 and shRNA targeting tat/rev and flexible linker in between (RNA) | GCCUGGGAGAGCUGGGGAAUUUGUACGUAGGUUCCCCAGCU CUCCCAGGCgguggcaguggcuccggagguucaggaagcggcgguagugggag cGCGGAGACAGCGACGAAGAGCCUUCCUGUCAGAGCGGAGAC AGCGACGAAGAGC |
| 13 | Extracellular antigen-specific recognition region (the amino acid sequence linked by the D1 and D2 of CD4 molecule) | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLA |
| 14 | CD4 D1-D4 amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAFQKASSI VYKKEGEQVEFSFPLAFTVEKLTGSGELWWQAERASSSKSWITFD LKNKEVSVKRVTQDPKLQMGKKLPLHLTLPQALPQYAGSGNLTLAL EAKTGKLHQEVNLVVMRATQLQKNLTCEVWGPTSPKLMLSLKLEN KEAKVSKREKAVWVLNPEAGMWQCLLSDSGQVLLESNIKVLPTWS TPV |
| 15 | NAb-scFv amino acid sequence( (GGGG S) ₃ linker in the middle) | DIQMTQSPSSLSASVGDTVTITCQANGYLNWYQQRRGKAPKLLIYD GSKLERGVPSRFSGRRWGQEYNLTINNLQPEDIATYFCQVYEFVV PGTRLDLKRTVAAPGGGGSGGGGSGGGGSQVQLLQSGAAVTKP GASVRVSCEASGYNIRDYFIHWWRQAPGQGLQWVGWINPKTGQP |
| | | NNPRQFQGRVSLTRHASWDFDTFSFYMDLKALRSDDTAVYFCAR QRSDYWDFDVWGSGTQVTVSSASTKGP |
| 16 | CD4 signal peptide amino acid sequence | MNRGVPFRHLLLVLQLALLPAATQG |
| 17 | CD8 signal peptide amino acid sequence | MALPVTALLLPLALLLHAARP |
| 18 | CD8α TM amino acid sequence | IYIWAPLAGTCGVLLLSLVITLYCNHRN |
| 19 | CD28 TM amino acid sequence | KIEVMYPPPYLDNEKSNGTIIHVKGKHLCPSPLFPGPSKPFWVLVVV GGVLACYSLLVTVAFIIFWV |
| 20 | CD8 hinge region amino acid sequence | AAAFVPVFLPAKPTTTPAPRPPTPAPTIASQPLSLRPEACRPAAGGA VHTRGLDFACD |
| 21 | L3 amino acid sequence | GGGGSGGGGSGGGGS |
| 22 | L amino acid sequence | GGGGS |
| 23 | CD3ζ signal transduction domain amino acid sequence | RVKFSRSADAPAYQQGQNQLYNELNLGRREEYDVLDKRRGRDPE MGGKPRRKNPQEGLYNELQKDKMAEAYSEIGMKGERRRGKGHD GLYQGLSTATKDTYDALHMQALPPR |
| 24 | CD137 (4-1BB) signal transduction domain amino acid sequence | KRGRKKLLYIFKQPFMRPVQTTQEEDGCSCRFPEEEEGGCEL |
| 25 | CD28 signal transduction domain amino acid sequence | RSKRSRLLHSDYMNMTPRRPGPTRKHYQPYAPPRDFAAYRS |
| 26 | DAP10 signal transduction domain amino acid sequence | LCARPRRSPAQDGKVYINMPGRG |
| 27 | 2B4 signal transduction domain amino acid sequence | WRRKRKEKQSETSPKEFLTIYEDVKDLKTRRNHEQEQTFPGGGST IYSMIQSQSSAPTSQEPAYTLYSLIQPSRKSGSRKRNHSPSFNSTIY EVIGKSQPKAQNPARLSRKELENFDVYS |
| 28 | Intracellular region of Z01/Z04/Z05/Z06/Z 09/Z12/Z13/Z14/Z3 8 (CD137+CD3ζ) amino acid sequence | KRGRKKLLYIFKQPFMRPVQTTQEEDGCSCRFPEEEEGGCELRVK FSRSADAPAYQQGQNQLYNELNLGRREEYDVLDKRRGRDPEMGG KPRRKNPQEGLYNELQKDKMAEAYSEIGMKGERRRGKGHDGLYQ GLSTATKDTYDALHMQALPPR |
| 29 | Intracellular region of Z02/Z03/Z07/Z10/Z 11/Z15/Z39 (CD28+L+CD137 +L+CD3ζ) amino acid sequence | RSKRSRLLHSDYMNMTPRRPGPTRKHYQPYAPPRDFAAYRSGGG GSKRGRKKLLYIFKQPFMRPVQTTQEEDGCSCRFPEEEEGGCELG GGGSRVKFSRSADAPAYQQGQNQLYNELNLGRREEYDVLDKRRG RDPEMGGKPRRKNPQEGLYNELQKDKMAEAYSEIGMKGERRRGK GHDGLYQGLSTATKDTYDALHMQALPPR |
| 30 | Intracellular region of Z34/Z40 (CD137+CD3C+D AP10) amino acid sequence | KRGRKKLLYIFKQPFMRPVQTTQEEDGCSCRFPEEEEGGCELRVK FSRSADAPAYQQGQNQLYNELNLGRREEYDVLDKRRGRDPEMGG KPRRKNPQEGLYNELQKDKMAEAYSEIGMKGERRRGKGHDGLYQ GLSTATKDTYDALHMQALPPRLCARPRRSPAQDGKVYINMPGRG |
| 31 | Intracellular region of Z35 (CD28+DAP10+C D3ζ) amino acid sequence | RSKRSRLLHSDYMNMTPRRPGPTRKHYQPYAPPRDFAAYRSLCA RPRRSPAQDGKVYINMPGRGRVKFSRSADAPAYQQGQNQLYNEL NLGRREEYDVLDKRRGRDPEMGGKPRRKNPQEGLYNELQKDKM AEAYSEIGMKGERRRGKGHDGLYQGLSTATKDTYDALHMQALPPR |
| 32 | Intracellular region of Z36 (2B4+DAP10+CD 3ζ) amino acid sequence | WRRKRKEKQSETSPKEFLTIYEDVKDLKTRRNHEQEQTFPGGGST IYSMIQSQSSAPTSQEPAYTLYSLIQPSRKSGSRKRNHSPSFNSTIY EVIGKSQPKAQNPARLSRKELENFDVYSLCARPRRSPAQDGKVYIN MPGRGRVKFSRSADAPAYQQGQNQLYNELNLGRREEYDVLDKRR GRDPEMGGKPRRKNPQEGLYNELQKDKMAEAYSEIGMKGERRRG KGHDGLYQGLSTATKDTYDALHMQALPPR |
| 33 | Intracellular region of Z37/Z41 (CD137+2B4+CD 3ζ) amino acid sequence | KRGRKKLLYIFKQPFMRPVQTTQEEDGCSCRFPEEEEGGCELWRR KRKEKQSETSPKEFLTIYEDVKDLKTRRNHEQEQTFPGGGSTIYSM IQSQSSAPTSQEPAYTLYSLIQPSRKSGSRKRNHSPSFNSTIYEVIG KSQPKAQNPARLSRKELENFDVYSRVKFSRSADAPAYQQGQNQLY NELNLGRREEYDVLDKRRGRDPEMGGKPRRKNPQEGLYNELQKD KMAEAYSEIGMKGERRRGKGHDGLYQGLSTATKDTYDALHMQALP PR |
| 34 | Intracellular region of Z08/Z16 (CD28+CD3ζ) amino acid sequence | RSKRSRLLHSDYMNMTPRRPGPTRKHYQPYAPPRDFAAYRSRVK FSRSADAPAYQQGQNQLYNELNLGRREEYDVLDKRRGRDPEMGG KPRRKNPQEGLYNELQKDKMAEAYSEIGMKGERRRGKGHDGLYQ GLSTATKDTYDA |
| 35 | Z09/Z01/Z38 CAR (CD4D1D2+CD8h +CD8oTM+CD137 +CD3ζ) amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAAAAFVPV FLPAKPTTTPAPRPPTPAPTIASQPLSLRPEACRPAAGGAVHTRGLD FACDIYIWAPLAGTCGVLLLSLVITLYCNHRNKRGRKKLLYIFKQPFM RPVQTTQEEDGCSCRFPEEEEGGCELRVKFSRSADAPAYQQGQN QLYNELNLGRREEYDVLDKRRGRDPEMGGKPRRKNPQEGLYNEL QKDKMAEAYSEIGMKGERRRGKGHDGLYQGLSTATKDTYDALHM QALPPR |
| 36 | Z10/Z02/Z39 CAR (CD4D1D2+CD8h +CD8oTM+CD28+ L+CD137+L+CD3ζ ) amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAAAAFVPV FLPAKPTTTPAPRPPTPAPTIASQPLSLRPEACRPAAGGAVHTRGLD FACDIYIWAPLAGTCGVLLLSLVITLYCNHRNRSKRSRLLHSDYMNM TPRRPGPTRKHYQPYAPPRDFAAYRSGGGGSKRGRKKLLYIFKQP FMRPVQTTQEEDGCSCRFPEEEEGGCELGGGGSRVKFSRSADAP AYQQGQNQLYNELNLGRREEYDVLDKRRGRDPEMGGKPRRKNP QEGLYNELQKDKMAEAYSEIGMKGERRRGKGHDGLYQGLSTATKD TYDALHMQALPPR |
| 37 | Z11/Z03 CAR (CD4D1D2+L3+C D28TM+CD28+L+ CD137+L+CD3ζ) amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAGGGGSG GGGSGGGGSFPGPSKPFWVLVVVGGVLACYSLLVTVAFIIFWVRS KRSRLLHSDYMNMTPRRPGPTRKHYQPYAPPRDFAAYRSGGGGS KRGRKKLLYIFKQPFMRPVQTTQEEDGCSCRFPEEEEGGCELGG GGSRVKFSRSADAPAYQQGQNQLYNELNLGRREEYDVLDKRRGR DPEMGGKPRRKNPQEGLYNELQKDKMAEAYSEIGMKGERRRGKG HDGLYQGLSTATKDTYDALHMQALPPR |
| 38 | Z12/Z13/Z04/Z05 CAR (CD4D1-D4+CD4TM+CD13 7+CD3ζ) amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAFQKASSI VYKKEGEQVEFSFPLAFTVEKLTGSGELWWQAERASSSKSWITFD LKNKEVSVKRVTQDPKLQMGKKLPLHLTLPQALPQYAGSGNLTLAL EAKTGKLHQEVNLVVMRATQLQKNLTCEVWGPTSPKLMLSLKLEN KEAKVSKREKAVWVLNPEAGMWQCLLSDSGQVLLESNIKVLPTWS TPVQPMALIVLGGVAGLLLFIGLGIFFKRGRKKLLYIFKQPFMRPVQT TQEEDGCSCRFPEEEEGGCELRVKFSRSADAPAYQQGQNQLYNE LNLGRREEYDVLDKRRGRDPEMGGKPRRKNPQEGLYNELQKDKM AEAYSEIGMKGERRRGKGHDGLYQGLSTATKDTYDALHMQALPPR |
| 39 | Z14/Z06 CAR (Nab-scFv+CD8h+CD8a TM+CD137+CD3ζ ) amino acid sequence | DIQMTQSPSSLSASVGDTVTITCQANGYLNWYQQRRGKAPKLLIYD GSKLERGVPSRFSGRRWGQEYNLTINNLQPEDIATYFCQVYEFVV PGTRLDLKRTVAAPGGGGSGGGGSGGGGSQVQLLQSGAAVTKP GASVRVSCEASGYNIRDYFIHWWRQAPGQGLQWVGWINPKTGQP NNPRQFQGRVSLTRHASWDFDTFSFYMDLKALRSDDTAVYFCAR QRSDYWDFDVWGSGTQVTVSSASTKGPAAAFVPVFLPAKPTTTPA PRPPTPAPTIASQPLSLRPEACRPAAGGAVHTRGLDFACDIYIWAPL AGTCGVLLLSLVITLYCNHRNKRGRKKLLYIFKQPFMRPVQTTQEED GCSCRFPEEEEGGCELRVKFSRSADAPAYQQGQNQLYNELNLGR REEYDVLDKRRGRDPEMGGKPRRKNPQEGLYNELQKDKMAEAYS EIGMKGERRRGKGHDGLYQGLSTATKDTYDALHMQALPPR |
| 40 | Z15/Z07 CAR (Nab-scFv+CD8h+CD8α TM+ CD28+L+CD137+L +CD3ζ) amino acid sequence | DIQMTQSPSSLSASVGDTVTITCQANGYLNWYQQRRGKAPKLLIYD GSKLERGVPSRFSGRRWGQEYNLTINNLQPEDIATYFCQVYEFVV PGTRLDLKRTVAAPGGGGSGGGGSGGGGSQVQLLQSGAAVTKP GASVRVSCEASGYNIRDYFIHWWRQAPGQGLQWVGWINPKTGQP NNPRQFQGRVSLTRHASWDFDTFSFYMDLKALRSDDTAVYFCAR QRSDYWDFDVWGSGTQVTVSSASTKGPAAAFVPVFLPAKPTTTPA PRPPTPAPTIASQPLSLRPEACRPAAGGAVHTRGLDFACDIYIWAPL AGTCGVLLLSLVITLYCNHRNRSKRSRLLHSDYMNMTPRRPGPTRK HYQPYAPPRDFAAYRSGGGGSKRGRKKLLYIFKQPFMRPVQTTQE EDGCSCRFPEEEEGGCELGGGGSRVKFSRSADAPAYQQGQNQLY NELNLGRREEYDVLDKRRGRDPEMGGKPRRKNPQEGLYNELQKD KMAEAYSEIGMKGERRRGKGHDGLYQGLSTATKDTYDALHMQALP PR |
| 41 | Z16/Z08 CAR (CD4D-1D4+CD28TM+CD 28+CD3ζ) amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAFQKASSI VYKKEGEQVEFSFPLAFTVEKLTGSGELWWQAERASSSKSWITFD LKNKEVSVKRVTQDPKLQMGKKLPLHLTLPQALPQYAGSGNLTLAL EAKTGKLHQEVNLVVMRATQLQKNLTCEVWGPTSPKLMLSLKLEN KEAKVSKREKAVWVLNPEAGMWQCLLSDSGQVLLESNIKVLPTWS TPVPRKIEVMYPPPYLDNEKSNGTIIHVKGKHLCPSPLFPGPSKPF WVLVVVGGVLACYSLLVTVAFIIFWVRSKRSRLLHSDYMNMTPRRP GPTRKHYQPYAPPRDFAAYRSRVKFSRSADAPAYQQGQNQLYNEL NLGRREEYDVLDKRRGRDPEMGGKPRRKNPQEGLYNELQKDKM AEAYSEIGMKGERRRGKGHDGLYQGLSTATKDTYDA |
| 42 | Z17 CAR (CD4D1D2+CD8h +CD8oTM+CD137 ) amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAAAAFVPV FLPAKPTTTPAPRPPTPAPTIASQPLSLRPEACRPAAGGAVHTRGLD FACDIYIWAPLAGTCGVLLLSLVITLYCNHRNKRGRKKLLYIFKQPFM RPVQTTQEEDGCSCRFPEEEEGGCEL |
| 43 | Z34/Z40 CAR (CD4D1D2+CD8h +CD8oTM+CD137 +CD3ζ+DAP10) amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAAAAFVPV FLPAKPTTTPAPRPPTPAPTIASQPLSLRPEACRPAAGGAVHTRGLD FACDIYIWAPLAGTCGVLLLSLVITLYCNHRNKRGRKKLLYIFKQPFM RPVQTTQEEDGCSCRFPEEEEGGCELRVKFSRSADAPAYQQGQN QLYNELNLGRREEYDVLDKRRGRDPEMGGKPRRKNPQEGLYNEL QKDKMAEAYSEIGMKGERRRGKGHDGLYQGLSTATKDTYDALHM QALPPRLCARPRRSPAQDGKVYINMPGRG |
| 44 | Z35 CAR (CD4D1D2+CD8h + CD8αTM+CD28+D AP10+ CD3ζ) amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAAAAFVPV FLPAKPTTTPAPRPPTPAPTIASQPLSLRPEACRPAAGGAVHTRGLD FACDIYIWAPLAGTCGVLLLSLVITLYCNHRNRSKRSRLLHSDYMNM TPRRPGPTRKHYQPYAPPRDFAAYRSLCARPRRSPAQDGKVYINM PGRGRVKFSRSADAPAYQQGQNQLYNELNLGRREEYDVLDKRRG RDPEMGGKPRRKNPQEGLYNELQKDKMAEAYSEIGMKGERRRGK GHDGLYQGLSTATKDTYDALHMQALPPR |
| 45 | Z36 CAR (CD4D1D2+CD8h + CD8αTM+2B4+DA P10+ CD3ζ) amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAAAAFVPV FLPAKPTTTPAPRPPTPAPTIASQPLSLRPEACRPAAGGAVHTRGLD FACDIYIWAPLAGTCGVLLLSLVITLYCNHRNWRRKRKEKQSETSPK EFLTIYEDVKDLKTRRNHEQEQTFPGGGSTIYSMIQSQSSAPTSQE PAYTLYSLIQPSRKSGSRKRNHSPSFNSTIYEVIGKSQPKAQNPARL SRKELENFDVYSLCARPRRSPAQDGKVYINMPGRGRVKFSRSADA |
| | | PAYQQGQNQLYNELNLGRREEYDVLDKRRGRDPEMGGKPRRKNP QEGLYNELQKDKMAEAYSEIGMKGERRRGKGHDGLYQGLSTATKD TYDALHMQALPPR |
| 46 | Z37/Z41 CAR (CD4D1D2+CD8 h+ CD8αTM+CD137+ 2B4+ CD3ζ) amino acid sequence | KKVVLGKKGDTVELTCTASQKKSIQFHWKNSNQIKILGNQGSFLTK GPSKLNDRADSRRSLWDQGNFPLIIKNLKIEDSDTYICEVEDQKEEV QLLVFGLTANSDTHLLQGQSLTLTLESPPGSSPSVQCRSPRGKNIQ GGKTLSVSQLELQDSGTWTCTVLQNQKKVEFKIDIVVLAAAAFVPV FLPAKPTTTPAPRPPTPAPTIASQPLSLRPEACRPAAGGAVHTRGLD FACDIYIWAPLAGTCGVLLLSLVITLYCNHRNKRGRKKLLYIFKQPFM RPVQTTQEEDGCSCRFPEEEEGGCELWRRKRKEKQSETSPKEFL TIYEDVKDLKTRRNHEQEQTFPGGGSTIYSMIQSQSSAPTSQEPAY TLYSLIQPSRKSGSRKRNHSPSFNSTIYEVIGKSQPKAQNPARLSRK ELENFDVYSRVKFSRSADAPAYQQGQNQLYNELNLGRREEYDVLD KRRGRDPEMGGKPRRKNPQEGLYNELQKDKMAEAYSEIGMKGER RRGKGHDGLYQGLSTATKDTYDALHMQALPPR |
| 47 | P2A amino acid sequence | ATNFSLLKQAGDVEENPGP |
| 48 | RFP amino acid sequence | ASSEDVIKEFMRFKVRMEGSVNGHEFEIEGEGEGRPYEGTQTAKL KVTKGGPLPFAWDILSPQFQYGSKAYVKHPADIPDYLKLSFPEGFK WERVMNFEDGGVVTVTQDSSLQDGEFIYKVKLRGTNFPSDGPVM QKKTMGWEASTERMYPEDGALKGEIKMRLKLKDGGHYDAEVKTT YMAKKPVQLPGAYKTDIKLDITSHNEDYTIVEQYERAEGRHSTGA |
| 49 | tEGFR amino acid sequence | MLLLVTSLLLCELPHPAFLLIPRKVCNGIGIGEFKDSLSINATNIKHFK NCTSISGDLHILPVAFRGDSFTHTPPLDPQELDILKTVKEITGFLLIQA WPENRTDLHAFENLEIIRGRTKQHGQFSLAVVSLNITSLGLRSLKEI SDGDVIISGNKNLCYANTINWKKLFGTSGQKTKIISNRGENSCKATG QVCHALCSPEGCWGPEPRDCVSCRNVSRGRECVDKCNLLEGEP REFVENSECIQCHPECLPQAMNITCTGRGPDNCIQCAHYIDGPHCV KTCPAGVMGENNTLVWKYADAGHVCHLCHPNCTYGCTGPGLEGC PTNGPKIPSIATGMVGALLLLLVVALGIGLFM |
| 50 | tEGFR amino acid sequence (without signal peptide) | RKVCNGIGIGEFKDSLSINATNIKHFKNCTSISGDLHILPVAFRGDSF THTPPLDPQELDILKTVKEITGFLLIQAWPENRTDLHAFENLEIIRGR TKQHGQFSLAVVSLNITSLGLRSLKEISDGDVIISGNKNLCYANTINW KKLFGTSGQKTKIISNRGENSCKATGQVCHALCSPEGCWGPEPRD CVSCRNVSRGRECVDKCNLLEGEPREFVENSECIQCHPECLPQA MNITCTGRGPDNCIQCAHYIDGPHCVKTCPAGVMGENNTLVWKYA DAGHVCHLCHPNCTYGCTGPGLEGCPTNGPKIPSIATGMVGALLLL LVVALGIGLFM |

## Claims

1. A recombinant cell comprising the following functional structure:
1) a chimeric antigen receptor (CAR) or a nucleic acid coding therefor, wherein the CAR comprises an extracellular region, a transmembrane region and an intracellular region, wherein the extracellular region can specifically bind to the gp120 protein of HIV; and
2) at least one shRNA or a nucleic acid coding therefor, wherein the shRNA targets one or more host genes or HIV genes involved in the HIV life cycle selected from the group consisting of NF-κB, CCR5, TSG101, CXCR4, P-TEFb, tat, rev, nef, env, LTR and gag.

2. The recombinant cell of claim 1, wherein the shRNA comprises:
(i) shRNA targeting gag and shRNA targeting LTR, or
(ii) shRNA targeting gag and shRNA targeting nef.

3. The recombinant cell of claim 1 or 2, wherein the sequence of shRNA targeting gag comprises the sequence of SEQ ID NO: 1 or 5 or the sequence having at least 85% identity to the sequence of SEQ ID NO: 1 or 5; the sequence of the shRNA targeting LTR comprises the sequence of SEQ ID NO: 2 or the sequence having at least 85% identity to the sequence of SEQ ID NO: 2; the shRNA targeting nef comprises the sequence of SEQ ID NO: 6 or the sequence having at least 85% identity to the sequence of SEQ ID NO: 6.

4. The recombinant cell of claim 3, wherein the shRNA comprises:
(i) shRNA targeting gag and shRNA targeting LTR, wherein the shRNA targeting gag comprises the sequence of SEQ ID NO: 1, and the shRNA targeting LTR comprises the sequence of SEQ ID NO: 2.
(ii) shRNA targeting gag and shRNA targeting nef, wherein the shRNA targeting gag comprises the sequence of SEQ ID NO: 5, and the shRNA targeting nef comprises the sequence of SEQ ID NO: 6.

5. The recombinant cell of claim 1, wherein the nucleic acid coding for shRNA comprises:
(i) a nucleic acid coding for shRNA targeting gag and a nucleic acid coding for shRNA targeting LTR, or
(ii) a nucleic acid coding for shRNA targeting gag and a nucleic acid coding for shRNA targeting nef.

6. The recombinant cell of claim 1 or 5, wherein the nucleic acid coding for shRNA targeting gag comprises the sequence of SEQ ID NO: 3 or 7, or comprises the sequence having at least 85% identity to the sequence of SEQ ID NO: 3 or 7; the nucleic acid coding for shRNA targeting LTR comprises the sequence of SEQ ID NO: 4 or the sequence having at least 85% identity to the sequence of SEQ ID NO: 4; the nucleic acid coding for shRNA targeting nef comprises the sequence of SEQ ID NO: 8 or the sequence having at least 85% identity to the sequence of SEQ ID NO: 8.

7. The recombinant cell of claim 6, wherein the nucleic acid coding for shRNA comprises:
(i) a nucleic acid coding for shRNA targeting gag and a nucleic acid coding for shRNA targeting LTR, wherein the nucleic acid coding for shRNA targeting gag comprises the sequence of SEQ ID NO: 3, and the nucleic acid coding for shRNA targeting LTR comprises the sequence of SEQ ID NO: 4, or
(ii) a nucleic acid coding for shRNA targeting gag and a nucleic acid coding for shRNA targeting nef, wherein the nucleic acid coding for shRNA targeting gag comprises the sequence of SEQ ID NO: 7, and the nucleic acid coding for shRNA targeting nef comprises the sequence of SEQ ID NO: 8.

8. The recombinant cell of any one of claims 1-7, wherein the CAR further comprises a hinge region, and the extracellular region is connected to the transmembrane region by the hinge region.

9. The recombinant cell of claim 8, wherein the hinge region is from or comprises human Ig hinge region, GS linker, KIR2DS2 hinge, or the hinge region of CD8α.

10. The recombinant cell of claim 9, wherein the hinge region comprises the sequence of SEQ ID NO: 20, or comprises the sequence having at least 85% identity to the sequence of SEQ ID NO: 20.

11. The recombinant cell of any one of claims 1-10, wherein the extracellular region comprises the extracellular region of human CD4 molecule, preferably D1-D4 domain of CD4 molecule, and most preferably D1 and D2 domain of CD4 molecule.

12. The recombinant cell of claim 11, wherein the extracellular region comprises the amino acid sequence of SEQ ID NO: 13 or the amino acid sequence having at least 85% identity to the amino acid sequence of SEQ ID NO: 13.

13. The recombinant cell of any one of claims 1-12, wherein the transmembrane region is from or comprises the transmembrane domain of one or more of protein molecules selected from the group consisting of: T cell receptor α, β or ζ chain, CD28, CD3ε, CD45, CD4, CD5, CD8, CD9, CD16, CD22, CD33, CD37, CD64, CD80, CD86, CD134, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2Rβ, IL2Ry, IL7Rα, ITGA1, VLA1, CD49a, ITGA4, IA4, CD49D4, ITGA6, VLA-6, CD49f, ITGAD, CD11d, ITGAE, CD103, ITGAL, CD11a, ITGAM, CD11b, ITGAX, CD11c, ITGB1, CD29, ITGB2, CD18, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKp44, NKp30, NKp46, NKG2D, and NKG2C.

14. The recombinant cell of claim 13, wherein the transmembrane region is from or comprises the transmembrane domain of CD8α protein molecule.

15. The recombinant cell of claim 14, the transmembrane region comprises the amino acid sequence of SEQ ID NO: 18 or comprises the amino acid sequence having at least 85% identity to the amino acid sequence of SEQ ID NO: 18.

16. The recombinant cell of any one of claims 1-15, wherein the intracellular region comprises a primary signal transduction domain and a co-stimulatory domain, and the primary signal transduction domain is from or comprises the signal transduction domain of one or more of the protein molecule selected from the group consisting of: CD3ζ, CD3γ, CD3δ, CD3ε, FCER1G, FcεR1b, CD79a, CD79b, FcγRIIa, DAP10, and DAP12, and the co-stimulatory domain is from or comprises the signal transduction domain of one or more of the protein molecule selected from the group consisting of: CD27, CD28, 4-1BB, OX40, CD30, CD40, PD-1, ICOS, LFA-1, CD2, CD7, LIGHT, NKG2C, B7-H3, ligands specifically bind to CD83, CDS, ICAM-1, GITR, BAFFR, HVEM (LIGHTR), SLAMF7, NKp80, CD160, CD19, CD4, CD8α, CD8β, IL2Rβ, IL2Ry, IL7Rα, VLA1, CD49a, ITGA4, IA4, CD49D, ITGA6, VLA-6, CD49f , ITGAD, CD11d, ITGAE, CD103, ITGAL, CD11a, ITGAM, CD11b, ITGAX, CD11c, ITGB1, CD29, ITGB2, CD18, ITGB7, TNFR2, TRANCE/RANKL, DNAM1, SLAMF4, CD84, CD96, CEACAM1, CRTAM, Ly9, CD160, PSGL1, CD100, CD69, SLAMF6, SLAM, BLAME, SELPLG, LTBR, LAT, GADS, SLP-76, PAG/Cbp, NKp44, NKp30, NKp46, NKG2D, and DAP10.

17. The recombinant cell of claim 16, wherein the primary signal transduction domain is from or comprises the signal transduction domain of CD3ζ, and the co-stimulatory domain is from or comprises one of the signal transduction domains of CD137, CD28, 2B4, or DAP10, or a combination thereof.

18. The recombinant cell of any one of claims 1-15, wherein the intracellular region comprises:
(i) the signal transduction domains of CD137 and CD3ζ;
(ii) the signal transduction domains of CD28, CD137, and CD3ζ;
(iii) the signal transduction domains of CD137, CD3ζ, and DAP10;
(iv) the signal transduction domains of CD28, DAP10, and CD3ζ;
(v) the signal transduction domains of 2B4, DAP10, and CD3ζ; or
(vi) the signal transduction domains of CD137, 2B4, and CD3ζ.

19. The recombinant cell of any one of claims 16-18, wherein a linker peptide is also comprised in the intracellular region between the primary signal transduction domain and the co-stimulatory domain, and preferably, the linker peptide comprises the amino acid sequence of SEQ ID NO: 22.

20. The recombinant cell of any one of claims 1-19, wherein the intracellular region comprises the amino acid sequence of any one of SEQ ID NO: 28-34 or the amino acid sequence having at least 85% identity to any one of SEQ ID NO: 28-34.

21. The recombinant cell of any one of claims 1-20, wherein the CAR comprises an amino acid sequence selected from SEQ ID NO: 35-46 or the amino acid sequence having at least 85% identity to any one of SEQ ID NO: 35-46.

22. The recombinant cell of any one of claims 1-21, wherein a further signal peptide is linked to the CAR, and the signal peptide is from or comprises the signal peptide of any secretory proteins or membrane proteins.

23. The recombinant cell of claim 22, wherein the signal peptide is from or comprises the signal peptide of CD4 or CD8, and preferably, the signal peptide comprises the amino acid sequence of SEQ ID NO: 16 or 17.

24. The recombinant cell of any one of claims 1-23, wherein the recombinant cell further comprises a reporter molecule and/or a safety switch.

25. The recombinant cell of claim 24, wherein the safety switch is selected from one or more of the following: iCaspase-9, iCaspase-1, iCaspase-8, HSV-TK, VZV-TK, cytosine deaminase (CD), CD20, tEGFR, FR806, and RQP8.

26. The recombinant cell of claim 25, wherein the amino acid sequence of the tEGFR is shown in SEQ ID NO: 49 or 50.

27. The recombinant cell of any one of claims 1-26, wherein the recombinant cell is derived from HIV receptor cell, peripheral blood mononuclear cell or lymphocyte; preferably, the recombinant cell is derived from T cells (e.g., naive T cell, memory T cell, effector T cell, cytotoxic T cell, helper T cell, regulatory T cell, CD4+ T cell, CD8+ T cell, NKT cell, γδ T cells, etc.), NK cell, antigen-presenting cell (e.g., macrophage, dendritic cell, etc.); or the recombinant cell is derived from progenitor cell or stem cell, including but not limited to hematopoietic stem cell, hematopoietic progenitor cell, memory T stem cell (e.g., central memory T cell, effector memory T cell or stem cell-like memory T cell).

28. The recombinant cell of claim 27, wherein the recombinant cell is derived from human or primate animal; preferably, the cell is derived from patients infected with HIV or is derived from healthy population.

29. A vector comprising a polynucleotide encoding the CAR, shRNA, reporter molecule, and/or safety switch of the recombinant cell of any one of claims 1-28.

30. The vector of claim 29, selected from the group consisting of plasmids, viral vectors, or linear nucleic acid molecules.

31. The vector of claim 29 or 30, wherein the expression of chimeric antigen receptor (CAR) and shRNA are driven by a single promoter or by different promoters, and the expression of at least one shRNA is driven by a single promoter or by different promoters.

32. A chimeric antigen receptor (CAR), comprising an extracellular region, a transmembrane region and an intracellular region, wherein the extracellular region is from or comprises the extracellular region of human CD4 molecule, and the transmembrane region is from or comprising the transmembrane domain of CD8α.

33. The chimeric antigen receptor of claim 32, wherein the chimeric antigen receptor further comprises a hinge region, and the extracellular region is connected to the transmembrane region by the hinge region.

34. The chimeric antigen receptor of claim 33, wherein the hinge region is from or comprises human Ig hinge region, GS linker, KIR2DS2 hinge, or the hinge region of CD8α, preferably the hinge region of CD8α.

35. The chimeric antigen receptor of claim 34, wherein the hinge region comprises the amino acid sequence of SEQ ID NO: 20 or comprises the amino acid sequence having at least 85% identity to the sequence of SEQ ID NO: 20.

36. The chimeric antigen receptor of any one of claims 32-35, wherein the extracellular region consists of D1 and D2 domain of CD4 molecule.

37. The chimeric antigen receptor of claim 36, wherein the extracellular region comprises the amino acid sequence of SEQ ID NO: 13.

38. The chimeric antigen receptor of any one of claims 32-37, wherein the transmembrane region comprises the amino acid sequence of SEQ ID NO: 18.

39. The chimeric antigen receptor of any one of claims 32-38, wherein the intracellular region comprises a primary signal transduction domain and a co-stimulatory domain, and the primary signal transduction domain is from or comprises the signal transduction domain of one or more of the protein molecule selected from the group consisting of: CD3ζ, CD3γ, CD3δ, CD3ε, FCER1G, FcεR1b, CD79a, CD79b, FcyRlla, DAP10, and DAP12, and the co-stimulatory domain is from or comprises the signal transduction domain of one or more of the protein molecule selected from the group consisting of: CD27, CD28, 4-1BB, OX40, CD30, CD40, PD-1, ICOS, LFA-1, CD2, CD7, LIGHT, NKG2C, B7-H3, ligands specifically bind to CD83, CDS, ICAM-1, GITR, BAFFR, HVEM (LIGHTR), SLAMF7, NKp80, CD160, CD19, CD4, CD8α, CD8β, IL2Rβ, IL2Ry, IL7Rα, VLA1, CD49a, ITGA4, IA4, CD49D, ITGA6, VLA-6, CD49f , ITGAD, CD11d, ITGAE, CD103, ITGAL, CD11a, ITGAM, CD11b, ITGAX, CD11c, ITGB1, CD29, ITGB2, CD18, ITGB7, TNFR2, TRANCE/RANKL, DNAM1, SLAMF4, CD84, CD96, CEACAM1, CRTAM, Ly9, CD160, PSGL1, CD100, CD69, SLAMF6, SLAM, BLAME, SELPLG, LTBR, LAT, GADS, SLP-76, PAG/Cbp, NKp44, NKp30, NKp46, NKG2D, and DAP10.

40. The chimeric antigen receptor of claim 39, wherein the primary signal transduction domain is from or comprises the signal transduction domain of CD3ζ or DAP10, and the co-stimulatory domain is from or comprises one of the signal transduction domains of CD137, CD28, 2B4, or DAP10, or a combination thereof.

41. The chimeric antigen receptor of any one of claims 32-40, wherein the intracellular region comprises:
(i) the signal transduction domains of CD137 and CD3ζ;
(ii) the signal transduction domains of CD28, CD137, and CD3ζ;
(iii) the signal transduction domains of CD137, CD3ζ, and DAP10;
(iv) the signal transduction domains of CD28, DAP10, and CD3ζ;
(v) the signal transduction domains of 2B4, DAP10, and CD3ζ; or
(vi) the signal transduction domains of CD137, 2B4, and CD3ζ.

42. The chimeric antigen receptor of any one of claims 39-41, wherein a linker peptide is also comprised in the intracellular region between the primary signal transduction domain and the co-stimulatory domain, or between each co-stimulatory domain.

43. The chimeric antigen receptor of claim 42, wherein the sequence of the linker peptide comprises the amino acid sequence of SEQ ID NO: 22.

44. The chimeric antigen receptor of any one of claims 32-43, wherein the sequence of the intracellular region comprises the amino acid sequence of any one of SEQ ID NO: 28-34 or the amino acid sequence having at least 85% identity to any one of SEQ ID NO: 28-34.

45. The chimeric antigen receptor of any one of claims 32-44, wherein a further signal peptide is linked to the chimeric antigen receptor, and the signal peptide is from or comprises the signal peptide of any secretory proteins or membrane proteins.

46. The chimeric antigen receptor of claim 45, wherein the signal peptide is from or comprises the signal peptide of CD4 or CD8 molecule, and preferably, the signal peptide comprises the amino acid sequence of SEQ ID NO: 16 or 17.

47. The chimeric antigen receptor of any one of claims 32-46, wherein the chimeric antigen receptor comprises the amino acid sequence of any one of SEQ ID NO: 35, 36, and 43-46, or comprises the amino acid sequence having at least 85% identity to any one of SEQ ID NO: 35, 36, and 43-46.

48. A polynucleotide encoding the chimeric antigen receptor of any one of claims 32-47.

49. A vector comprising the polynucleotide of claim 48.

50. The vector of claim 49, selected from the group consisting of plasmids, viral vectors, or linear nucleic acid molecules.

51. A recombinant cell comprising the chimeric antigen receptor of any one of claims 32-47, or the polynucleotide of claim 48, or the vector of claim 49 or 50.

52. The recombinant cell of claim 51, wherein the recombinant cell is derived from human or primate animal; more preferably, the recombinant cell is derived from patients infected with HIV or is derived from healthy population; more preferably, the recombinant cell is derived from HIV receptor cell, peripheral blood mononuclear cell or lymphocyte; further preferably, the recombinant cell is derived from T cells (e.g., naive T cell, memory T cell, effector T cell, cytotoxic T cell, helper T cell, regulatory T cell, CD4+ T cell, CD8+ T cell, NKT cell, γδ T cells, etc.), NK cell, antigen-presenting cell (e.g., macrophage, dendritic cell, etc.); or the recombinant cell is derived from progenitor cell or stem cell, including but not limited to hematopoietic stem cell, hematopoietic progenitor cell, memory T stem cell (e.g., central memory T cell, effector memory T cell or stem cell-like memory T cell).

53. The combination of chimeric antigen receptor of claims 32-47, or the polynucleotide of claim 48, the vector of claim 49 or 50, or the recombinant cell of claim 51 or 52 and at least one shRNA targeting HIV or the nucleic acid coding therefor, or a vector comprising the nucleic acid thereof.

54. The combination of claim 53, wherein the at least one shRNA targets any one or more host genes or HIV genes involved in the HIV life cycle selected from the group consisting of NF-κB, CCR5, TSG101, CXCR4, P-TEFb, tat, rev, nef, env, LTR, and gag.

55. The combination of claim 54, wherein the combination comprises:
(i) shRNA targeting gag and shRNA targeting LTR, or
(ii) shRNA targeting gag and shRNA targeting nef.

56. The combination of claim 54 or 55, wherein the shRNA targeting gag comprises the sequence of SEQ ID NO: 1 or 5 or the sequence having at least 85% identity to the sequence of SEQ ID NO: 1 or 5, the shRNA targeting LTR comprises the sequence of SEQ ID NO: 2 or the sequence having at least 85% identity to the sequence of SEQ ID NO: 2, and the shRNA targeting nef comprises the sequence of SEQ ID NO: 6 or the sequence having at least 85% identity to the sequence of SEQ ID NO: 6.

57. The combination of claim 56, wherein the combination comprises:
(i) shRNA targeting gag and shRNA targeting LTR, wherein the shRNA targeting gag comprises the sequence of SEQ ID NO: 1, and the shRNA targeting LTR comprises the sequence of SEQ ID NO: 2.
(ii) shRNA targeting gag and shRNA targeting nef, wherein the shRNA targeting gag comprises the sequence of SEQ ID NO: 5, and the shRNA targeting nef comprises the sequence of SEQ ID NO: 6.

58. The combination of claim 54, wherein the combination comprises:
(i) a nucleic acid coding for the shRNA targeting gag and a nucleic acid coding for the shRNA targeting LTR, or a vector comprising the above nucleic acid, or
(ii) a nucleic acid coding for the shRNA targeting gag and a nucleic acid coding for the shRNA targeting LTR, or a vector comprising the above nucleic acid.

59. The combination of claims 54 or 58, wherein the nucleic acid coding for the shRNA targeting gag comprises the sequence of SEQ ID NO: 3 or 7 or the sequence having at least 85% identity to SEQ ID NO: 3 or 7, and the nucleic acid coding for the shRNA targeting LTR comprises the sequence of SEQ ID NO: 4 or the sequence having at least 85% identity to SEQ ID NO: 4, and the nucleic acid coding for the shRNA targeting nef comprises the sequence of SEQ ID NO: 8 or the sequence having at least 85% identity to SEQ ID NO: 8.

60. The combination of claim 59, wherein the combination comprises:
(i) a nucleic acid coding for the shRNA targeting gag and a nucleic acid coding for the shRNA targeting LTR, wherein the nucleic acid coding for the shRNA targeting gag comprises the sequence of SEQ ID NO: 3, and the nucleic acid coding for the shRNA targeting LTR comprises the sequence of SEQ ID NO: 4, or a vector comprising the above nucleic acid, or
(ii) a nucleic acid coding for the shRNA targeting gag and a nucleic acid coding for the shRNA targeting nef, wherein the nucleic acid coding for the shRNA targeting gag comprises the sequence of SEQ ID NO: 7, and the nucleic acid coding for the shRNA targeting nef comprises the sequence of SEQ ID NO: 8, or a vector comprising the above nucleic acid.

61. A pharmaceutical composition comprising the combination of the recombinant cell of any one of claims 1-28, the vector of any one of claims 29-31, the chimeric antigen receptor of any one of claims 32-47, the polynucleotide of claim 48, the vector of claim 49 or 50, the recombinant cell of claim 51 or 52, or the combination of the recombinant cell of any one of claims 53-60 and at least one shRNA or the nucleic acid coding therefor or a vector comprising the above nucleic acid, and a pharmaceutically acceptable carrier.

62. The pharmaceutical composition of claim 61, for use in treating HIV infection or AIDS.

63. The pharmaceutical composition of claim 61 or 62, wherein the pharmaceutical composition further comprises other anti-HIV drugs, including but not limited to nucleoside inhibitors, HIV vaccines, broad-spectrum neutralizing antibodies and/or CAR-T cells.

64. The use of the combination of the recombinant cell of any one of claims 1-28, the vector of any one of claims 29-31, the chimeric antigen receptor of any one of claims 32-47, or the polynucleotide of claim 48, the vector of claim 49 or 50, the recombinant cell of claim 51 or 52, or any one of claims 53-60 in the preparation of a medicament for treating and/or preventing HIV infection or AIDS.

65. A method for treating HIV infection or AIDS, comprising administering to a subject infected with HIV or suffering from AIDS the combination of the recombinant cell of any one of claims 1-28, the vector of any one of claims 29-31, the chimeric antigen receptor of any one of claims 32-47, the polynucleotide of claim 48, the vector of claim 49 or 50, the recombinant cell of claim 51 or 52, or the recombinant cell of any one of claims 53-60 and at least one shRNA or the nucleic acid coding therefor or a vector comprising the nucleic acid thereof, or the pharmaceutical composition of any one of claims 61-63.

66. The treatment method of claim 65, wherein when administrating the combination of the recombinant cell of any one of claims 53-60 and at least one shRNA or the nucleic acid coding therefor or a vector comprising the nucleic acid thereof, the recombinant cell and the at least one shRNA or the nucleic acid coding therefor or the vector comprising the nucleic acid thereof can be administrated sequentially or simultaneously.

67. The treatment method of claim 65 or 66, further comprising administering to the subject other anti-HIV drugs, including but not limited to other nucleoside inhibitors, HIV vaccines, broad-spectrum neutralizing antibodies and/or CAR-T cells.
